# EUROPEAN PATENT APPLICATION

(11) **EP 4 703 353 A1**
(43) Date of publication of application: **04.03.2026**
(21) Application number: 24198211.5
(22) Date of filing: 03.09.2024
(51) Int. Cl.: C07D 207/34, A01N 31/08, A01N 33/04, A01N 37/18, A01N 41/06, A01N 41/12, A01N 43/30, A01N 43/40, A01N 43/42, A01N 43/56, A01N 43/78, A01N 43/80, A01N 43/82, C07C 211/15, C07C 233/13, C07C 233/66, C07C 311/00

(54) **PESTICIDALLY-ACTIVE 2,2-DIHALOCYCLOPROPYL COMPOUNDS**

(71) Applicant: Syngenta Crop Protection AG, 4058 Basel (CH)
(72) Inventor: JUNG, Pierre Joseph Marcel, 4332 Stein (CH); BENFATTI, Fides, 4332 Stein (CH); MUELLER, Thorben, 4332 Stein (CH); ETEROVIC, Marisa, 4332 Stein (CH)
(74) Representative: SYNGENTA IP

(57) **Abstract**

A compound of formula (I)

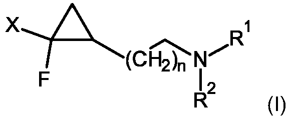

wherein the substituents are as defined in claim 1, and the agrochemically acceptable salts, stereoisomers, enantiomers, tautomers and N-oxides of those compounds, can be used as insecticides or acaricides.

## Description

The present invention relates to pesticidally-active, and in particular, insecticidally-active, 2,2-dihalocyclopropyl compounds, to compositions comprising those compounds, and to their use for controlling insect and acarine pests. Insecticidally-active 2,2-difluoro- or 2,2-fluoro,chloro-cyclopropyl compounds are known from WO 95/09151, DE 41 04 377, EP 0 468 927, US 5,326,901 and EP 0 387 974.

It has now been found that further 2,2-dihalocyclopropyl compounds have such properties.

According to the present invention, there is provided a compound of Formula (I): 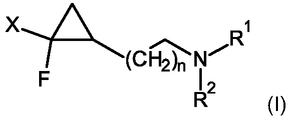 wherein:
n is 3 or 5;
X is F or Cl;
R¹ is hydrogen, hydroxy, C₁-C₆alkyl, C₁-C₄haloalkyl, C₁-C₆alkoxy, C₁-C₄haloalkoxy, C₂-C₆alkenyl, C₂-C₄haloalkenyl, C₂-C₆alkynyl, C₂-C₄haloalkynyl, C₁-C₄alkylsulfanyl, C₁-C₄alkylsulfinyl, C₁-C₄haloalkylsulfanyl, C₁-C₄haloalkylsulfinyl, phenoxy, -N=C(H)Ph, -NHC₁-C₄alkyl, -N(C₁-C₂alkyl)(C₁-C₂alkyl), C₁-C₄alkoxy substituted by a cyclopropyl group, C₁-C₄nitroalkyl, C₁-C₄alkoxyC₁-C₄alkyl, C₁-C₄haloalkoxyC₁-C₄alkyl, C₁-C₄alkylsulfonylC₁-C₄alkyl, 4-(2,2-difluorocyclopropyl)butyl, 4-(2-chloro-2-fluorocyclopropyl)butyl, 6-(2,2-difluorocyclopropyl)hexyl, 6-(2-chloro-2-fluorocyclopropyl)hexyl, -SO₂R⁴ or -C(O)R⁵; or
R¹ is phenyl optionally substituted by 1 to 3 substituents independently selected from R³, C₃-C₆cycloalkyl optionally substituted by 1 to 3 substituents independently selected from R³, a 4- to 6-membered saturated or partially saturated heterocyclic ring system comprising 1 or 2 heteroatoms individually selected from N, O, and S, wherein the heterocyclic ring is optionally substituted by 1 to 3 substituents independently selected from R³, a 5- or 6-membered heteroaromatic ring system comprising 1 to 3 heteroatoms individually selected from N, O, and S, wherein the heteroaromatic ring is optionally substituted by 1 to 3 substituents independently selected from R³, or a 9- or 10-membered heteroaromatic bicyclic ring system comprising 1 to 4 nitrogen atoms, or 0, 1 or 2 nitrogen atoms and a single atom selected from O or S, wherein the heteroaromatic bicyclic ring is optionally substituted by 1 to 3 substituents independently selected from R³; or
R¹ is C₁-C₂alkyl mono-substituted by:
   (i) a 5- or 6-membered heteroaromatic ring system comprising 1 to 3 heteroatoms individually selected from N, O, and S, wherein the heteroaromatic ring is optionally substituted by 1 to 3 substituents independently selected from R³,
   (ii) phenyl optionally substituted by 1 to 3 substituents independently selected from R³,
   (iii) a 4- to 6-membered saturated or partially saturated heterocyclic ring system comprising 1 or 2 heteroatoms individually selected from N, O, and S, wherein the heterocyclic ring is optionally substituted by 1 to 3 substituents independently selected from R³, or
   (iv) C₃-C₆cycloalkyl optionally substituted by 1 to 3 substituents independently selected from R³;
   and
R² is hydrogen, amino, C₁-C₆alkyl, C₂-C₆alkenyl, C₂-C₆alkynyl, C₁-C₄cyanoalkyl, C₁-C₄alkoxyC₁-C₄alkyl, C₁-C₄haloalkoxyC₁-C₄alkyl, or -C(O)C₁-C₄alkyl;
   or
R¹ and R², together with the nitrogen atom they share, form a 4- to 6-membered saturated or partially saturated heterocyclic ring system, optionally further comprising 1 or 2 heteroatoms individually selected from N, O, and S, wherein the heterocyclic ring is optionally substituted by 1 to 3 substituents independently selected from R⁶, or R¹ and R², together with the nitrogen atom they share, form a 9-membered saturated or partially saturated heterocyclic ring system comprising 1 or 2 heteroatoms individually selected from N, O, and S;
R³ is halogen, cyano, nitro, hydroxyl, formyl, C₁-C₆alkyl, C₁-C₄haloalkyl, C₂-C₆alkenyl, C₂-C₄haloalkenyl, C₂-C₆alkynyl, C₂-C₄haloalkynyl, C₃-C₆cycloalkyl, C₃-C₆halocycloalkyl, C₁-C₄alkylsulfanyl, C₁-C₄alkylsulfinyl, C₁-C₄alkylsulfonyl, C₁-C₄haloalkylsulfanyl, C₁-C₄haloalkylsulfinyl, C₁-C₄haloalkylsulfonyl, -C(O)C₁-C₄alkyl, -C(O)NH(C₁-C₄alkyl), -C(O)N(C₁-C₄alkyl)₂, C₁-C₆alkoxy, C₁-C₄alkoxyC₁-C₄alkyl, C₁-C₄haloalkoxy, phenyl, thiophen, C₁-C₄alkylNHC(O)C₁-C₄alkyl, C₁-C₄alkylC(O)NHC₁-C₄alkyl, -NC(O)(C₁-C₄alkyl)C₁-C₄alkyl, -NHC(O)C₁-C₄alkyl , C₁-C₄alkylNHS(O)₂-, - NHS(O)₂C₁-C₄alkyl, -N(C₁-C₄alkyl)S(O)₂C₁-C₄alkyl, -OCH₂C₃-C₆cycloalkyl, C₁-C₄cyanoalkyl, or C₃-C₆cycloalkyl substituted by a cyano;
R⁴ is C₁-C₆alkyl, C₁-C₄haloalkyl, C₁-C₆alkoxy, or C₁-C₆haloalkoxy; or
R⁴ is C₃-C₆cycloalkyl optionally substituted by 1 to 3 substituents independently selected from R³, phenyl optionally substituted by 1 to 3 substituents independently selected from R³; a 5- or 6-membered heteroaromatic ring system comprising 1 to 3 heteroatoms individually selected from N, O, and S, wherein the heteroaromatic ring is optionally substituted by 1 to 3 substituents independently selected from R³; or a 4- to 6-membered saturated or partially saturated heterocyclic ring system comprising 1 or 2 heteroatoms individually selected from N, O, and S, wherein the heterocyclic ring is optionally substituted by 1 to 3 substituents independently selected from R³; or
R⁴ is C₁-C₂alkyl mono-substituted by:
   (i) a 5- or 6-membered heteroaromatic ring system comprising 1 to 3 heteroatoms individually selected from N, O, and S, wherein the heteroaromatic ring is optionally substituted by 1 to 3 substituents independently selected from R³,
   (ii) phenyl optionally substituted by 1 to 3 substituents independently selected from R³,
   (iii) a 4- to 6-membered saturated or partially saturated heterocyclic ring system comprising 1 or 2 heteroatoms individually selected from N, O, and S, wherein the heterocyclic ring is optionally substituted by 1 to 3 substituents independently selected from R³, or
   (iv) C₃-C₆cycloalkyl optionally substituted by 1 to 3 substituents independently selected from R³; or
R⁴ is -NH(C₁-C₆alkyl), -N(C₁-C₂alkyl)(C₁-C₂alkyl), -NH(C₂-C₆alkenyl), -N(C₂-C₆alkenyl)(C₁-C₆alkyl), -NH(C₂-C₆alkynyl), -N(C₂-C₆alkynyl)(C₁-C₆alkyl), -NH(C₃-C₆cycloalkyl), -N(C₃-C₆cycloalkyl)(C₁-C₆alkyl), or -NH(phenyl) or -NH(CH₂phenyl), wherein phenyl is optionally substituted by 1 to 3 substituents independently selected from R⁶;
R⁵ is C₁-C₆alkyl, C₁-C₆haloalkyl, C₁-C₆alkoxy, C₁-C₄haloalkoxy, C₂-C₆alkenyl, C₁-C₄alkoxyC₁-C₄alkyl, 3-(2,2-difluorocyclopropyl)propyl, 3-(2-chloro-2-fluorocyclopropyl)propyl, 5-(2,2-difluorocyclopropyl)pentyl, 5-(2-chloro-2-fluorocyclopropyl)pentyl, benzylamino, or naphthyl; or
R⁵ is phenyl optionally substituted by 1 to 3 substituents independently selected from R⁶, a 5- or 6-membered heteroaromatic ring system comprising 1 to 3 heteroatoms individually selected from N, O, and S, wherein the heteroaromatic ring is optionally substituted by 1 to 3 substituents independently selected from R⁶, a 9- or 10-membered heteroaromatic bicyclic ring system comprising 1 to 4 nitrogen atoms, or 0, 1 or 2 nitrogen atoms and a single atom selected from O or S; a 5- to 6-membered saturated heterocyclic ring system comprising 1 or 2 heteroatoms individually selected from N, O, and S, wherein the heterocyclic ring is optionally substituted by 1 to 3 substituents independently selected from R⁶; or C₃-C₆cycloalkyl optionally substituted by 1 to 3 substituents independently selected from R⁶; or
R⁵ is C₁-C₂alkyl mono-substituted by:
   (i) phenoxy; or
   (ii) phenyl optionally substituted by 1 to 3 substituents independently selected from R⁶; or
R⁵ is 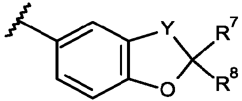 wherein Y is O or -CH₂;
R⁶ is halogen, cyano, C₁-C₄alkyl, C₁-C₄haloalkyl, C₁-C₄alkoxy, C₁-C₄haloalkoxy, C₁-C₄alkylsulfanyl, or C₁-C₄alkylNHSO₂-; and
R⁷ and R⁸ are independently selected from hydrogen, fluoro, or chloro, or R⁷ and R⁸, together with the carbon atom they share, form a C=O group;
or a salt, stereoisomer, enantiomer, tautomer or N-oxide thereof.

Surprisingly, it has been found that the novel compounds of Formula (I) have, for practical purposes, a very advantageous level of biological activity for protecting plants against insects and acarines.

According to a second aspect of the invention, there is provided an agrochemical composition comprising an insecticidally or acaricidally effective amount of a compound of formula (I) as defined according to the invention.

According to a third aspect of the invention, there is provided a method of controlling insects or acarines which comprises applying an insecticidally or acaricidally effective amount of a compound of formula (I) as defined according to the invention, or a composition comprising this compound as active ingredient, to an insect or acarine pest, a locus of the pest (preferably a plant), to a plant susceptible to attack by the pest or to a plant propagation material thereof (such as a seed). According to this particular aspect of the invention, the method may exclude methods for the treatment of the human or animal body by surgery or therapy.

According to a fourth aspect of the invention, there is provided the use of a compound according to Formula (I) as an insecticide or acaricide. According to this particular aspect of the invention, the use may exclude methods for the treatment of the human or animal body by surgery or therapy.

As used herein, the term "halogen" or "halo" refers to fluorine (fluoro), chlorine (chloro), bromine (bromo) or iodine (iodo), preferably fluorine, chlorine or bromine.

As used herein, cyano means a -CN group.

As used herein, the term "hydroxyl" or "hydroxy" means an -OH group.

As used herein, nitro means an -NO₂ group.

As used herein, amino means a -NH₂ group.

As used herein, formyl means a -C(O)H group.

As used herein, Ph means a phenyl group.

As used herein, the term "C₁-C₆alkyl" refers to a straight or branched hydrocarbon chain radical consisting solely of carbon and hydrogen atoms, containing no unsaturation, having from one to six carbon atoms, and which is attached to the rest of the molecule by a single bond. C₁-C₄alkyl, C₁-C₃alkyl and C₁-C₂alkyl are to be construed accordingly. Examples of C₁-C₆alkyl include, but are not limited to, methyl, ethyl, *n*-propyl, 1-methylethyl (isopropyl), *n*-butyl, and 1,1-dimethylethyl (*t*-butyl). A "C₁-C₄alkylene" group refers to the corresponding definition of C₁-C₄alkyl, except that such radical is attached to the rest of the molecule by two single bonds. Examples of C₁-C₄alkylene, are -CH₂- and - CH₂CH₂-.

As used herein, the term "C₁-C₆haloalkyl" refers to a C₁-C₆alkyl radical as generally defined above substituted by one or more of the same or different halogen atoms. Examples of C₁-C₆haloalkyl include, but are not limited to fluoromethyl, fluoroethyl, difluoromethyl, trifluoromethyl, 2,2-difluoroethyl, 2,2,2-trifluoroethyl, and 3,3,3-trifluoropropyl.

As used herein, the term "C₁-C₆alkoxy" refers to a radical of the formula RₐO- where Rₐ is a C₁-C₆alkyl radical as generally defined above. The term "C₁-C₄alkoxy" should be construed accordingly. Examples of C₁-C₆alkoxy include, but are not limited to, methoxy, ethoxy, propoxy, iso-propoxy, and t-butoxy.

As used herein, the term "C₁-C₄haloalkoxy" refers to a C₁-C₄alkoxy group as defined above substituted by one or more of the same or different halogen atoms. Examples of C₁-C₄haloalkoxy include, but are not limited to, fluoromethoxy, difluoromethoxy, fluoroethoxy, trifluoromethoxy, and trifluoroethoxy.

As used herein, the term "C₂-C₆alkenyl" refers to a straight or branched hydrocarbon chain radical group consisting solely of carbon and hydrogen atoms, containing at least one double bond that can be of either the (*E*)- or (*Z*)-configuration, having from two to six carbon atoms, which is attached to the rest of the molecule by a single bond. The term "C₂-C₄alkenyl" should be construed accordingly. Examples of C₂-C₆alkenyl include, but are not limited to, prop-1-enyl, allyl (prop-2-enyl), and but-1-enyl.

As used herein, the term "C₂-C₄haloalkenyl" refers to a C₂-C₄alkenyl radical as generally defined above substituted by one or more of the same or different halogen atoms.

As used herein, the term "C₂-C₆alkynyl" refers to a straight or branched hydrocarbon chain radical group consisting solely of carbon and hydrogen atoms, containing at least one triple bond, having from two to six carbon atoms, and which is attached to the rest of the molecule by a single bond. The term "C₂-C₄alkynyl" should be construed accordingly. Examples of C₂-C₆alkynyl include, but are not limited to, prop-1-ynyl, propargyl (prop-2-ynyl), and but-1-ynyl.

As used herein, the term "C₂-C₄haloalkynyl" refers to a C₂-C₄alkynyl radical as generally defined above substituted by one or more of the same or different halogen atoms.

As used herein, the term "C₃-C₆cycloalkyl" refers to a stable, monocyclic ring radical which is saturated or partially unsaturated and contains 3 to 6 carbon atoms. C₃-C₄cycloalkyl is to be construed accordingly. Examples of C₃-C₆cycloalkyl include, but are not limited to cyclopropyl, cyclobutyl, cyclopentyl, cyclopenten-1-yl, cyclopenten-3-yl, and cyclohexen-3-yl.

As used herein, the term "C₃-C₆halocycloalkyl" refers to a C₃-C₆cycloalkyl ring as defined above substituted by one or more of the same or different halogen atoms.

As used herein, the term "C₁-C₄alkoxyC₁-C₄alkyl" refers to a radical of the formula R_{y}-O-Rₓ- where R_{y} is a C₁-C₄alkyl radical as generally defined above, and Rₓ is a C₁-C₄alkylene radical as generally defined above.

As used herein, the term "C₁-C₄haloalkoxyC₁-C₄alkyl" refers to a radical of the formula R_{y}-O-Rₓ-where R_{y} is a C₁-C₄alkyl radical as generally defined above substituted by one or more of the same or different halogen atoms, and Rₓ is a C₁-C₄alkylene radical as generally defined above.

As used herein, the term "C₁-C₄cyanoalkyl" refers to a C₁-C₄alkyl radical as generally defined above substituted by a cyano group.

As used herein, the term "C₁-C₄nitroalkyl" refers to a C₁-C₄alkyl radical as generally defined above substituted by a nitro group.

As used herein, the term "C₁-C₄alkylsulfanyl" refers to a radical of the formula RₓS- wherein Rₓ is a C₁-C₄alkyl radical as generally defined above.

As used herein, the term "C₁-C₄haloalkylsulfanyl" refers to a C₁-C₄alkylsulfanyl radical as generally defined above substituted by one or more of the same or different halogen atoms.

As used herein, the term "C₁-C₄alkylsulfinyl" refers to a radical of the formula RₓS(O)- wherein Rₓ is a C₁-C₄alkyl radical as generally defined above.

As used herein, the term "C₁-C₄haloalkylsulfinyl" refers to a C₁-C₄alkylsulfinyl radical as generally defined above substituted by one or more of the same or different halogen atoms.

As used herein, the term "C₁-C₄alkylsulfonyl" refers to a radical of the formula RₓS(O)₂- wherein Rₓ is a C₁-C₄alkyl radical as generally defined above.

As used herein, the term "C₁-C₄haloalkylsulfonyl" refers to a C₁-C₄alkylsulfonyl radical as generally defined above substituted by one or more of the same or different halogen atoms.

As used herein, the term "C₁-C₄alkylsulfonylC₁-C₄alkyl" refers to a radical of the formula R_{y}S(O)₂Rₓ- where R_{y} is a C₁-C₄alkyl radical as generally defined above, and Rₓ is a C₁-C₄alkylene radical as generally defined above.

Examples of a 5- or 6-membered heteroaromatic ring system, which is monocyclic and which comprises 1 to 4 heteroatoms selected from nitrogen, oxygen and sulfur, include pyridyl, pyrimidinyl, pyrrolyl, pyrazolyl, furyl, thienyl, imidazolyl, isoxazolyl, oxazolyl, thiazolyl, isothiazolyl, triazolyl, tetrazolyl, oxadiazolyl, thiadiazolyl, pyrazinyl, pyridazinyl and triazinyl. These rings systems will not comprise contiguous oxygen and sulfur atoms.

Examples of a 9- or 10-membered heteroaromatic ring system, which is bicyclic and which comprises 1 to 4 heteroatoms selected from nitrogen, oxygen and sulfur, include quinazolinyl, quinolyl, isoquinolinyl, indolizinyl, isobenzofuranylnaphthyridinyl, quinoxalinyl, cinnolinyl, phthalazinyl, benzothiazolyl, benzoxazolyl, benzotriazolyl, indazolyl, indolyl, tetrahydroquinolynyl, benzofuranyl, benzisofuryl, benzothienyl, benzisothienyl, isoindolyl, naphthyridinyl, benzisothiazolyl, benzisoxazolyl, benzoxazolyl, benzotriazinyl, purinyl, pteridinyl, indolizinyl, phenylpyridyl, and pyridylphenyl. These rings systems will not comprise contiguous oxygen and sulfur atoms.

Examples of a 4- to 6-membered saturated or partially saturated heterocyclic ring system, which is monocyclic and which comprises 1 to 3 heteroatoms selected from nitrogen, oxygen and sulfur, include dihydropyranyl, tetrahydrofuranyl, tetrahydrothienyl, pyrrolidinyl, isoxazolidinyl, isothiazolidinyl, pyrazolidinyl, oxazolidinyl, thiazolidinyl, imidazolidinyl, oxadiazolidinyl, thiadiazolidinyl, dihydrofuranyl, dihydrothienyl, pyrrolinyl, isoxazolinyl, dihydropyrazolyl, dihydrooxazolyl, piperidinyl, dioxanyl, tetrahydropyranyl, hexahydropyridazinyl, hexahydropyrimidinyl, oxiranyl, morpholinyl and piperazinyl. These rings systems will not comprise contiguous oxygen and sulfur atoms.

The compounds of formula (I) according to the invention, which have at least one basic centre can form, for example, acid addition salts, for example with strong inorganic acids such as mineral acids, for example perchloric acid, sulfuric acid, nitric acid, a phosphorus acid or a hydrohalic acid, with strong organic carboxylic acids, such as C₁-C₄alkylcarboxylic acids which are unsubstituted or substituted, for example by halogen, for example acetic acid, such as saturated or unsaturated dicarboxylic acids, for example oxalic acid, malonic acid, succinic acid, maleic acid, fumaric acid or phthalic acid, such as hydroxycarboxylic acids, for example ascorbic acid, lactic acid, malic acid, tartaric acid or citric acid, or such as benzoic acid, or with organic sulfonic acids, such as C₁-C₄-alkane- or arylsulfonic acids which are unsubstituted or substituted, for example by halogen, for example methane- or p-toluenesulfonic acid. Compounds of formula (I) which have at least one acidic group can form, for example, salts with bases, for example mineral salts such as alkali metal or alkaline earth metal salts, for example sodium, potassium or magnesium salts, or salts with ammonia or an organic amine, such as morpholine, piperidine, pyrrolidine, a mono-, di- or tri-lower-alkylamine, for example ethyl-, diethyl-, triethyl- or dimethylpropylamine, or a mono-, di- or trihydroxy-lower-alkylamine, for example mono-, di- or triethanolamine.

The presence of one or more possible asymmetric carbon atoms in a compound of Formula (I) means that the compounds may occur in chiral isomeric forms, i.e., enantiomeric or diastereomeric forms. Also, atropisomers may occur as a result of restricted rotation about a single bond. Formula (I) is intended to include all those possible isomeric forms and mixtures thereof. The present invention includes all those possible isomeric forms and mixtures thereof for a compound of Formula (I). Likewise, Formula (I) is intended to include all possible tautomers (including lactam-lactim tautomerism and keto-enol tautomerism) where present. The present invention includes all possible tautomeric forms for a compound of Formula (I).

In each case, the compounds of Formula (I) according to the invention are in free form, in oxidized form as an N-oxide, in covalently hydrated form, or in salt form, e.g., an agronomically usable or agrochemically acceptable salt form. N-oxides are oxidized forms of tertiary amines or oxidized forms of nitrogen containing heteroaromatic compounds. They are described for instance in the book "Heterocyclic N-oxides" by A. Albini and S. Pietra, CRC Press, Boca Raton 1991. The compounds of formula (I) according to the invention also include hydrates, which may be formed during salt formation.

The following list provides definitions, including preferred definitions, for substituents X, n, R¹, R², R³, R⁴, R⁵, R⁶, R⁷, and R⁸ with reference to the compounds of Formula (I) of the present invention. For any one of these substituents, any of the definitions given below may be combined with any definition of any other substituent given below or elsewhere in this document.

X is For Ci.

In certain embodiments of the invention, X is F.

In certain embodiments of the invention, X is Cl.

R¹ is hydrogen, hydroxy, C₁-C₆alkyl, C₁-C₄haloalkyl, C₁-C₆alkoxy, C₁-C₄haloalkoxy, C₂-C₆alkenyl, C₂-C₄haloalkenyl, C₂-C₆alkynyl, C₂-C₄haloalkynyl, C₁-C₄alkylsulfanyl, C₁-C₄alkylsulfinyl, C₁-C₄haloalkylsulfanyl, C₁-C₄haloalkylsulfinyl, phenoxy, -N=C(H)Ph, -NHC₁-C₄alkyl, -N(C₁-C₂alkyl)(C₁-C₂alkyl), C₁-C₄alkoxy substituted by a cyclopropyl group, C₁-C₄nitroalkyl, C₁-C₄alkoxyC₁-C₄alkyl, C₁-C₄haloalkoxyC₁-C₄alkyl, C₁-C₄alkylsulfonylC₁-C₄alkyl, 4-(2,2-difluorocyclopropyl)butyl, 4-(2-chloro-2-fluorocyclopropyl)butyl, 6-(2,2-difluorocyclopropyl)hexyl, 6-(2-chloro-2-fluorocyclopropyl)hexyl, -SO₂R⁴ or -C(O)R⁵; or R¹ is phenyl optionally substituted by 1 to 3 substituents independently selected from R³, C₃-C₆cycloalkyl optionally substituted by 1 to 3 substituents independently selected from R³, a 4- to 6-membered saturated or partially saturated heterocyclic ring system comprising 1 or 2 heteroatoms individually selected from N, O, and S, wherein the heterocyclic ring is optionally substituted by 1 to 3 substituents independently selected from R³, a 5- or 6-membered heteroaromatic ring system comprising 1 to 3 heteroatoms individually selected from N, O, and S, wherein the heteroaromatic ring is optionally substituted by 1 to 3 substituents independently selected from R³, or a 9- or 10-membered heteroaromatic bicyclic ring system comprising 1 to 4 nitrogen atoms, or 0, 1 or 2 nitrogen atoms and a single atom selected from O or S, wherein the heteroaromatic bicyclic ring is optionally substituted by 1 to 3 substituents independently selected from R³; or R¹ is C₁-C₂alkyl mono-substituted by: (i) a 5- or 6-membered heteroaromatic ring system comprising 1 to 3 heteroatoms individually selected from N, O, and S, wherein the heteroaromatic ring is optionally substituted by 1 to 3 substituents independently selected from R³, (ii) phenyl optionally substituted by 1 to 3 substituents independently selected from R³, (iii) a 4- to 6-membered saturated or partially saturated heterocyclic ring system comprising 1 or 2 heteroatoms individually selected from N, O, and S, wherein the heterocyclic ring is optionally substituted by 1 to 3 substituents independently selected from R³, or (iv) C₃-C₆cycloalkyl optionally substituted by 1 to 3 substituents independently selected from R³.

Preferably, R¹ is hydrogen, hydroxy, C₁-C₄alkyl, C₁-C₄haloalkyl, C₁-C₄alkoxy, C₁-C₄haloalkoxy, C₂-C₄alkenyl, C₂-C₄alkynyl, C₁-C₄alkylsulfanyl, C₁-C₄alkylsulfinyl, phenoxy, -N=C(H)Ph, -NHC₁-C₄alkyl, -N(C₁-C₂alkyl)(C₁-C₂alkyl), C₁-C₄alkoxyC₁-C₄alkyl, C₁-C₄alkylsulfonylC₁-C₄alkyl, C₃-C₆cycloalkyl, 4-(2,2-difluorocyclopropyl)butyl, 4-(2-chloro-2-fluorocyclopropyl)butyl, 6-(2,2-difluorocyclopropyl)hexyl, 6-(2-chloro-2-fluorocyclopropyl)hexyl, -SO₂R⁴ or -C(O)R⁵; or R¹ is phenyl optionally substituted by 1 or 2 substituents independently selected from R³, or a 5- or 6-membered heteroaromatic ring system comprising 1 or 2 heteroatoms individually selected from N, O, and S, wherein the heteroaromatic ring is optionally substituted by 1 or 2 substituents independently selected from R³; or R¹ is C₁-C₂alkyl mono-substituted by: (i) a 5- or 6-membered heteroaromatic ring system comprising 1 to 3 heteroatoms individually selected from N, O, and S, wherein the heteroaromatic ring is optionally substituted by 1 or 2 substituents independently selected from R³, (ii) phenyl optionally substituted by 1 or 2 substituents independently selected from R³.

More preferably, R¹ is hydrogen, hydroxy, C₁-C₄alkyl, C₁-C₄haloalkyl, C₁-C₄alkoxy, C₂-C₄alkynyl, -N=C(H)Ph, C₄-C₆cycloalkyl, -N(C₁-C₂alkyl)(C₁-C₂alkyl), 4-(2,2-difluorocyclopropyl)butyl, 4-(2-chloro-2-fluorocyclopropyl)butyl, 6-(2,2-difluorocyclopropyl)hexyl, 6-(2-chloro-2-fluorocyclopropyl)hexyl, -SO₂R⁴, or -C(O)R⁵; or R¹ is phenyl, benzyl, pyridyl, (pyridyl)methyl, (pyridyl)ethyl, or pyrimidinyl, which are optionally substituted by 1 or 2 substituents selected from R³.

R² is hydrogen, amino, C₁-C₆alkyl, C₂-C₆alkenyl, C₂-C₆alkynyl, C₁-C₄cyanoalkyl, C₁-C₄alkoxyC₁-C₄alkyl, C₁-C₄haloalkoxyC₁-C₄alkyl, or -C(O)C₁-C₄alkyl. Preferably, R² is hydrogen, amino, C₁-C₄alkyl, C₂-C₄alkenyl, C₂-C₄alkynyl, C₁-C₄cyanoalkyl, C₁-C₂alkoxyC₁-C₂alkyl, or -C(O)C₁-C₄alkyl. More preferably, R² is hydrogen, methyl or cyanomethyl. Even more preferably, R² is hydrogen.

Alternatively, R¹ and R², together with the nitrogen atom they share, form a 4- to 6-membered saturated or partially saturated heterocyclic ring system, optionally further comprising 1 or 2 heteroatoms individually selected from N, O, and S, wherein the heterocyclic ring is optionally substituted by 1 to 3 substituents independently selected from R⁶, or R¹ and R², together with the nitrogen atom they share, form a 9-membered saturated or partially saturated heterocyclic ring system comprising 1 or 2 heteroatoms individually selected from N, O, and S.

Preferably, R¹ and R², together with the nitrogen atom they share, form a 4-, 5- or 6-membered saturated or partially saturated heterocyclic ring system optionally further comprising a single group selected from -O-, -S-, -N(H)- or =N-, wherein the ring system is optionally substituted by 1 or 2 substituents independently selected from fluoro, chloro, cyano, methyl, ethyl, methoxy. More preferably, R¹ and R², together with the nitrogen atom they share, form an isoxazolidinyl, 3,4-dihydro-pyrazolyl, thiomorpholinyl, azetidinyl, pyrrolidinyl, piperidinyl, or piperazinyl group optionally substituted by 1 or 2 substituents independently selected from fluoro, chloro, cyano, methyl, ethyl, methoxy. Even more preferably, R¹ and R², together with the nitrogen atom they share, form a pyrrolidinyl or a piperidinyl group.

R³ is halogen, cyano, nitro, hydroxyl, formyl, C₁-C₆alkyl, C₁-C₄haloalkyl, C₂-C₆alkenyl, C₂-C₄haloalkenyl, C₂-C₆alkynyl, C₂-C₄haloalkynyl, C₃-C₆cycloalkyl, C₃-C₆halocycloalkyl, C₁-C₄alkylsulfanyl, C₁-C₄alkylsulfinyl, C₁-C₄alkylsulfonyl, C₁-C₄haloalkylsulfanyl, C₁-C₄haloalkylsulfinyl, C₁-C₄haloalkylsulfonyl, -C(O)C₁-C₄alkyl, -C(O)NH(C₁-C₄alkyl), -C(O)N(C₁-C₄alkyl)₂, C₁-C₆alkoxy, C₁-C₄alkoxyC₁-C₄alkyl, C₁-C₄haloalkoxy, phenyl, thiophen, C₁-C₄alkylNHC(O)C₁-C₄alkyl, C₁-C₄alkylC(O)NHC₁-C₄alkyl, -NC(O)(C₁-C₄alkyl)C₁-C₄alkyl, -NHC(O)C₁-C₄alkyl , C₁-C₄alkylNHS(O)₂-, - NHS(O)₂C₁-C₄alkyl, -N(C₁-C₄alkyl)S(O)₂C₁-C₄alkyl, -OCH₂C₃-C₆cycloalkyl, C₁-C₄cyanoalkyl, or C₃-C₆cycloalkyl substituted by a cyano.

Preferably, R³ is halogen, cyano, hydroxyl, C₁-C₄alkyl, C₁-C₄haloalkyl, C₃-C₆cycloalkyl, C₃-C₆halocycloalkyl, C₁-C₄alkylsulfanyl, C₁-C₄alkylsulfonyl, -C(O)C₁-C₄alkyl, -C(O)NH(C₁-C₄alkyl), C₁-C₄alkoxy, C₁-C₄alkoxyC₁-C₄alkyl, C₁-C₄haloalkoxy, C₁-C₄alkylNHS(O)₂-, C₁-C₄cyanoalkyl, or C₃-C₆cycloalkyl substituted by a cyano. More preferably, R³ is halogen, cyano, hydroxy, C₁-C₄alkyl, C₁-C₄alkoxy, C₁-C₄alkoxy-C₁-C₄alkyl, C₁-C₂alkylsulfonyl, or C₁-C₂cyanoalkyl.

R⁴ is C₁-C₆alkyl, C₁-C₄haloalkyl, C₁-C₆alkoxy, or C₁-C₆haloalkoxy; or R⁴ is C₃-C₆cycloalkyl optionally substituted by 1 to 3 substituents independently selected from R³, phenyl optionally substituted by 1 to 3 substituents independently selected from R³; a 5- or 6-membered heteroaromatic ring system comprising 1 to 3 heteroatoms individually selected from N, O, and S, wherein the heteroaromatic ring is optionally substituted by 1 to 3 substituents independently selected from R³; or a 4- to 6-membered saturated or partially saturated heterocyclic ring system comprising 1 or 2 heteroatoms individually selected from N, O, and S, wherein the heterocyclic ring is optionally substituted by 1 to 3 substituents independently selected from R³; or R⁴ is C₁-C₂alkyl mono-substituted by: (i) a 5- or 6-membered heteroaromatic ring system comprising 1 to 3 heteroatoms individually selected from N, O, and S, wherein the heteroaromatic ring is optionally substituted by 1 to 3 substituents independently selected from R³, (ii) phenyl optionally substituted by 1 to 3 substituents independently selected from R³, (iii) a 4- to 6-membered saturated or partially saturated heterocyclic ring system comprising 1 or 2 heteroatoms individually selected from N, O, and S, wherein the heterocyclic ring is optionally substituted by 1 to 3 substituents independently selected from R³, or (iv) C₃-C₆cycloalkyl optionally substituted by 1 to 3 substituents independently selected from R³; or R⁴ is -NH(C₁-C₆alkyl), - N(C₁-C₂alkyl)(C₁-C₂alkyl), -NH(C₂-C₆alkenyl), -N(C₂-C₆alkenyl)(C₁-C₆alkyl), -NH(C₂-C₆alkynyl), -N(C₂-C₆alkynyl)(C₁-C₆alkyl), -NH(C₃-C₆cycloalkyl), -N(C₃-C₆cycloalkyl)(C₁-C₆alkyl), or -NH(phenyl) or - NH(CH₂phenyl), wherein phenyl is optionally substituted by 1 to 3 substituents independently selected from R⁶.

Preferably, R⁴ is C₁-C₄alkyl, C₁-C₄haloalkyl, C₁-C₄alkoxy, or C₁-C₄haloalkoxy; or R⁴ is C₃-C₆cycloalkyl optionally substituted by 1 or 2 substituents independently selected from R³, phenyl optionally substituted by 1 or 2 substituents independently selected from R³; a 5- or 6-membered heteroaromatic ring system comprising 1 to 3 heteroatoms individually selected from N, O, and S, wherein the heteroaromatic ring is optionally substituted by 1 or 2 substituents independently selected from R³; or R⁴ is C₁-C₂alkyl mono-substituted by:(i) a 5- or 6-membered heteroaromatic ring system comprising 1 to 3 heteroatoms individually selected from N, O, and S, wherein the heteroaromatic ring is optionally substituted by 1 or 2 substituents independently selected from R³, (ii) phenyl optionally substituted by 1 or 2 substituents independently selected from R³, or (iii) C₃-C₆cycloalkyl optionally substituted by 1 or 2 substituents independently selected from R³; or R⁴ is -NH(C₁-C₄alkyl), or -N(C₁-C₂alkyl)(C₁-C₂alkyl).

More preferably, R⁴ is C₁-C₄alkyl, pyrrolidinyl, or piperidyl; or R⁴ is a ring selected from cyclopropyl, cyclobutyl, cyclopentyl, azetidinyl, imidazolyl, triazolyl, thienyl, isoxazolyl, pyridyl, pyrazolyl, pyrimidinyl, or phenyl, wherein the ring is optionally substituted by 1 or 2 substituents independently selected from R³; or R⁴ is C₁-C₂alkyl mono-substituted by cyclopropyl, cyclobutyl, pyridyl, phenyl, thienyl, pyrazolyl, isoxazolyl, or isothiazolyl, which are optionally substituted by 1 or 2 substituents independently selected from R³; or R⁴ is -NH(C₁-C₄alkyl), or -N(C₁-C₂alkyl)(C₁-C₂alkyl).

Even more preferably, R⁴ is C₁-C₄alkyl, pyrrolidinyl, or piperidyl; or R⁴ is a ring selected from cyclopropyl, cyclobutyl, imidazolyl, triazolyl, thiophenyl, isoxazolyl, pyridyl, pyrimidinyl, pyrazolyl, or phenyl, wherein the ring is optionally substituted by 1 or 2 substituents independently selected from R³; or R⁴ is C₁-C₂alkyl mono-substituted by cyclopropyl, cyclobutyl, pyridyl, or phenyl, which are optionally substituted by 1 or 2 substituents independently selected from R³; or R⁴ is -NH(C₁-C₄alkyl), or -N(C₁-C₂alkyl)(C₁-C₂alkyl).

R⁵ is C₁-C₆alkyl, C₁-C₆haloalkyl, C₁-C₆alkoxy, C₁-C₄haloalkoxy, C₂-C₆alkenyl, C₁-C₄alkoxyC₁-C₄alkyl, 3-(2,2-difluorocyclopropyl)propyl, 3-(2-chloro-2-fluorocyclopropyl)propyl, 5-(2,2-difluorocyclopropyl)pentyl, 5-(2-chloro-2-fluorocyclopropyl)pentyl, benzylamino, or naphthyl; or

R⁵ is phenyl optionally substituted by 1 to 3 substituents independently selected from R⁶, a 5- or 6-membered heteroaromatic ring system comprising 1 to 3 heteroatoms individually selected from N, O, and S, wherein the heteroaromatic ring is optionally substituted by 1 to 3 substituents independently selected from R⁶, a 9- or 10-membered heteroaromatic bicyclic ring system comprising 1 to 4 nitrogen atoms, or 0, 1 or 2 nitrogen atoms and a single atom selected from O or S; a 5- to 6-membered saturated heterocyclic ring system comprising 1 or 2 heteroatoms individually selected from N, O, and S, wherein the heterocyclic ring is optionally substituted by 1 to 3 substituents independently selected from R⁶; or C₃-C₆cycloalkyl optionally substituted by 1 to 3 substituents independently selected from R⁶; or

R⁵ is C₁-C₂alkyl mono-substituted by:
(i) phenoxy; or
(ii) phenyl optionally substituted by 1 to 3 substituents independently selected from R⁶; or R5 is 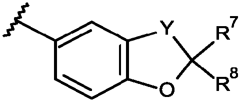 wherein Y is O or -CH₂.

Preferably, R⁵ is C₁-C₄alkyl, C₁-C₆haloalkyl, C₂-C₄alkenyl, C₁-C₄alkoxy-C₁-C₄alkyl, 3-(2,2-difluorocyclopropyl)propyl, 3-(2-chloro-2-fluorocyclopropyl)propyl, 5-(2,2-difluorocyclopropyl)pentyl, 5-(2-chloro-2-fluorocyclopropyl)pentyl, benzylamino, or naphthyl; or R⁵ is phenyl optionally substituted by 1 or 2 substituents independently selected from R⁶, a 5- or 6-membered heteroaromatic ring system comprising 1 to 3 heteroatoms individually selected from N, O, and S, wherein the heteroaromatic ring is optionally substituted by 1 or 2 substituents independently selected from R⁶, a 9- or 10-membered heteroaromatic bicyclic ring system comprising 1 to 4 nitrogen atoms, or 0, 1 or 2 nitrogen atoms and a single atom selected from O or S; a 5- to 6-membered saturated heterocyclic ring system comprising 1 or 2 heteroatoms individually selected from N, O, and S, wherein the heterocyclic ring is optionally substituted by 1 or 2 substituents independently selected from R⁶; or C₃-C₆cycloalkyl optionally substituted by 1 or 2 substituents independently selected from R⁶; or R⁵ is C₁-C₂alkyl mono-substituted by: (i) phenoxy; or (ii) phenyl optionally substituted by 1 or 2 substituents independently selected from R⁶, or
R5 is 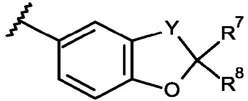 wherein Y is O or -CH₂.

More preferably R⁵ is C₁-C₄alkyl, C₁-C₆haloalkyl, C₂-C₄alkenyl, C₁-C₄alkoxy-C₁-C₄alkyl, 3-(2,2-difluorocyclopropyl)propyl, 3-(2-chloro-2-fluorocyclopropyl)propyl, 5-(2,2-difluorocyclopropyl)pentyl, 5-(2-chloro-2-fluorocyclopropyl)pentyl, benzylamino, naphthyl, quinolyl, or 2,2-difluoro-1,3-benzodioxolyl; or R⁵ is a ring selected from cyclopropyl, cyclobutyl, phenyl, pyrrolidinyl, piperidyl, 4,5-dihydroisoxazolyl, isoxazolyl, pyrrolyl, pyrazolyl, thiazolyl, thiadiazolyl, imidazolyl, isothiazolyl, pyridazinyl, or pyridyl, wherein each ring is optionally substituted by 1 or 2 substituents independently selected from R⁶; or R⁵ is C₁-C₂alkyl mono-substituted by: (i) phenoxy; or (ii) phenyl optionally substituted by a single substituent independently selected from R⁶.

R⁶ is halogen, cyano, C₁-C₄alkyl, C₁-C₄haloalkyl, C₁-C₄alkoxy, C₁-C₄haloalkoxy, C₁-C₄alkylsulfanyl, or C₁-C₄alkylNHSO₂-. Preferably, R⁶ is halogen, C₁-C₄alkyl, C₁-C₄haloalkyl, C₁-C₄alkoxy, or C₁-C₂alkylsulfanyl. More preferably, R⁶ is chloro, fluoro, methyl, ethyl, trifluoromethyl, methoxy, ethoxy, or methylsulfanyl.

R⁷ and R⁸ are independently selected from hydrogen, fluoro, or chloro, or R⁷ and R⁸, together with the carbon atom they share, form a C=O group. Preferably R⁷ and R⁸ are independently selected from hydrogen, fluoro, and even more preferably fluoro.

In certain embodiments of the invention, R¹ is -SO₂R⁴, wherein:
R⁴ is C₁-C₄alkyl, C₁-C₄haloalkyl, C₁-C₄alkoxy, or C₁-C₄haloalkoxy; or R⁴ is C₃-C₆cycloalkyl optionally substituted by 1 or 2 substituents independently selected from R³, phenyl optionally substituted by 1, 2, or 3 substituents independently selected from R³; a 5- or 6-membered heteroaromatic ring system comprising 1 to 3 heteroatoms individually selected from N, O, and S, wherein the heteroaromatic ring is optionally substituted by 1, 2, or 3 substituents independently selected from R³; or a 4- to 6-membered saturated or partially saturated heterocyclic ring system comprising 1 or 2 heteroatoms individually selected from N, O, and S, wherein the heterocyclic ring is optionally substituted by 1 or 2 substituents independently selected from R³; or R⁴ is C₁-C₂alkyl mono-substituted by:(i) a 5- or 6-membered heteroaromatic ring system comprising 1 to 3 heteroatoms individually selected from N, O, and S, wherein the heteroaromatic ring is optionally substituted by 1 or 2 substituents independently selected from R³, (ii) phenyl optionally substituted by 1 or 2 substituents independently selected from R³, or (iii) C₃-C₆cycloalkyl optionally substituted by 1 or 2 substituents independently selected from R³; or R⁴ is -NH(C₁-C₄alkyl), or -N(C₁-C₂alkyl)(C₁-C₂alkyl); and
R³ is halogen, cyano, hydroxy, C₁-C₄alkyl, C₁-C₄alkoxy, C₁-C₄alkoxy-C₁-C₄alkyl, C₁-C₂alkylsulfonyl, or C₁-C₂cyanoalkyl.

In preferred embodiments of the invention, R¹ is -SO₂R⁴, where:
R⁴ is C₁-C₄alkyl, C₁-C₄haloalkyl, C₁-C₄alkoxy, or C₁-C₄haloalkoxy; or R⁴ is C₃-C₆cycloalkyl optionally substituted by 1 or 2 substituents independently selected from R³; phenyl optionally substituted by 1 or 2 substituents independently selected from R³; a 5- or 6-membered heteroaromatic ring system comprising 1 or 2 heteroatoms individually selected from N, O, and S, wherein the heteroaromatic ring is optionally substituted by 1 or 2 substituents independently selected from R³; or a 5- or 6-membered saturated heterocyclic ring system comprising 1 or 2 nitrogen atoms; or R⁴ is C₁-C₂alkyl mono-substituted by: (i) a 5- or 6-membered heteroaromatic ring system comprising 1 or 2 nitrogen atoms, wherein the heteroaromatic ring is optionally substituted by a single substituent independently selected from R³, (ii) phenyl optionally substituted by 1 or 2 substituents independently selected from R³, or (iii) C₃-C₆cycloalkyl optionally substituted by a single substituents independently selected from R³; or R⁴ is -NH(C₁-C₄alkyl), or -N(C₁-C₂alkyl)(C₁-C₂alkyl); and
R³ is halogen, cyano, hydroxy, C₁-C₄alkyl, C₁-C₄alkoxy, C₁-C₄alkoxy-C₁-C₄alkyl, C₁-C₂alkylsulfonyl, or C₁-C₂cyanoalkyl.

In certain embodiments of the invention, R¹ is -C(O)R⁵, where
R⁵ is C₁-C₄alkyl, C₁-C₆haloalkyl, C₂-C₄alkenyl, C₁-C₄alkoxy-C₁-C₄alkyl, 3-(2,2-difluorocyclopropyl)propyl, 3-(2-chloro-2-fluorocyclopropyl)propyl, 5-(2,2-difluorocyclopropyl)pentyl, 5-(2-chloro-2-fluorocyclopropyl)pentyl,benzylamino, or naphthyl; or R⁵ is phenyl optionally substituted by 1 or 2 substituents independently selected from R⁶, a 5- or 6-membered heteroaromatic ring system comprising 1 or 2 heteroatoms individually selected from N, O, and S, wherein the heteroaromatic ring is optionally substituted by 1 or 2 substituents independently selected from R⁶, a 9- or 10-membered heteroaromatic bicyclic ring system comprising 1 to 4 nitrogen atoms, or 0, 1 or 2 nitrogen atoms and a single atom selected from O or S; a 5- to 6-membered saturated heterocyclic ring system comprising 1 or 2 heteroatoms individually selected from N, O, and S, wherein the heterocyclic ring is optionally substituted by 1 or 2 substituents independently selected from R⁶; or C₃-C₆cycloalkyl optionally substituted by a single substituent independently selected from R⁶; or
R⁵ is C₁-C₂alkyl mono-substituted by:
   (i) phenoxy; or
   (ii) phenyl optionally substituted by a single substituent independently selected from R⁶; or
R5 is 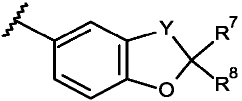 wherein Y is O or -CH₂; and
R⁶ is chloro, fluoro, methyl, ethyl, trifluoromethyl, methoxy, ethoxy or methylsulfanyl; and
R⁷ and R⁸ are independently selected from hydrogen, fluoro, and even more preferably fluoro.

In some preferred embodiments, R¹ is hydrogen, hydroxy, C₁-C₄alkyl, C₁-C₄haloalkyl, C₁-C₄alkoxy, C₂-C₄alkynyl, -N=C(H)Ph, C₃-C₆cycloalkyl, -N(C₁-C₂alkyl)(C₁-C₂alkyl), 4-(2,2-difluorocyclopropyl)butyl, 4-(2-chloro-2-fluorocyclopropyl)butyl, 6-(2,2-difluorocyclopropyl)hexyl, 6-(2-chloro-2-fluorocyclopropyl)hexyl, -SO₂R⁴ or -C(O)R⁵; or R¹ is phenyl, benzyl, pyridyl, (pyridyl)methyl, (pyridyl)ethyl, or pyrimidinyl, which are optionally substituted by 1 or 2 substituents independently selected from R³; and wherein
R³ is halogen, cyano, hydroxy, C₁-C₄alkyl, C₁-C₄alkoxy, C₁-C₄alkoxy-C₁-C₄alkyl, C₁-C₂alkylsulfonyl, or C₁-C₂cyanoalkyl;
R⁴ is C₁-C₄alkyl, pyrrolidinyl, or piperidyl; or R⁴ is a ring selected from cyclopropyl, cyclobutyl, phenyl, pyrazolyl, imidazolyl, triazolyl, thiophenyl, isoxazolyl, pyridyl, or pyrimidinyl, wherein the ring is optionally substituted by 1 or 2 substituents independently selected from R³; or R⁴ is C₁-C₂alkyl mono-substituted by cyclopropyl, cyclobutyl, pyridyl, or phenyl, which are optionally substituted by 1 or 2 substituents independently selected from R³; or R⁴ is -NH(C₁-C₄alkyl), or -N(C₁-C₂alkyl)(C₁-C₂alkyl);
R⁵ is C₁-C₄alkyl, C₁-C₆haloalkyl, C₂-C₄alkenyl, C₁-C₄alkoxy-C₁-C₄alkyl, 3-(2,2-difluorocyclopropyl)propyl, 3-(2-chloro-2-fluorocyclopropyl)propyl, 5-(2,2-difluorocyclopropyl)pentyl, 5-(2-chloro-2-fluorocyclopropyl)pentyl,benzylamino, naphthyl, quinolyl, or 2,2-difluoro-1,3-benzodioxolyl; or R⁵ is a ring selected from cyclopropyl, cyclobutyl, phenyl, pyrrolidinyl, piperidyl, 4,5-dihydroisoxazolyl, isoxazolyl, pyrrolyl, pyrazolyl, thiazolyl, thiadiazolyl, imidazolyl, isothiazolyl, pyridazinyl, or pyridyl, wherein each ring is optionally substituted by 1 or 2 substituents independently selected from R⁶; or R⁵ is C₁-C₂alkyl mono-substituted by: (i) phenoxy; or (ii) phenyl optionally substituted by a single substituent independently selected from R⁶; and
R⁶ is chloro, fluoro, methyl, ethyl, trifluoromethyl, methoxy, ethoxy or methylsulfanyl.

Preferably, the compound according to Formula (I) is selected from a compound A1 to A41 listed in Table A (below) or a compound disclosed in Tables 1 to 13 (below).

The present compound of formula (I) contains asymmetric centers and, consequently, could be chiral. Unless otherwise specified, the present invention includes a mixture of isomers/diastereoisomers.

When X is chlorine compounds of formula (I) may be a mixture of isomers and diastereoisomers. For example, to illustrate this: 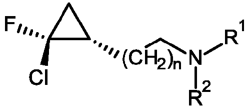 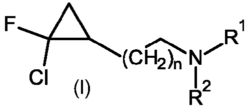 is a mixture of

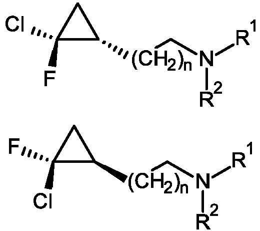

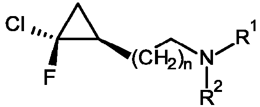

When X is fluorine, compounds of formula (I) may be a mixture of enantiomers. For example, to illustrate this: 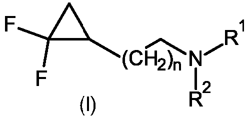 is a mixture of

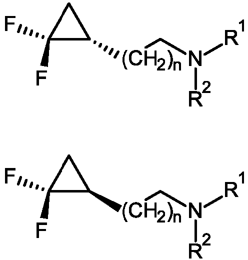

Compounds of the present invention can be made as shown in the following schemes 1 to 10, in which, unless otherwise stated, the definition of each variable is as defined herein for a compound of formula (I).

The process according to the invention for preparing compounds of formula (I) is carried out by methods known to those skilled in the art. Compounds of formula (I), wherein R¹ and R² are as defined in the present invention, can be readily prepared, by analogy, according to, previews literature such as, for example, by analogy with EP 318425, EP 468927, EP 3459948 and WO 2017/106607.

Examples of such synthesis are shown below for compounds of formula (I). 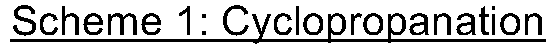 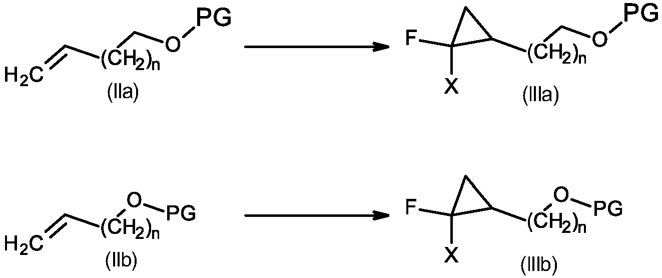 wherein PG is protecting group

A compound of formula (Illa) can be prepared by cyclopropanation of an alkene of formula (IIa). Reactions of cyclopropanation are very well know and may include reaction of a compound of formula (II) with reactive carbenoid species generate or not *in situ.*

For example, carbenoid species wherein X is F, may be generated from trimethylsilyl difluoro(fluorosulfonyl)acetate (TFDA) at temperatures from 80°C to 200°C in the presence of sodium fluoride as described in J Fluor Chem (2004), 125, 459, in WO 2017/106607. Or, alternatively, by reaction of the compound of formula (IIa) with sodium chlorodifluoroacetate dissolved in a solvent such as diglyme, at temperatures from 100°C to 200°C, optionally under microwave irradiation (see for example US 5,089,662 or Chemistry Letters (2005), 34(10), 1440-1441), or alternatively, by reaction of the compound of formula (IIa) with a suitable organometallic precursor such as phenyl(trifluoromethyl) mercury or bis(trifluoromethyl)cadmium dissolved in a solvent such as benzene or toluene, in presence or not of sodium iodide at temperatures from -50°C to 150°C (see for example: e-EROS Encyclopedia of Reagents for Organic Synthesis (2006), 1-3 or Journal of Organometallic Chemistry (1995), 503(2), C51-C54). Or, alternatively, by reaction of the compound of formula (IIa) with silyl derivatives such as (chlorodifluoromethyl)trimethylsilane or (bromodifluoromethyl)trimethylsilane preferably under phase transfer catalysis conditions at temperatures from 80°C to 150°C (see for example Chemical Communications (2011), 47(8), 2411-2413, European Journal of Organic Chemistry (2019), 2019(7), 1669-1676 or Chemical Communications (2021), 57(3), 319-322. Other methods for *in situ* carbenoid generation include, for example, the use of trifluoromethane in presence of a base, such as sodium hydroxide, preferably under phase transfer catalysis conditions or the use of ester derivatives such as ethyl bromodifluoroacetate, preferably under phase transfer catalysis conditions in the appropriate solvent such as tetrahydrofuran or/and anisole (see for example WO 2022/003584).

Other similar examples are for carbenoid species, wherein X is Cl. These carbenoid species could be generated from a halogenate derivative such as dichlorofluoromethane in presence of a base, such as sodium hydroxide or butyllithium or reagents such as titanium tetrachloride, lithium aluminum hydride, under phase transfer catalysis conditions or not (see for example and analogy Russian Chemical Bulletin (2019), 68(7), 1391-1401, Journal of Organic Chemistry (2012), 77(21), 9893-9899, Science of Synthesis (2006), 34, 245-265, Tetrahedron Letters 1988, p 6749 or Journal of Organic Chemistry (1990), 55(2), 589-94)). Alternatively, carbenoid species wherein X is Cl, could be generated from the use of ester, acid or salt of acid derivatives such as sodium dichlorofluoroacetate, ethyl dichlorofluoroacetate under phase transfer catalysis conditions or not, in the appropriate solvent such as dichloromethane and under or not presence of a catalyst, such as silver derivatives or a base or not, such as sodium hydroxide (see for example, Organic Letters (2022), 24(29), 5417-5421and cited references), or by reaction of the compound of formula (IIa) with silyl derivatives such as fluorodichloromethyl)trimethylsilane, preferably under phase transfer catalysis conditions at temperature ranged from 80°C to 150°C (see for example Chemical Communications (2021), 57(3), 319-322). Other reagents could be used to generate carbenoid species wherein X is Cl, for example, polyhalogenated carbonyl compounds such as 1,1,3,3-tetrachloro-1,3-difluoroacetone in the appropriate solvent such as dichloromethane and under presence or not of a base, such as sodium methoxide (see for example Tetrahedron Letters (1967), (29), 2773-6 or Recueil des Travaux Chimiques des Pays-Bas et de la Belgique 1947 p 419. Other reagents that could be used to generate carbenoid species wherein X is Cl are, for example, by reaction with a compound of formula (IIa): with a suitable organometallic precursor such as phenyl(fluorodichloromethyl) mercury (see for example: Journal of Organic Chemistry (1970), 35(5), 1297-302 or Journal of Organometallic Chemistry (1968), 11(1), P9-P12), or with diazirines (see for example: Journal of the American Chemical Society (2005), 127(8), 2408-2409). These types of reactions are known to a person skilled in the art and can be prepared according to known methods described in these reviews, see for example: Studies of reactions of fluorinated carbenes and lithium carbenoids Hahnfeld, Jerry L (1975) PhD, 243 pages, ChemMedChem (2022), 17(21), e202200365, Emerging Fluorinated Motifs (2020), 1, 135-194 or Science of Synthesis Fluorine (2006), 245-266.
Similarly, a compound of formula (IIIb) can be prepared by cyclopropanation of an alkene of formula (IIb).

Synthesis of compound of formula (IIa) and (IIb) are very well known by those skilled in the art. For example, if the protection group PG is a benzoyl, the compound is commercially available (e.g. for compound (IIa): CAS 41795-26-0 or 2855129-34-7 and for compound (IIb): CAS 29264-40-2 or 1198803-20-1). Others protecting group could be used and are described in see for example Protective Groups in organic synthesis, Third Edition, Greene, Theodora W.; Wuts, Peter G. M. 1999, Publisher John Wiley and Sons, Inc. p 17.

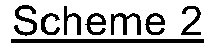

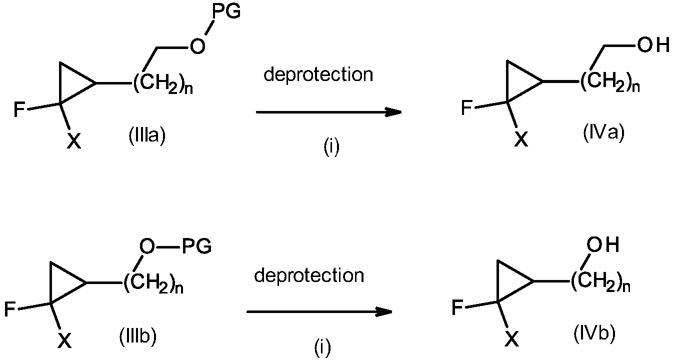

Compounds of formula (IVa), respectively (IVb), may be prepared by deprotection of the protecting group using various methods, as function of the protecting group use (see for example Protective Groups in organic synthesis, Third Edition, Greene, Theodora W.; Wuts, Peter G. M. 1999, Publisher John Wiley and Sons, Inc. p 17). For example, if the protecting group is benzoyl, the deprotection could be made by hydrolysis in presence of a base such as sodium hydroxide or potassium carbonate in solvent such as methanol, ethanol, or water. The reaction is carried out at a temperature of from 0°C to +100°C.

This process is very well known when X is F and could be apply, after adaptation / by analogy to the synthesis of all compounds of formula (Illa). See for example: EP 468927, EP 3459948 and WO 2017/106607.

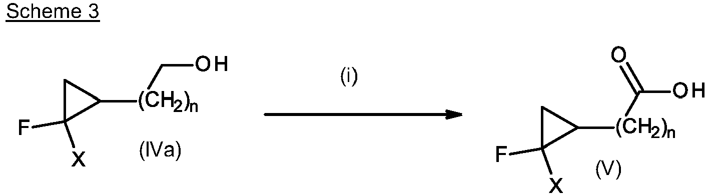

The intermediate compounds of formula (V) could be obtained by oxidation of the compound of formula (IVa). This transformation is very well known and could be made by methods known to a person skilled in the art (see for example Comprehensive Organic Transformations. A Guide to Functional Group Preparations. Edited by Richard C. Larock 1989 p 834, VCH publishers). For example, compound of formula (V) may be prepared by reaction of a compound of formula (IVa), via oxidation with the chromium trioxide reagent in the presence of a solvent or mixture of solvent, such as, for example, water and acetone, in presence or not of an acid such as sulfuric acid at a temperature of from 0°C to 50°C.

This process is very well known and could be apply, after adaptation / analogy to the synthesis of all compounds of formula (V). See for example: EP 0 468 927, EP 3 459 948 and WO 2017/106607.

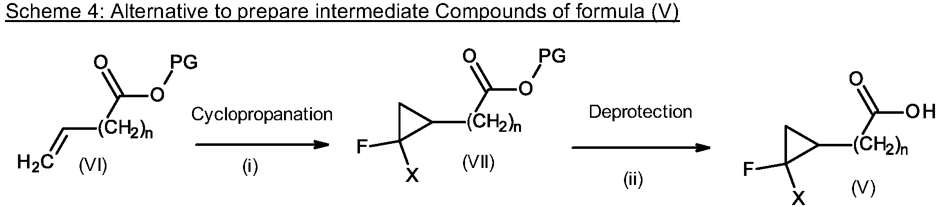

Alternatively, Compounds of formula (V), may be prepared by:
i) cyclopropanation of compounds of formula (VI) wherein PG is a protecting group such as alkyl or benzyl derivatives, as described in scheme 1; followed by
ii) deprotection of compound of formula (VII), wherein PG is a protecting group. For instance, in the case where PG is methyl or ethyl, the hydrolysis can be done in a solvent such as water and a base, such as potassium hydroxide or lithium hydroxide, in the absence or in the presence of another solvent, such as, for instance, tetrahydrofuran or methanol. In the case where PG is, for example, tert-butyl, the hydrolysis is done in the presence of acid, such as trifluoroacetic acid or hydrochloric acid. The reaction is carried out at a temperature of from -120°C to +130°C, preferably from -100°C to 100°C. This transformation is well known and could be made by methods known to a person skilled in the art (see for example Comprehensive Organic Transformations. A Guide to Functional Group Preparations. Edited by Richard C. Larock 1989 p 981, VCH publishers). Others protecting group could be used and are described in see for example Protective Groups in organic synthesis, Third Edition, Greene, Theodora W.; Wuts, Peter G. M. 1999, Publisher John Wiley and Sons, Inc. p 17.

Synthesis of compound of formula (VI) are very well known by those skilled in the art and some derivatives type compounds of formula (VI) are commercially available, for example, compounds of formula (VI) wherein n is 3: if the protection group PG is a benzyl, the compound is commercially available (CAS 87995-27-5), same if PG is methyl or ethyl (CAS 2396-80-7 or 54653-25-7). And for compounds of formula (VI) wherein n is 5: if the protection group PG is a benzyl, the compound is commercially available (CAS 2245790-52-5), same if PG is methyl or ethyl (CAS 15766-90-2 or 35194-38-8). Others protecting group could be used and are described in see for example Protective Groups in organic synthesis, Third Edition, Greene, Theodora W.; Wuts, Peter G. M. 1999, Publisher John Wiley and Sons, Inc. p 17.

This process is very well known and could be apply, after adaptation to the synthesis of all compounds of formula (V). See for example: US 5,089,662.

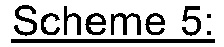

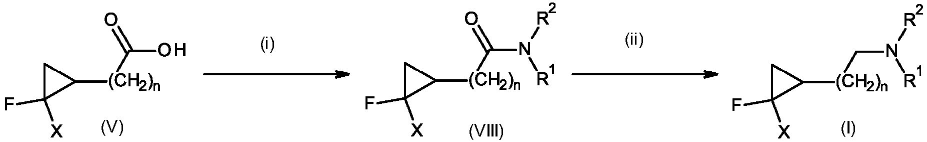

Compounds of formula (I), may be prepared by:
i) coupling of the compound of formula (V) with an amine NHR¹R², using similar conditions described in scheme 5; followed by
ii) reduction of the amide (VIII) to the amine (I) using a reducing agent such as, for example, sodium borohydride or lithium aluminum hydride in the presence or not of acid, such as acetic acid in the appropriate solvent. This transformation is well known and could be made by methods known to a person skilled in the art (see for example Comprehensive Organic Transformations. A Guide to Functional Group Preparations. Edited by Richard C. Larock 1989 p 432, VCH publishers).

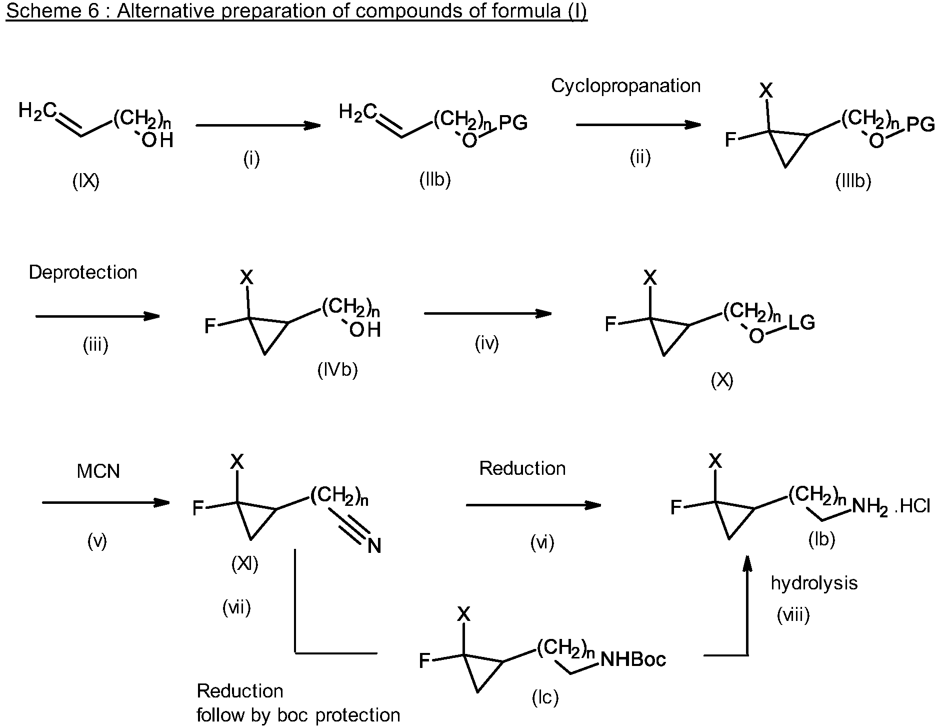

Compounds of formula (Ib), may be prepared by
i) protection of the alcohol (IX) with a protecting group such as benzoyl. Many protecting groups could be use (see for example Protective Groups in organic synthesis, Third Edition, Greene, Theodora W.; Wuts, Peter G. M. 1999, Publisher John Wiley and Sons, Inc. p 17). For example, the use of benzoyl acid activated by derivation in acyl chloride or activation via treatment with, for example, 1-ethyl-3-(3-dimethylaminopropyl) carbodiimide or dicyclohexyl carbodiimide will generate an activated species that could reacted with alcohol (IX). This type of reaction is very well known to those skilled in the art and described in, for example, Tetrahedron, 2005, 61 (46), 10827-10852; followed by
ii) cyclopropanation with a carbene, using condition described in scheme 1, to synthesize compounds of formula (IIb); followed by
iii) deprotection of the protecting group using various methods, as function of the protecting group use (see for example Protective Groups in organic synthesis, Third Edition, Greene, Theodora W.; Wuts, Peter G. M. 1999, Publisher John Wiley and Sons, Inc. p 17) to give compounds of formula (IVb). For example, if the protecting group is benzoyl, the deprotection could be made by hydrolysis in presence of a base such as sodium hydroxide or potassium carbonate in solvent such as methanol, ethanol, or water. The reaction is carried out at a temperature of from 0°C to +100°C; followed by
iv) activation of the hydroxyl via formation of a leaving group via, such, for example, as an aryl- or alkylsulfonate derivatives, preferably 4-methylbenzenesulfonate. These reactions are well known to those skilled in the art and described in, for example, Synthetic Organic Methodology: Comprehensive Organic Transformations. A Guide to Functional Group Preparations, Larock, R. C. 1989 p 353 then 360 too. Another way to active the hydroxyl group is to transform it to a halogen (for analogy, see scheme 7). These reactions are well known to those skilled in the art and described in, for example, Comprehensive Organic Transformations (second Edition). A Guide to Functional Group Preparations, Larock, R. C. 1999 p 689; followed by
v) substitution of the leaving group type aryl- or alkylsulfonate derivatives or halogen by a cyano via reaction with a reagent such as sodium cyanide or potassium cyanide in solvent such as dimethylformamide or DMSO. The reaction is carried out at a temperature of from 25°C to +150°C; followed by
vi) Reduction of the cyano group to an amine group with reaction of compounds of formula (XI) under classical reduction conditions (see for example: Synthetic Organic Methodology: Comprehensive Organic Transformations. A Guide to Functional Group Preparations, Larock, R. C. 1989 p 437).
(vii) and (viii). Alternatively, it's sometime more convenient for the isolation of compounds of formula (Ib) to first make the reduction of the cyano group to an amine group followed by protection with a Boc group for example, in presence of Boc anhydride and, or not, in presence of a base such as triethylamine, in presence, or not, of solvent such as dimethylformamide. The reaction is carried out at a temperature of from 0°C to +100°C. (see for example, Protective Groups in organic synthesis, Third Edition, Greene, Theodora W.; Wuts, Peter G. M. 1999, Publisher John Wiley and Sons, Inc. p 518); followed by hydrolysis of compounds of formula (Ic) in the presence of acid, such as hydrochloric acid in a solvent such as water or methanol. The reaction is carried out at a temperature of from -120°C to +130°C, preferably from 25 to 100°C.

Synthesis of some analogues of compounds of formula (IVb) or type (IIIb) are analogue to known compounds and consequently could be made by analogy : See for example, Jingxi Huagong (2015), 32(2), 237-240 or JP07109237.

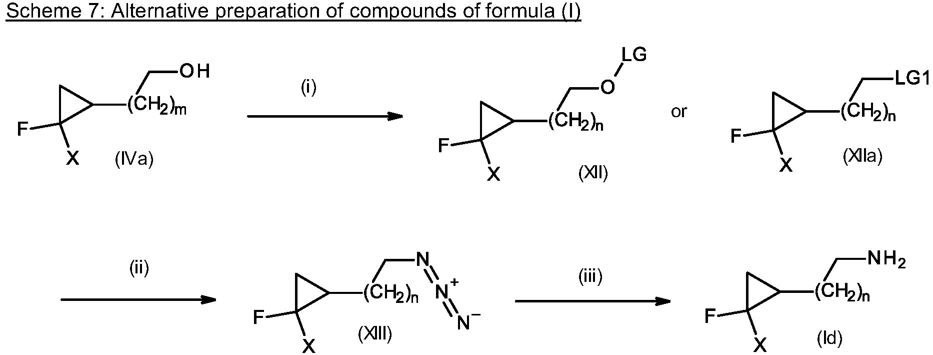

Compounds of formula (Id), may be prepared by
i) activation of the hydroxyl via formation of a leaving group via, such, for example, as aryl- or alkylsulfonate derivatives, such as 4-methylbenzenesulfonate to give analogues compounds of formula (XII). Alternatively, the hydroxyl of Compounds of formula (IVa), could be transformed in halogen such as chlorine or bromide to give compounds of formula (XIIa) wherein LG1 is chloride or bromide. These reactions are well known to those skilled in the art and described in, for example, Synthetic Organic Methodology: Comprehensive Organic Transformations. A Guide to Functional Group Preparations, Larock, R. C. 1989 p 353 then 360 too; followed by
ii) substitution of the leaving group LG1 or -OLG with an azide salt such as Sodium azide, in inert solvent such as dimethylformamide, to give compounds of formula (XIII). These reactions are well known to those skilled in the art and described in, for example, US20200079772, Synthesis (2015), 47(7), 992-1006, WO2013122821, Chemistry - A European Journal (2018), 24(47), 12291-12297 or Tetrahedron: Asymmetry (2015), 26(21-22), 1268-1272; followed by
iii) Reduction of the azide to an amine via classical conditions to give compounds of formula (Id) or a salt equivalent such as, for example, (Ib). These reactions are well known to those skilled in the art and described in, for example, US20200079772, Synthesis (2015), 47(7), 992-1006, WO2013122821, Chemistry - A European Journal (2018), 24(47), 12291-12297 or Tetrahedron: Asymmetry (2015), 26(21-22), 1268-1272.

For preparing all other compounds of the formula (I) functionalized according to the definitions of formula I above, there are a large number of suitable known standard methods, for example alkylation, halogenation, acylation, amidation, aminoreduction (e.g. see Comprehensive Organic Transformations. A Guide to Functional Group Preparations (1989) Larock, R. C. (Publisher: VCH Weinheim, Fed. Rep. Ger.) p 421), oxidation and reduction, the choice of the preparation methods which are suitable depending on the properties (reactivity) of the substituents in the intermediates. These methods including transformation of functional group as well, for example, transformation of amide to amine, of alcohol to amine, of amine to amide etc... More specific methods of preparation are exemplified in the following schemes.

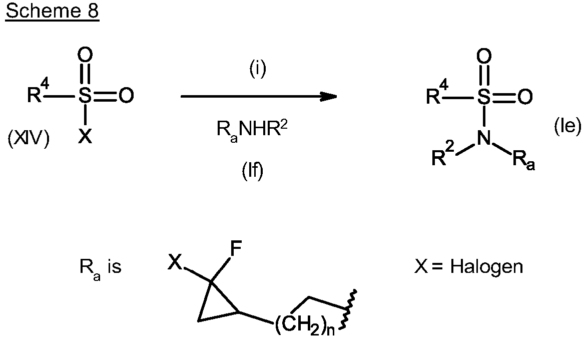

Compounds of formula (Ie), wherein R⁴ is as defined above, and Rₐ as described in scheme 8 may be prepared by reaction of compounds of formula (If), wherein Rₐ as described in scheme 8 and R² is described above (RₐNHR²) with a suitably sulfonyl chloride of formula (XIV). Typically, the reaction of compounds of formula (If) with a reagent such as a R⁴ sulfonyl halide in solvent such as tetrahydrofuran or any other suitable solvent, in presence of a base such as triethylamine at a temperature of from 25°C to +150°C led to the desired compound. Compound of formula (XIV) are commercially available or can be prepared from commercially available starting materials using methods known to the person skilled in the art. These types of reaction are well known to those skilled in the art. 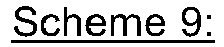 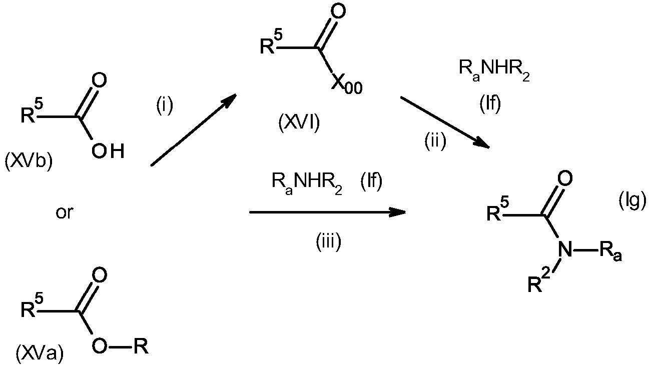 R is a 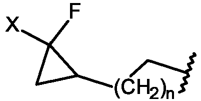 X₀₀ = Halogen,

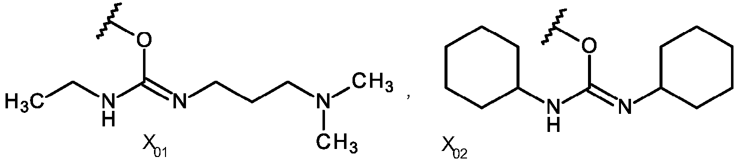

Compounds of formula (Ig), wherein R⁵ and R² are as described in the present invention, and Rₐ as described in scheme 9 may be prepared by
i) activation of a compound of formula (XVb), wherein R⁵ as described above, by methods known to those skilled in the art and described in, for example, Tetrahedron, 2005, 61 (46), 10827-10852, to form an activated species (XVI), wherein R⁵ as described above and wherein X₀₀ is halogen, preferably chlorine. For example, compounds (XXVIII) where X₀₀ is halogen, preferably chlorine, are formed by treatment of XVb), with, for example, oxalyl chloride or thionyl chloride in the presence of catalytic quantities of *N,N*-dimethylformamide in inert solvents such as methylene chloride or tetrahydrofuran at temperatures between 20°C to 100°C, preferably 25°C. Alternatively, treatment of compounds of formula (XVb) with, for example, 1-ethyl-3-(3-dimethylaminopropyl)carbodiimide or dicyclohexyl carbodiimide will generate an activated species (XVI), wherein X₀₀ is X₀₁ or X₀₂ respectively, in an inert solvent, such as pyridine or tetrahydrofuran (THF), optionally in the presence of a base, such as triethylamine, at temperatures between 50°C-180°C; followed by
ii) treatment of the activated species (XVI) with an amine reagent of formula (If), wherein Rₐ as described in scheme 9 and R² as described above (RₐNHR²), or a salt thereof (such as a hydrohalide salt, preferably a hydrochloride or a hydrobromide salt, or any other equivalent salt, optionally in the presence of a base, such as triethylamine or pyridine, in an inert solvent such as dichloromethane, tetrahydrofuran, dioxane or toluene, at temperatures between 0°C and 50°C, to form the compounds of formula (Ig). Certain bases, such as pyridine and triethylamine, may be employed successfully as both base and solvent.
   These types of reaction are well known to those skilled in the art, see for example, Synthetic Organic Methodology: Comprehensive Organic Transformations. A Guide to Functional Group Preparations, Larock, R. C. 1989 p 972.
(iii) Alternatively, compounds of formula (Ig), wherein R⁵ is as defined above, and Rₐ as described in scheme 5 may be prepared by reaction of a compound of formula (XVa), by methods known to those skilled in the art and described in, for example, by heating compounds of formula (XVa), wherein R is alkyl or benzyl and R⁵ as described above, in presence of amine (If), with or without solvent such as methanol or water, and with or without presence of a catalyst, such as Lewis acid derivatives optionally in the presence of a base, such as triethylamine, at temperatures between 50°C-250°C, optionally under microwave irradiation. These types of reaction are well known to those skilled in the art, see for example, Synthetic Organic Methodology: Comprehensive Organic Transformations. A Guide to Functional Group Preparations, Larock, R. C. 1989 p 987.

In accordance with the reactions described in any of Schemes 1 to 9, examples of suitable bases may include alkali metal or alkaline earth metal hydroxides, alkali metal or alkaline earth metal hydrides, alkali metal or alkaline earth metal amides, alkali metal or alkaline earth metal alkoxides, alkali metal or alkaline earth metal acetates, alkali metal or alkaline earth metal carbonates, alkali metal or alkaline earth metal dialkylamides or alkali metal or alkaline earth metal alkylsilylamides, alkylamines, alkylenediamines, free or N-alkylated saturated or unsaturated cycloalkylamines, basic heterocycles, ammonium hydroxides and carbocyclic amines. Examples which may be mentioned are sodium hydroxide, sodium hydride, sodium amide, sodium methoxide, sodium acetate, sodium carbonate, potassium tert-butoxide, potassium hydroxide, potassium carbonate, potassium hydride, lithium diisopropylamide, potassium bis(trimethylsilyl)amide, calcium hydride, triethylamine, diisopropylethylamine, triethylenediamine, cyclohexylamine, N-cyclohexyl-N,N-dimethylamine, N,N-diethylaniline, pyridine, 4-(N,N-dimethylamino)pyridine, quinuclidine, N-methylmorpholine, benzyltrimethylammonium hydroxide and 1,8-diazabicyclo[5.4.0]undec-7-ene (DBU).

The reactants can be reacted with each other as such, i.e. without adding a solvent or diluent. In most cases, however, it is advantageous to add an inert solvent or diluent or a mixture of these. If the reaction is carried out in the presence of a base, bases which are employed in excess, such as triethylamine, pyridine, N-methylmorpholine or N,N-diethylaniline, may also act as solvents or diluents.

Reactions are advantageously carried out in a temperature range from approximately -80 °C to approximately 140 °C, preferably from approximately -30 °C to approximately 100 °C, in many cases in the range between ambient temperature and approximately 80 °C.

A compound of formula (I) can be converted in a manner known per se into another compound of formula (I) by replacing one or more substituents of the starting compound of formula (I) in the customary manner by (an)other substituent(s) according to the invention.

Depending on the choice of the reaction conditions and starting materials which are suitable in each case, it is possible, for example, in one reaction step only to replace one substituent by another substituent according to the invention, or a plurality of substituents can be replaced by other substituents according to the invention in the same reaction step.

Salts of compounds of formula (I) can be prepared in a manner known perse. Thus, for example, acid addition salts of compounds of formula (I) are obtained by treatment with a suitable acid or a suitable ion exchanger reagent and salts with bases are obtained by treatment with a suitable base or with a suitable ion exchanger reagent.

Salts of compounds of formula (I) can be converted in the customary manner into the free compounds (I), acid addition salts, for example, by treatment with a suitable basic compound or with a suitable ion exchanger reagent and salts with bases, for example, by treatment with a suitable acid or with a suitable ion exchanger reagent.

Salts of compounds of formula (I) can be converted in a manner known *per se* into other salts of compounds of formula (I), acid addition salts, for example, into other acid addition salts, for example by treatment of a salt of inorganic acid such as hydrochloride with a suitable metal salt such as a sodium, barium or silver salt, of an acid, for example with silver acetate, in a suitable solvent in which an inorganic salt which forms, for example silver chloride, is insoluble and thus precipitates from the reaction mixture.

Depending on the procedure or the reaction conditions, the compounds of formula (I), which have salt-forming properties, can be obtained in free form or in the form of salts.

The compounds of formula (I) and, where appropriate, the tautomer's thereof, in each case in free form or in salt form, can be present in the form of one of the isomers which are possible or as a mixture of these, for example in the form of pure isomers, such as antipodes and/or diastereomers, or as isomer mixtures, such as enantiomer mixtures, for example racemates, diastereomer mixtures or racemate mixtures, depending on the number, absolute and relative configuration of asymmetric carbon atoms which occur in the molecule and/or depending on the configuration of non-aromatic double bonds which occur in the molecule, the invention relates to the pure isomers and also to all isomer mixtures which are possible and is to be understood in each case in this sense hereinabove and herein below, even when stereochemical details are not mentioned specifically in each case.

Diastereomeric mixtures or racemic mixtures of compounds of formula (I), in free form or in salt form, which can be obtained depending on which starting materials and procedures have been chosen can be separated in a known manner into the pure diastereomers or racemates on the basis of the physicochemical differences of the components, for example by fractional crystallization, distillation and/or chromatography.

Enantiomeric mixtures, such as racemates, which can be obtained in a similar manner can be resolved into the optical antipodes by known methods, for example by recrystallization from an optically active solvent, by chromatography on chiral adsorbents, for example high-performance liquid chromatography (HPLC) on acetyl cellulose, with the aid of suitable microorganisms, by cleavage with specific, immobilized enzymes, via the formation of inclusion compounds, for example using chiral crown ethers, where only one enantiomer is complexed, or by conversion into diastereomeric salts, for example by reacting a basic end-product racemate with an optically active acid, such as a carboxylic acid, for example camphor, tartaric or malic acid, or sulfonic acid, for example camphorsulfonic acid, and separating the diastereomer mixture which can be obtained in this manner, for example by fractional crystallization based on their differing solubilities, to give the diastereomers, from which the desired enantiomer can be set free by the action of suitable agents, for example basic agents.

Pure diastereomers or enantiomers can be obtained according to the invention not only by separating suitable isomer mixtures, but also by generally known methods of diastereoselective or enantioselective synthesis, for example by carrying out the process according to the invention with starting materials of a suitable stereochemistry.

It is advantageous to isolate or synthesize in each case the biologically more effective isomer, for example enantiomer or diastereomer, or isomer mixture, for example enantiomer mixture or diastereomer mixture, if the individual components have a different biological activity.

The compounds of formula (I) and, where appropriate, the tautomers thereof, in each case in free form or in salt form, can, if appropriate, also be obtained in the form of hydrates and/or include other solvents, for example those which may have been used for the crystallization of compounds which are present in solid form.

The compounds of formula (I) according to the invention are preventively and/or curatively valuable active ingredients in the field of pest control, even at low rates of application, which have a very favorable biocidal spectrum and may be well-tolerated by warm-blooded species, fish and plants. The compounds of formula (I) may have a beneficial safety profile towards non-target species, such as bees, and accordingly a good toxicity profile. The active ingredients according to the invention may act against all or individual developmental stages of normally sensitive, but also resistant pests, such as insects or representatives of the order Acarina. The insecticidal or acaricidal activity of the active ingredients according to the invention can manifest itself directly, i. e. in destruction of the pests, which takes place either immediately or only after some time has elapsed, for example during ecdysis, or indirectly, for example in a reduced oviposition and/or hatching rate.

Examples of the above-mentioned pests are:
from the order *Acarina,* for example,
Acalitus spp., Aculus spp., Acaricalus spp., Aceria spp., Acarus siro, Amblyomma spp., Argas spp., Boophilus spp., Brevipalpus spp., Bryobia spp., Calipitrimerus spp., Chorioptes spp., Dermanyssus gallinae, Dermatophagoides spp., Eotetranychus spp., Eriophyes spp., Hemitarsonemus spp., Hyalomma spp., Ixodes spp., Olygonychus spp., Ornithodoros spp., Polyphagotarsone latus, Panonychus spp., Phyllocoptruta oleivora, Phytonemus spp., Polyphagotarsonemus spp., Psoroptes spp., Rhipicephalus spp., Rhizoglyphus spp., Sarcoptes spp., Steneotarsonemus spp., Tarsonemus spp. and Tetranychus spp.,
from the order *Anoplura,* for example,
Haematopinus spp., Linognathus spp., Pediculus spp., Pemphigus spp. and Phylloxera spp.,
from the order *Coleoptera,* for example,
Agriotes spp., Amphimallon majale, Anomala orientalis, Anthonomus spp., Aphodius spp., Astylus atromaculatus, Ataenius spp., Atomaria linearis, Chaetocnema tibialis, Cerotoma spp., Conoderus spp., Cosmopolites spp., Cotinis nitida, Curculio spp., Cyclocephala spp., Dermestes spp., Diabrotica spp., Diloboderus abderus, Epilachna spp., Eremnus spp., Heteronychus arator, Hypothenemus hampei, Lagria vilosa, Leptinotarsa decemLineata, Lissorhoptrus spp., Liogenys spp., Maecolaspis spp., Maladera castanea, Megascelis spp., Melighetes aeneus, Melolontha spp., Myochrous armatus, Orycaephilus spp., Otiorhynchus spp., Phyllophaga spp., Phlyctinus spp., Popillia spp., Psylliodes spp., Rhyssomatus aubtilis, Rhizopertha spp., Scarabeidae, Sitophilus spp., Sitotroga spp., Somaticus spp., Sphenophorus spp., Sternechus subsignatus, Tenebrio spp., Tribolium spp. and Trogoderma spp.,
from the order *Diptera,* for example,
Aedes spp., Anopheles spp., Antherigona soccata,_Bactrocea oleae, Bibio hortulanus, Bradysia spp., Calliphora erythrocephala, Ceratitis spp., Chrysomyia spp., Culex spp., Cuterebra spp., Dacus spp., Delia spp., Drosophila melanogaster, Fannia spp., Gastrophilus spp., Geomyza tripunctata, Glossina spp., Hypoderma spp., Hyppobosca spp., Liriomyza spp., Lucilia spp., Melanagromyza spp., Musca spp., Oestrus spp., Orseolia spp., Oscinella frit, Pegomyia hyoscyami, Phorbia spp., Rhagoletis spp., Rivelia quadrifasciata, Scatella spp., Sciara spp., Stomoxys spp., Tabanus spp., Tannia spp. and Tipula spp.,
from the order *Hemiptera,* for example,
Acanthocoris scabrator, Acrosternum spp., Adelphocoris lineolatus, Amblypelta nitida, Bathycoelia thalassina, Blissus spp., Cimex spp., Clavigralla tomentosicollis, Creontiades spp., Distantiella theobroma, Dichelops furcatus, Dysdercus spp., Edessa spp., Euchistus spp., Euηdema pulchrum, Eurygaster spp., *Euschistus* spp. (stinkbugs), Halyomorpha halys, Horcias nobilellus, Leptocorisa spp., Lygus spp., Margarodes spp., Murgantia histrionic, Neomegalotomus spp., Nesidiocoris tenuis, Nezara spp., Nysius simulans, Oebalus insularis, Piesma spp., Piezodorus spp., Rhodnius spp., Sahlbergella singularis, Scaptocoris castanea, Scotinophara spp., Thyanta spp., Triatoma spp., Vatiga illudens,
Acyrthosium pisum, Adalges spp., Agalliana ensigera, Agonoscena targionii, Aleurodicus spp., Aleurocanthus spp., Aleurolobus barodensis, Aleurothrixus floccosus, Aleyrodes brassicae, Amarasca biguttula, Amritodus atkinsoni, Aonidiella spp., Aphididae, Aphis spp., Aspidiotus spp., Aulacorthum solani, Bactericera cockerelli, Bemisia spp., Brachycaudus spp., Brevicoryne brassicae, Cacopsylla spp., Cavariella aegopodii Scop., Ceroplaster spp., Chrysomphalus aonidium, Chrysomphalus dictyospermi, Cicadella spp., Cofana spectra, Cryptomyzus spp., Cicadulina spp., Coccus hesperidum, Dalbulus maidis, Dialeurodes spp., Diaphorina citri, Diuraphis noxia, Dysaphis spp., Empoasca spp., Eriosoma larigerum, Erythroneura spp., Gascardia spp., Glycaspis brimblecombei, Hyadaphis pseudobrassicae, Hyalopterus spp., Hyperomyzus pallidus, Idioscopus clypealis, Jacobiasca lybica, Laodelphax spp., Lecanium corni, Lepidosaphes spp., Lopaphis erysimi, Lyogenys maidis, Macrosiphum spp., Mahanarva spp., Metcalfa pruinosa, Metopolophium dirhodum, Myndus crudus, Myzus spp., Neotoxoptera sp, Nephotettix spp., Nilaparvata spp., Nippolachnus piri Mats, Odonaspis ruthae, Oregma lanigera Zehnter, Parabemisia myricae, Paratrioza cockerelli, Parlatoria spp., Pemphigus spp., Peregrinus maidis, Perkinsiella spp., Phorodon humuli, Phylloxera spp., Pianococcus spp., Pseudaulacaspis spp., Pseudococcus spp., Pseudatomoscelis seriatus, Psylla spp., Pulvinaria aethiopica, Quadraspidiotus spp., Quesada gigas, Recilia dorsalis, Rhopalosiphum spp., Saissetia spp., Scaphoideus spp., Schizaphis spp., Sitobion spp., Sogatella furcifera, Spissistilus festinus, Tarophagus Proserpina, Toxoptera spp., Trialeurodes spp., Tridiscus sporoboli, Trionymus spp., Trioza erytreae Unaspis citri, Zygina flammigera, Zyginidia scutellaris,
from the order *Hymenoptera,* for example,
Acromyrmex, Arge spp., Atta spp., Cephus spp., Diprion spp., Diprionidae, Gilpinia polytoma, Hoplocampa spp., Lasius spp., Monomorium pharaonis, Neodiprion spp., Pogonomyrmex spp., Slenopsis invicta, Solenopsis spp. and Vespa spp.,
from the order *Isoptera,* for example,
Coptotermes spp., Corniternes cumulans, Incisitermes spp., Macrotermes spp., Mastotermes spp., Microtermes spp., Reticulitermes spp., Solenopsis geminate
from the order *Lepidoptera,* for example,
Acleris spp., Adoxophyes spp., Aegeria spp., Agrotis spp., Alabama argillaceae, Amylois spp., Anticarsia gemmatalis, Archips spp., Argyresthia spp., Argyrotaenia spp., Autographa spp., Bucculatrix thurberiella, Busseola fusca, Cadra cautella, Carposina nipponensis, Chilo spp., Choristoneura spp., Chrysoteuchia topiaria, Clysia ambiguella, Cnaphalocrocis spp., Cnephasia spp., Cochylis spp., Coleophora spp., Colias lesbia, Cosmophila flava, Crambus spp., Crocidolomia binotalis, Cryptophlebia leucotreta, Cydalima perspectalis, Cydia spp., Diaphania perspectalis, Diatraea spp., Diparopsis castanea, Earias spp., Eldana saccharina, Ephestia spp., Epinotia spp., Estigmene acrea, Etiella zinckinella, Eucosma spp., Eupoecilia ambiguella, Euproctis spp., Euxoa spp., Feltia jaculiferia, Grapholita spp., Hedya nubiferana, Heliothis spp., Hellula undalis, Herpetogramma spp., Hyphantria cunea, Keiferia lycopersicella, Lasmopalpus lignosellus, Leucoptera scitella, Lithocollethis spp., Lobesia botrana, Loxostege bifidalis, Lymantria spp., Lyonetia spp., Malacosoma spp., Mamestra brassicae, Manduca sexta, Mythimna spp., Noctua spp., Operophtera spp., Orniodes indica, Ostrinia nubilalis, Pammene spp., Pandemis spp., Panolis flammea, Papaipema nebris, Pectinophora gossypiela, Perileucoptera coffeella, Pseudaletia unipuncta, Phthorimaea operculella, Pieris rapae, Pieris spp., Plutella xylostella, Prays spp., Pseudoplusia spp., Rachiplusia nu, Richia albicosta, Scirpophaga spp., Sesamia spp., Sparganothis spp., Spodoptera spp., Sylepta derogate, Synanthedon spp., Thaumetopoea spp., Tortrix spp., Trichoplusia ni, Tuta absoluta, and Yponomeuta spp.,
from the order *Mallophaga*, for example,
Damalinea spp. and Trichodectes spp.,
from the order *Orthoptera,* for example,
Blatta spp., Blattella spp., Gryllotalpa spp., Leucophaea maderae, Locusta spp., Neocurtilla hexadactyla, Periplaneta spp., Scapteriscus spp., and Schistocerca spp.,
from the order *Psocoptera,* for example,
Liposcelis spp.,
from the order *Siphonaptera*, for example,
Ceratophyllus spp., Ctenocephalides spp. and Xenopsylla cheopis,
from the order *Thysanoptera,* for example,
Calliothrips phaseoli, Frankliniella spp., Heliothrips spp., Hercinothrips spp., Parthenothrips spp., Scirtothrips aurantii, Sericothrips variabilis, Taeniothrips spp., Thrips spp.,
from the order *Thysanura,* for example, Lepisma saccharina.

The active ingredients according to the invention can be used for controlling, i. e. containing or destroying, pests of the abovementioned type which occur in particular on plants, especially on useful plants and ornamentals in agriculture, in horticulture and in forests, or on organs, such as fruits, flowers, foliage, stalks, tubers or roots, of such plants, and in some cases even plant organs which are formed at a later point in time remain protected against these pests.

Suitable target crops are, in particular, cereals, such as wheat, barley, rye, oats, rice, maize or sorghum, beet, such as sugar or fodder beet, fruit, for example pomaceous fruit, stone fruit or soft fruit, such as apples, pears, plums, peaches, almonds, cherries or berries, for example strawberries, raspberries or blackberries, leguminous crops, such as beans, lentils, peas or soya, oil crops, such as oilseed rape, mustard, poppies, olives, sunflowers, coconut, castor, cocoa or ground nuts, cucurbits, such as pumpkins, cucumbers or melons, fibre plants, such as cotton, flax, hemp or jute, citrus fruit, such as oranges, lemons, grapefruit or tangerines, vegetables, such as spinach, lettuce, asparagus, cabbages, carrots, onions, tomatoes, potatoes or bell peppers, Lauraceae, such as avocado, Cinnamonium or camphor, and also tobacco, nuts, coffee, eggplants, sugarcane, tea, pepper, grapevines, hops, the plantain family, latex plants and ornamentals.

The active ingredients according to the invention may especially be suitable for controlling Aphis craccivora, Diabrotica balteata, Thrips tabaci, Euschistus heros, Heliothis virescens, Myzus persicae, Plutella xylostella and Spodoptera littoralis in cotton, vegetable, maize, rice and soya crops. The active ingredients according to the invention are further especially suitable for controlling Mamestra (preferably in vegetables), Cydia pomonella (preferably in apples), Empoasca (preferably in vegetables, vineyards), Leptinotarsa (preferably in potatos) and Chilo supressalis (preferably in rice).

The term "crops" is to be understood as including also crop plants which have been so transformed by the use of recombinant DNA techniques that they are capable of synthesising one or more selectively acting toxins, such as are known, for example, from toxin-producing bacteria, especially those of the genus Bacillus.

Toxins that can be expressed by such transgenic plants include, for example, insecticidal proteins, for example insecticidal proteins from Bacillus cereus or Bacillus popilliae, or insecticidal proteins from Bacillus thuringiensis, such as δ-endotoxins, e.g. Cry1Ab, Cry1Ac, Cry1F, Cry1Fa2, Cry2Ab, Cry3A, Cry3Bb1 or Cry9C, or vegetative insecticidal proteins (Vip), e.g. Vip1, Vip2, Vip3 or Vip3A, or insecticidal proteins of bacteria colonising nematodes, for example Photorhabdus spp. or Xenorhabdus spp., such as Photorhabdus luminescens, Xenorhabdus nematophilus, toxins produced by animals, such as scorpion toxins, arachnid toxins, wasp toxins and other insect-specific neurotoxins, toxins produced by fungi, such as Streptomycetes toxins, plant lectins, such as pea lectins, barley lectins or snowdrop lectins, agglutinins, proteinase inhibitors, such as trypsin inhibitors, serine protease inhibitors, patatin, cystatin, papain inhibitors, ribosome-inactivating proteins (RIP), such as ricin, maize-RIP, abrin, luffin, saporin or bryodin, steroid metabolism enzymes, such as 3-hydroxysteroidoxidase, ecdysteroid-UDP-glycosyl-transferase, cholesterol oxidases, ecdysone inhibitors, HMG-COA-reductase, ion channel blockers, such as blockers of sodium or calcium channels, juvenile hormone esterase, diuretic hormone receptors, stilbene synthase, bibenzyl synthase, chitinases and glucanases.

In the context of the present invention there are to be understood by δ-endotoxins, for example Cry1Ab, Cry1Ac, Cry1F, Cry1Fa2, Cry2Ab, Cry3A, Cry3Bb1 or Cry9C, or vegetative insecticidal proteins (Vip), for example Vip1, Vip2, Vip3 or Vip3A, expressly also hybrid toxins, truncated toxins and modified toxins. Hybrid toxins are produced recombinantly by a new combination of different domains of those proteins (see, for example, WO 02/15701). Truncated toxins, for example a truncated Cry1Ab, are known. In the case of modified toxins, one or more amino acids of the naturally occurring toxin are replaced. In such amino acid replacements, preferably non-naturally present protease recognition sequences are inserted into the toxin, such as, for example, in the case of Cry3A055, a cathepsin-G-recognition sequence is inserted into a Cry3A toxin (see WO 03/018810).

Examples of such toxins or transgenic plants capable of synthesizing such toxins are disclosed, for example, in EP-A-0 374 753, WO 93/07278, WO 95/34656, EP-A-0 427 529, EP-A-451 878 and WO 03/052073.

The processes for the preparation of such transgenic plants are generally known to the person skilled in the art and are described, for example, in the publications mentioned above. Cryl-type deoxyribonucleic acids and their preparation are known, for example, from WO 95/34656, EP-A-0 367 474, EP-A-0 401 979 and WO 90/13651.

The toxin contained in the transgenic plants imparts to the plants tolerance to harmful insects. Such insects can occur in any taxonomic group of insects, but are especially commonly found in the beetles (Coleoptera), two-winged insects (Diptera) and moths (Lepidoptera).

Transgenic plants containing one or more genes that code for an insecticidal resistance and express one or more toxins are known and some of them are commercially available. Examples of such plants are: YieldGard^{®} (maize variety that expresses a Cry1Ab toxin), YieldGard Rootworm^{®} (maize variety that expresses a Cry3Bb1 toxin), YieldGard Plus^{®} (maize variety that expresses a Cry1Ab and a Cry3Bb1 toxin), Starlink^{®} (maize variety that expresses a Cry9C toxin), Herculex I^{®} (maize variety that expresses a Cry1Fa2 toxin and the enzyme phosphinothricine N-acetyltransferase (PAT) to achieve tolerance to the herbicide glufosinate ammonium), NuCOTN 33B^{®} (cotton variety that expresses a Cry1Ac toxin), Bollgard I^{®} (cotton variety that expresses a Cry1Ac toxin), Bollgard II^{®} (cotton variety that expresses a Cry1Ac and a Cry2Ab toxin), VipCot^{®} (cotton variety that expresses a Vip3A and a Cry1Ab toxin), NewLeaf^{®} (potato variety that expresses a Cry3A toxin), NatureGard^{®}, Agrisure^{®} GT Advantage (GA21 glyphosate-tolerant trait), Agrisure^{®} CB Advantage (Bt11 corn borer (CB) trait) and Protecta^{®}.

Further examples of such transgenic crops are:
1. **Bt11 Maize** from Syngenta Seeds SAS, Chemin de l'Hobit 27, F-31 790 St. Sauveur, France, registration number C/FR/96/05/10. Genetically modified *Zea mays* which has been rendered resistant to attack by the European corn borer (*Ostrinia nubilalis* and *Sesamia nonagrioides*) by transgenic expression of a truncated Cry1Ab toxin. Bt11 maize also transgenically expresses the enzyme PAT to achieve tolerance to the herbicide glufosinate ammonium.
2. **Bt176 Maize** from Syngenta Seeds SAS, Chemin de l'Hobit 27, F-31 790 St. Sauveur, France, registration number C/FR/96/05/10. Genetically modified *Zea mays* which has been rendered resistant to attack by the European corn borer (*Ostrinia nubilalis* and *Sesamia nonagrioides*) by transgenic expression of a Cry1Ab toxin. Bt176 maize also transgenically expresses the enzyme PAT to achieve tolerance to the herbicide glufosinate ammonium.
3. **MIR604 Maize** from Syngenta Seeds SAS, Chemin de l'Hobit 27, F-31 790 St. Sauveur, France, registration number C/FR/96/05/10. Maize which has been rendered insect-resistant by transgenic expression of a modified Cry3A toxin. This toxin is Cry3A055 modified by insertion of a cathepsin-G-protease recognition sequence. The preparation of such transgenic maize plants is described in WO 03/018810.
4. **MON 863 Maize** from Monsanto Europe S.A. 270-272 Avenue de Tervuren, B-1150 Brussels, Belgium, registration number C/DE/02/9. MON 863 expresses a Cry3Bb1 toxin and has resistance to certain Coleoptera insects.
5. **IPC 531 Cotton** from Monsanto Europe S.A. 270-272 Avenue de Tervuren, B-1150 Brussels, Belgium, registration number C/ES/96/02.
**6. 1507 Maize** from Pioneer Overseas Corporation, Avenue Tedesco, 7 B-1160 Brussels, Belgium, registration number C/NL/00/10. Genetically modified maize for the expression of the protein Cry1F for achieving resistance to certain Lepidoptera insects and of the PAT protein for achieving tolerance to the herbicide glufosinate ammonium.
7. **NK603** × **MON 810 Maize** from Monsanto Europe S.A. 270-272 Avenue de Tervuren, B-1150 Brussels, Belgium, registration number C/GB/02/M3/03. Consists of conventionally bred hybrid maize varieties by crossing the genetically modified varieties NK603 and MON 810. NK603 × MON 810 Maize transgenically expresses the protein CP4 EPSPS, obtained from *Agrobacterium sp.* strain CP4, which imparts tolerance to the herbicide Roundup^{®} (contains glyphosate), and also a Cry1Ab toxin obtained from *Bacillus thuringiensis subsp. kurstaki* which brings about tolerance to certain Lepidoptera, include the European corn borer.

Transgenic crops of insect-resistant plants are also described in BATS (Zentrum für Biosicherheit und Nachhaltigkeit, Zentrum BATS, Clarastrasse 13, 4058 Basel, Switzerland) Report 2003, (http://bats.ch).

The term "crops" is to be understood as including also crop plants which have been so transformed by the use of recombinant DNA techniques that they are capable of synthesising antipathogenic substances having a selective action, such as, for example, the so-called "pathogenesis-related proteins" (PRPs, see e.g. EP-A-0 392 225). Examples of such antipathogenic substances and transgenic plants capable of synthesising such antipathogenic substances are known, for example, from EP-A-0 392 225, WO 95/33818 and EP-A-0 353 191. The methods of producing such transgenic plants are generally known to the person skilled in the art and are described, for example, in the publications mentioned above.

Crops may also be modified for enhanced resistance to fungal (for example Fusarium, Anthracnose, or Phytophthora), bacterial (for example Pseudomonas) or viral (for example potato leafroll virus, tomato spotted wilt virus, cucumber mosaic virus) pathogens.

Crops also include those that have enhanced resistance to nematodes, such as the soybean cyst nematode.

Crops that are tolerance to abiotic stress include those that have enhanced tolerance to drought, high salt, high temperature, chill, frost, or light radiation, for example through expression of NF-YB or other proteins known in the art.

Antipathogenic substances which can be expressed by such transgenic plants include, for example, ion channel blockers, such as blockers for sodium and calcium channels, for example the viral KP1, KP4 or KP6 toxins, stilbene synthases, bibenzyl synthases, chitinases, glucanases, the so-called "pathogenesis-related proteins" (PRPs, see e.g. EP-A-0 392 225), antipathogenic substances produced by microorganisms, for example peptide antibiotics or heterocyclic antibiotics (see e.g. WO 95/33818) or protein or polypeptide factors involved in plant pathogen defence (so-called "plant disease resistance genes", as described in WO 03/000906).

Further areas of use of the compositions according to the invention are the protection of stored goods and store ambients and the protection of raw materials, such as wood, textiles, floor coverings or buildings, and also in the hygiene sector, especially the protection of humans, domestic animals and productive livestock against pests of the mentioned type.

The present invention also provides a method for controlling pests (such as mosquitoes and other disease vectors, see also http://www.who.int/malaria/vector_control/irs/en/). In one embodiment, the method for controlling pests comprises applying the compositions of the invention to the target pests, to their locus or to a surface or substrate by brushing, rolling, spraying, spreading or dipping. By way of example, an IRS (indoor residual spraying) application of a surface such as a wall, ceiling or floor surface is contemplated by the method of the invention. In another embodiment, it is contemplated to apply such compositions to a substrate such as non-woven or a fabric material in the form of (or which can be used in the manufacture of) netting, clothing, bedding, curtains and tents. A further object of the invention is therefore a substrate selected from nonwoven and fabric material comprising a composition which contains a compound of formula I.

In one embodiment, the method for controlling such pests comprises applying a pesticidally effective amount of the compositions of the invention to the target pests, to their locus, or to a surface or substrate so as to provide effective residual pesticidal activity on the surface or substrate. Such application may be made by brushing, rolling, spraying, spreading or dipping the pesticidal composition of the invention. By way of example, an IRS application of a surface such as a wall, ceiling or floor surface is contemplated by the method of the invention so as to provide effective residual pesticidal activity on the surface. In another embodiment, it is contemplated to apply such compositions for residual control of pests on a substrate such as a fabric material in the form of (or which can be used in the manufacture of) netting, clothing, bedding, curtains and tents.

Substrates including non-woven, fabrics or netting to be treated may be made of natural fibres such as cotton, raffia, jute, flax, sisal, hessian, or wool, or synthetic fibres such as polyamide, polyester, polypropylene, polyacrylonitrile or the like. The polyesters are particularly suitable. The methods of textile treatment are known, e.g. WO 2008/151984, WO 03/034823, US 5631072, WO 2005/64072, WO 2006/128870, EP 1 724 392, WO 2005/113886 or WO 2007/090739.

Further areas of use of the compositions according to the invention are the field of tree injection/trunk treatment for all ornamental trees as well all sort of fruit and nut trees.

In the field of tree injection/trunk treatment, the compounds according to the present invention are especially suitable against wood-boring insects from the order *Lepidoptera* as mentioned above and from the order *Coleoptera,* especially against woodborers listed in the following Table:

### Examples of exotic woodborers of economic importance.

| Family | Species | Host or Crop Infested |
|---|---|---|
| Buprestidae | *Agrilus planipennis* | Ash |
| Cerambycidae | *Anoplura glabripennis* | Hardwoods |
| Scolytidae | *Xylosandrus crassiusculus* | Hardwoods |
| | *X. mutilatus* | Hardwoods |
| | *Tomicus piniperda* | Conifers |

**Table B. Examples of native woodborers of economic importance.**

| Family | Species | Host or Crop Infested |
|---|---|---|
| Buprestidae | *Agrilus anxius* | Birch |
| | *Agrilus politus* | Willow, Maple |
| | *Agrilus sayi* | Bayberry, Sweetfern |
| | *Agrilus vittaticolllis* | Apple, Pear, Cranberry, Serviceberry, Hawthorn |
| | *Chrysobothris femorata* | Apple, Apricot, Beech, Boxelder, Cherry, Chestnut, Currant, Elm, Hawthorn, Hackberry, Hickory, Horsechestnut, Linden, Maple, Mountain-ash, Oak, Pecan, Pear, Peach, Persimmon, Plum, Poplar, Quince, Redbud, Serviceberry, Sycamore, Walnut, Willow |
| | *Texania campestris* | Basswood, Beech, Maple, Oak, Sycamore, Willow, Yellow-poplar |
| | *Goes pulverulentus* | Beech, Elm, Nuttall, Willow, Black oak, Cherrybark oak, Water oak, Sycamore |
| | *Goes tigrinus* | Oak |
| | *Neoclytus acuminatus* | Ash, Hickory, Oak, Walnut, Birch, Beech, Maple, Eastern hophornbeam, Dogwood, Persimmon, Redbud, Holly, Hackberry, Black locust, Honeylocust, Yellow-poplar, Chestnut, Osage-orange, Sassafras, Lilac, Mountain-mahogany, Pear, Cherry, Plum, Peach, Apple, Elm, Basswood, Sweetgum |
| Cerambycidae | *Neoptychodes trilineatus* | Fig, Alder, Mulberry, Willow, Netleaf hackberry |
| | *Oberea ocellata* | Sumac, Apple, Peach, Plum, Pear, Currant, Blackberry |
| | *Oberea tripunctata* | Dogwood, Viburnum, Elm, Sourwood, Blueberry, Rhododendron, Azalea, Laurel, Poplar, Willow, Mulberry |
| | *Oncideres cingulata* | Hickory, Pecan, Persimmon, Elm, Sourwood, Basswood, Honeylocust, Dogwood, Eucalyptus, Oak, Hackberry, Maple, Fruit trees |
| | *Saperda calcarata* | Poplar |
| | *Strophiona nitens* | Chestnut, Oak, Hickory, Walnut, Beech, Maple |
| Scolytidae | *Corthylus columbianus* | Maple, Oak, Yellow-poplar, Beech, Boxelder, Sycamore, Birch, Basswood, Chestnut, Elm |
| | *Dendroctonus frontalis* | Pine |
| | *Dryocoetes betulae* | Birch, Sweetgum, Wild cherry, Beech, Pear |
| | *Monarthrum fasciatum* | Oak, Maple, Birch, Chestnut, Sweetgum, Blackgum, Poplar, Hickory, Mimosa, Apple, Peach, Pine |
| | *Phloeotribus liminaris* | Peach, Cherry, Plum, Black cherry, Elm, Mulberry, Mountain-ash |
| | *Pseudopityophthorus pruinosus* | Oak, American beech, Black cherry, Chickasaw plum, Chestnut, Maple, Hickory, Hornbeam, Hophornbeam |
| Sesiidae | *Paranthrene simulans* | Oak, American chestnut |
| | *Sannina uroceriformis* | Persimmon |
| | *Synanthedon exitiosa* | Peach, Plum, Nectarine, Cherry, Apricot, Almond, Black cherry |
| | *Synanthedon pictipes* | Peach, Plum, Cherry, Beach, Black Cherry |
| | *Synanthedon rubrofascia* | Tupelo |
| | *Synanthedon scitula* | Dogwood, Pecan, Hickory, Oak, Chestnut, Beech, Birch, Black cherry, Elm, Mountain-ash, Viburnum, Willow, Apple, Loquat, Ninebark, Bayberry |
| | *Vitacea polistiformis* | Grape |

The present invention may be also used to control any insect pests that may be present in turfgrass, including for example beetles, caterpillars, fire ants, ground pearls, millipedes, sow bugs, mites, mole crickets, scales, mealybugs ticks, spittlebugs, southern chinch bugs and white grubs. The present invention may be used to control insect pests at various stages of their life cycle, including eggs, larvae, nymphs and adults.

In particular, the present invention may be used to control insect pests that feed on the roots of turfgrass including white grubs (such as *Cyclocephala spp.* (e.g. masked chafer, C. *lurida*), *Rhizotrogus spp.* (e.g. European chafer, *R. majalis*), *Cotinus spp.* (e.g. Green June beetle, C. *nitida*), *Popillia spp.* (e.g. Japanese beetle, *P. japonica*), *Phyllophaga spp.* (e.g. May/June beetle), *Ataenius spp.* (e.g. Black turfgrass ataenius, *A. spretulus), Maladera spp.* (e.g. Asiatic garden beetle, *M. castanea*) and *Tomarus spp*.), ground pearls (*Margarodes* spp.), mole crickets (tawny, southern, and short-winged, *Scapteriscus* spp., *Gryllotalpa africana*) and leatherjackets (European crane fly, *Tipula spp*.).

The present invention may also be used to control insect pests of turfgrass that are thatch dwelling, including armyworms (such as fall armyworm *Spodoptera frugiperda,* and common armyworm *Pseudaletia unipuncta*), cutworms, billbugs (*Sphenophorus spp.,* such as S. *venatus verstitus* and S. *parvulus*), and sod webworms (such as *Crambus spp.* and the tropical sod webworm, *Herpetogramma phaeopteralis*).

The present invention may also be used to control insect pests of turfgrass that live above the ground and feed on the turfgrass leaves, including chinch bugs (such as southern chinch bugs, *Blissus insularis*), Bermudagrass mite (*Eriophyes cynodoniensis*), rhodesgrass mealybug (*Antonina graminis*), two-lined spittlebug (*Propsapia bicincta*), leafhoppers, cutworms (*Noctuidae* family), and greenbugs.

The present invention may also be used to control other pests of turfgrass such as red imported fire ants (*Solenopsis invicta*) that create ant mounds in turf.

In the hygiene sector, the compositions according to the invention are active against ectoparasites such as hard ticks, soft ticks, mange mites, harvest mites, flies (biting and licking), parasitic fly larvae, lice, hair lice, bird lice and fleas.

Examples of such parasites are:
Of the order Anoplurida: Haematopinus spp., Linognathus spp., Pediculus spp. and Phtirus spp., Solenopotes spp.,
Of the order Mallophagida: Trimenopon spp., Menopon spp., Trinoton spp., Bovicola spp., Werneckiella spp., Lepikentron spp., Damalina spp., Trichodectes spp. and Felicola spp.,
Of the order Diptera and the suborders Nematocerina and Brachycerina, for example Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp. and Melophagus spp.,
Of the order Siphonapterida, for example Pulex spp., Ctenocephalides spp., Xenopsylla spp., Ceratophyllus spp.,
Of the order Heteropterida, for example Cimex spp., Triatoma spp., Rhodnius spp., Panstrongylus spp.,
Of the order Blattarida, for example Blatta orientalis, Periplaneta americana, Blattelagermanica and Supella spp.,
Of the subclass Acaria (Acarida) and the orders Meta- and Meso-stigmata, for example Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp. and Varroa spp.,
Of the orders Actinedida (Prostigmata) and Acaridida (Astigmata), for example Acarapis spp., Cheyletiella spp., Ornithocheyletia spp., Myobia spp., Psorergatesspp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp. and Laminosioptes spp..

The compositions according to the invention are also suitable for protecting against insect infestation in the case of materials such as wood, textiles, plastics, adhesives, glues, paints, paper and card, leather, floor coverings and buildings.

The compositions according to the invention can be used, for example, against the following pests: beetles such as Hylotrupes bajulus, Chlorophorus pilosis, Anobium punctatum, Xestobium rufovillosum, Ptilinuspecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthesrugicollis, Xyleborus spec.,Tryptodendron spec., Apate monachus, Bostrychus capucins, Heterobostrychus brunneus, Sinoxylon spec. and Dinoderus minutus, and also hymenopterans such as Sirex juvencus, Urocerus gigas, Urocerus gigas taignus and Urocerus augur, and termites such as Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis and Coptotermes formosanus, and bristletails such as Lepisma saccharina.

The compounds according to the invention can be used as pesticidal agents in unmodified form, but they are generally formulated into compositions in various ways using formulation adjuvants or addditives, such as carriers, solvents and surface-active substances. The formulations can be in various physical forms, e.g. in the form of dusting powders, gels, wettable powders, water-dispersible granules, water-dispersible tablets, effervescent pellets, emulsifiable concentrates, microemulsifiable concentrates, oil-in-water emulsions, oil-flowables, aqueous dispersions, oily dispersions, suspo-emulsions, capsule suspensions, emulsifiable granules, soluble liquids, water-soluble concentrates (with water or a water-miscible organic solvent as carrier), impregnated polymer films or in other forms known e.g. from the Manual on Development and Use of FAO and WHO Specifications for Pesticides, United Nations, First Edition, Second Revision (2010). Such formulations can either be used directly or diluted prior to use. The dilutions can be made, for example, with water, liquid fertilisers, micronutrients, biological organisms, oil or solvents.

The formulations can be prepared e.g. by mixing the active ingredient with the formulation adjuvants in order to obtain compositions in the form of finely divided solids, granules, solutions, dispersions or emulsions. The active ingredients can also be formulated with other adjuvants, such as finely divided solids, mineral oils, oils of vegetable or animal origin, modified oils of vegetable or animal origin, organic solvents, water, surface-active substances or combinations thereof.

The active ingredients can also be contained in very fine microcapsules. Microcapsules contain the active ingredients in a porous carrier. This enables the active ingredients to be released into the environment in controlled amounts (e.g. slow-release). Microcapsules usually have a diameter of from 0.1 to 500 microns. They contain active ingredients in an amount of about from 25 to 95 % by weight of the capsule weight. The active ingredients can be in the form of a monolithic solid, in the form of fine particles in solid or liquid dispersion or in the form of a suitable solution. The encapsulating membranes can comprise, for example, natural or synthetic rubbers, cellulose, styrene/butadiene copolymers, polyacrylonitrile, polyacrylate, polyesters, polyamides, polyureas, polyurethane or chemically modified polymers and starch xanthates or other polymers that are known to the person skilled in the art. Alternatively, very fine microcapsules can be formed in which the active ingredient is contained in the form of finely divided particles in a solid matrix of base substance, but the microcapsules are not themselves encapsulated.

The formulation adjuvants that are suitable for the preparation of the compositions according to the invention are known *per se.* As liquid carriers there may be used: water, toluene, xylene, petroleum ether, vegetable oils, acetone, methyl ethyl ketone, cyclohexanone, acid anhydrides, acetonitrile, acetophenone, amyl acetate, 2-butanone, butylene carbonate, chlorobenzene, cyclohexane, cyclohexanol, alkyl esters of acetic acid, diacetone alcohol, 1,2-dichloropropane, diethanolamine, p-diethylbenzene, diethylene glycol, diethylene glycol abietate, diethylene glycol butyl ether, diethylene glycol ethyl ether, diethylene glycol methyl ether, *N,N*-dimethylformamide, dimethyl sulfoxide, 1,4-dioxane, dipropylene glycol, dipropylene glycol methyl ether, dipropylene glycol dibenzoate, diproxitol, alkylpyrrolidone, ethyl acetate, 2-ethylhexanol, ethylene carbonate, 1,1,1-trichloroethane, 2-heptanone, alpha-pinene, d-limonene, ethyl lactate, ethylene glycol, ethylene glycol butyl ether, ethylene glycol methyl ether, gamma-butyrolactone, glycerol, glycerol acetate, glycerol diacetate, glycerol triacetate, hexadecane, hexylene glycol, isoamyl acetate, isobornyl acetate, isooctane, isophorone, isopropylbenzene, isopropyl myristate, lactic acid, laurylamine, mesityl oxide, methoxypropanol, methyl isoamyl ketone, methyl isobutyl ketone, methyl laurate, methyl octanoate, methyl oleate, methylene chloride, m-xylene, n-hexane, n-octylamine, octadecanoic acid, octylamine acetate, oleic acid, oleylamine, o-xylene, phenol, polyethylene glycol, propionic acid, propyl lactate, propylene carbonate, propylene glycol, propylene glycol methyl ether, p-xylene, toluene, triethyl phosphate, triethylene glycol, xylenesulfonic acid, paraffin, mineral oil, trichloroethylene, perchloroethylene, ethyl acetate, amyl acetate, butyl acetate, propylene glycol methyl ether, diethylene glycol methyl ether, methanol, ethanol, isopropanol, and alcohols of higher molecular weight, such as amyl alcohol, tetrahydrofurfuryl alcohol, hexanol, octanol, ethylene glycol, propylene glycol, glycerol, N-methyl-2-pyrrolidone and the like.

Suitable solid carriers are, for example, talc, titanium dioxide, pyrophyllite clay, silica, attapulgite clay, kieselguhr, limestone, calcium carbonate, bentonite, calcium montmorillonite, cottonseed husks, wheat flour, soybean flour, pumice, wood flour, ground walnut shells, lignin and similar substances.

A large number of surface-active substances can advantageously be used in both solid and liquid formulations, especially in those formulations which can be diluted with a carrier prior to use. Surface-active substances may be anionic, cationic, non-ionic or polymeric and they can be used as emulsifiers, wetting agents or suspending agents or for other purposes. Typical surface-active substances include, for example, salts of alkyl sulfates, such as diethanolammonium lauryl sulfate, salts of alkylarylsulfonates, such as calcium dodecylbenzenesulfonate, alkylphenol/alkylene oxide addition products, such as nonylphenol ethoxylate, alcohol/alkylene oxide addition products, such as tridecylalcohol ethoxylate, soaps, such as sodium stearate, salts of alkylnaphthalenesulfonates, such as sodium dibutylnaphthalenesulfonate, dialkyl esters of sulfosuccinate salts, such as sodium di(2-ethylhexyl)sulfosuccinate, sorbitol esters, such as sorbitol oleate, quaternary amines, such as lauryltrimethylammonium chloride, polyethylene glycol esters of fatty acids, such as polyethylene glycol stearate, block copolymers of ethylene oxide and propylene oxide, and salts of mono- and di-alkylphosphate esters, and also further substances described e.g. in McCutcheon's Detergents and Emulsifiers Annual, MC Publishing Corp., Ridgewood New Jersey (1981).

Further adjuvants that can be used in pesticidal formulations include crystallisation inhibitors, viscosity modifiers, suspending agents, dyes, anti-oxidants, foaming agents, light absorbers, mixing auxiliaries, antifoams, complexing agents, neutralising or pH-modifying substances and buffers, corrosion inhibitors, fragrances, wetting agents, take-up enhancers, micronutrients, plasticisers, glidants, lubricants, dispersants, thickeners, antifreezes, microbicides, and liquid and solid fertilisers.

The compositions according to the invention can include an additive comprising an oil of vegetable or animal origin, a mineral oil, alkyl esters of such oils or mixtures of such oils and oil derivatives. The amount of oil additive in the composition according to the invention is generally from 0.01 to 10 %, based on the mixture to be applied. For example, the oil additive can be added to a spray tank in the desired concentration after a spray mixture has been prepared. Preferred oil additives comprise mineral oils or an oil of vegetable origin, for example rapeseed oil, olive oil or sunflower oil, emulsified vegetable oil, alkyl esters of oils of vegetable origin, for example the methyl derivatives, or an oil of animal origin, such as fish oil or beef tallow. Preferred oil additives comprise alkyl esters of C₈-C₂₂ fatty acids, especially the methyl derivatives of C₁₂-C₁₈ fatty acids, for example the methyl esters of lauric acid, palmitic acid and oleic acid (methyl laurate, methyl palmitate and methyl oleate, respectively). Many oil derivatives are known from the Compendium of Herbicide Adjuvants, 10th Edition, Southern Illinois University, 2010.

The inventive compositions generally comprise from 0.1 to 99 % by weight, especially from 0.1 to 95 % by weight, of compounds of the present invention and from 1 to 99.9 % by weight of a formulation adjuvant which preferably includes from 0 to 25 % by weight of a surface-active substance. Whereas commercial products may preferably be formulated as concentrates, the end user will normally employ dilute formulations.

The rates of application vary within wide limits and depend on the nature of the soil, the method of application, the crop plant, the pest to be controlled, the prevailing climatic conditions, and other factors governed by the method of application, the time of application and the target crop. As a general guideline, compounds may be applied at a rate of from 1 to 2000 l/ha, especially from 10 to 1000 l/ha.

Preferred formulations can have the following compositions (weight %):

### Emulsifiable concentrates:

| | |
|---|---|
| active ingredient: | 1 to 95 %, preferably 60 to 90 % |
| surface-active agent: | 1 to 30 %, preferably 5 to 20 % |
| liquid carrier: | 1 to 80 %, preferably 1 to 35 % |

### Dusts:

| | |
|---|---|
| active ingredient: | 0.1 to 10 %, preferably 0.1 to 5 % |
| solid carrier: | 99.9 to 90 %, preferably 99.9 to 99 % |

### Suspension concentrates:

| | |
|---|---|
| active ingredient: | 5 to 75 %, preferably 10 to 50 % |
| water: | 94 to 24 %, preferably 88 to 30 % |
| surface-active agent: | 1 to 40 %, preferably 2 to 30 % |

### Wettable powders:

| | |
|---|---|
| active ingredient: | 0.5 to 90 %, preferably 1 to 80 % |
| surface-active agent: | 0.5 to 20 %, preferably 1 to 15 % |
| solid carrier: | 5 to 95 %, preferably 15 to 90 % |

### Granules:

| | |
|---|---|
| active ingredient: | 0.1 to 30 %, preferably 0.1 to 15 % |
| solid carrier: | 99.5 to 70 %, preferably 97 to 85 % |

The following Examples further illustrate, but do not limit, the invention.

| Wettable powders | a) | b) | c) |
|---|---|---|---|
| active ingredients | 25 % | 50 % | 75 % |
| sodium lignosulfonate | 5 % | 5 % | - |
| sodium lauryl sulfate | 3 % | - | 5 % |
| sodium diisobutylnaphthalenesulfonate | - | 6 % | 10 % |
| phenol polyethylene glycol ether (7-8 mol of ethylene oxide) | - | 2 % | - |
| highly dispersed silicic acid | 5 % | 10 % | 10 % |
| Kaolin | 62 % | 27 % | |

The combination is thoroughly mixed with the adjuvants and the mixture is thoroughly ground in a suitable mill, affording wettable powders that can be diluted with water to give suspensions of the desired concentration.

| Powders for dry seed treatment | a) | b) | c) |
|---|---|---|---|
| active ingredients | 25 % | 50 % | 75 % |
| light mineral oil | 5 % | 5 % | 5 % |
| highly dispersed silicic acid | 5 % | 5 % | - |
| Kaolin | 65 % | 40 % | - |
| Talcum | - | - | 20% |

The combination is thoroughly mixed with the adjuvants and the mixture is thoroughly ground in a suitable mill, affording powders that can be used directly for seed treatment.

| Emulsifiable concentrate | |
|---|---|
| active ingredients | 10 % |
| octylphenol polyethylene glycol ether (4-5 mol of ethylene oxide) | 3 % |
| calcium dodecylbenzenesulfonate | 3 % |
| castor oil polyglycol ether (35 mol of ethylene oxide) | 4 % |
| Cyclohexanone | 30 % |
| xylene mixture | 50 % |

Emulsions of any required dilution, which can be used in plant protection, can be obtained from this concentrate by dilution with water.

| Dusts | a) | b) | c) |
|---|---|---|---|
| Active ingredients | 5 % | 6 % | 4 % |
| Talcum | 95 % | - | - |
| Kaolin | - | 94 % | - |
| mineral filler | - | - | 96 % |

Ready-for-use dusts are obtained by mixing the combination with the carrier and grinding the mixture in a suitable mill. Such powders can also be used for dry dressings for seed.

| Extruder granules | |
|---|---|
| Active ingredients | 15 % |
| sodium lignosulfonate | 2 % |
| carboxymethylcellulose | 1 % |
| Kaolin | 82 % |

The combination is mixed and ground with the adjuvants, and the mixture is moistened with water. The mixture is extruded and then dried in a stream of air.

| Coated granules | |
|---|---|
| Active ingredients | 8% |
| polyethylene glycol (mol. wt. 200) | 3% |
| Kaolin | 89% |

The finely ground combination is uniformly applied, in a mixer, to the kaolin moistened with polyethylene glycol. Non-dusty coated granules are obtained in this manner.

| Suspension concentrate | |
|---|---|
| active ingredients | 40 % |
| propylene glycol | 10 % |
| nonylphenol polyethylene glycol ether (15 mol of ethylene oxide) | 6 % |
| Sodium lignosulfonate | 10 % |
| carboxymethylcellulose | 1 % |
| silicone oil (in the form of a 75 % emulsion in water) | 1 % |
| Water | 32 % |

The finely ground combination is intimately mixed with the adjuvants, giving a suspension concentrate from which suspensions of any desired dilution can be obtained by dilution with water. Using such dilutions, living plants as well as plant propagation material can be treated and protected against infestation by microorganisms, by spraying, pouring or immersion.

| Flowable concentrate for seed treatment | |
|---|---|
| active ingredients | 40 % |
| propylene glycol | 5 % |
| copolymer butanol PO/EO | 2 % |
| Tristyrenephenole with 10-20 moles EO | 2 % |
| 1,2-benzisothiazolin-3-one (in the form of a 20% solution in water) | 0.5 % |
| monoazo-pigment calcium salt | 5 % |
| Silicone oil (in the form of a 75 % emulsion in water) | 0.2 % |
| Water | 45.3 % |

The finely ground combination is intimately mixed with the adjuvants, giving a suspension concentrate from which suspensions of any desired dilution can be obtained by dilution with water. Using such dilutions, living plants as well as plant propagation material can be treated and protected against infestation by microorganisms, by spraying, pouring or immersion.

### Slow-Release Capsule Suspension

28 parts of the combination are mixed with 2 parts of an aromatic solvent and 7 parts of toluene diisocyanate/polymethylene-polyphenylisocyanate-mixture (8:1). This mixture is emulsified in a mixture of 1.2 parts of polyvinylalcohol, 0.05 parts of a defoamer and 51.6 parts of water until the desired particle size is achieved. To this emulsion a mixture of 2.8 parts 1,6-diaminohexane in 5.3 parts of water is added. The mixture is agitated until the polymerization reaction is completed. The obtained capsule suspension is stabilized by adding 0.25 parts of a thickener and 3 parts of a dispersing agent. The capsule suspension formulation contains 28% of the active ingredients. The medium capsule diameter is 8-15 microns. The resulting formulation is applied to seeds as an aqueous suspension in an apparatus suitable for that purpose.

Formulation types include an emulsion concentrate (EC), a suspension concentrate (SC), a suspo-emulsion (SE), a capsule suspension (CS), a water dispersible granule (WG), an emulsifiable granule (EG), an emulsion, water in oil (EO), an emulsion, oil in water (EW), a micro-emulsion (ME), an oil dispersion (OD), an oil miscible flowable (OF), an oil miscible liquid (OL), a soluble concentrate (SL), an ultra-low volume suspension (SU), an ultra-low volume liquid (UL), a technical concentrate (TK), a dispersible concentrate (DC), a wettable powder (WP), a soluble granule (SG) or any technically feasible formulation in combination with agriculturally acceptable adjuvants.

In a further aspect, the present invention makes available a pesticidal composition comprising a compound of the first aspect, one or more formulation additives and a carrier.

The activity of the compositions according to the invention can be broadened considerably, and adapted to prevailing circumstances, by adding other insecticidally, acaricidally and/or fungicidally active ingredients. The mixtures of the compounds of formula (I) with other insecticidally, acaricidally and/or fungicidally active ingredients may also have further surprising advantages which can also be described, in a wider sense, as synergistic activity. For example, better tolerance by plants, reduced phytotoxicity, insects can be controlled in their different development stages or better behaviour during their production, for example during grinding or mixing, during their storage or during their use.

Suitable additions to active ingredients here are, for example, representatives of the following classes of active ingredients: organophosphorus compounds, nitrophenol derivatives, thioureas, juvenile hormones, formamidines, benzophenone derivatives, ureas, pyrrole derivatives, carbamates, pyrethroids, chlorinated hydrocarbons, acylureas, pyridylmethyleneamino derivatives, macrolides, neonicotinoids and *Bacillus thuringiensis* preparations.

The following mixtures of the compounds of formula (I) with active ingredients are preferred (the abbreviation "TX" means "one compound selected from the group consisting of a compound A1 to A41 listed in Table A (below) or a specific compound listed in Tables 1 to 13 (below):
(7E,9Z)-dodeca-7,9-dien-1-yl acetate + TX, (9Z,11E)-tetradeca-9,11-dien-1-yl acetate + TX, (9Z,12E)-tetradeca-9,12-dien-1-yl acetate + TX, (E)-6-methylhept-2-en-4-ol + TX, (E)-dec-5-en-1-yl acetate with (E)-dec-5-en-1-ol + TX, (E)-tridec-4-en-1-yl acetate + TX, (E,Z)-tetradeca-4,10-dien-1-yl acetate + TX, (Z)-dodec-7-en-1-yl acetate + TX, (Z)-hexadec-11-en-1-yl acetate + TX, (Z)-hexadec-11-enal + TX, (Z)-hexadec-13-en-11-yn-1-yl acetate + TX, (Z)-icos-13-en-10-one + TX, (Z)-tetradec-7-en-1-al + TX, (Z)-tetradec-9-en-1-ol + TX, (Z)-tetradec-9-en-1-yl acetate + TX, 1,2-dibromo-3-chloropropane + TX, 1,2-dichloropropane + TX, 1,2-dichloropropane with 1,3-dichloropropene + TX, 1,3-dichloropropene + TX, 14-methyloctadec-1-ene + TX, 1-hydroxy-1H-pyridine-2-thione + TX, 2-(octylthio)ethanol + TX, 2-chlorophenyl N-methylcarbamate (CPMC) + TX, 3-(4-chlorophenyl)-5-methylrhodanine + TX, 3,4-dichlorotetrahydrothiophene 1,1-dioxide + TX, 4-(quinoxalin-2-ylamino)benzenesulfonamide + TX, 4-methylnonan-5-ol with 4-methylnonan-5-one + TX, 5-methyl-6-thioxo-1,3,5-thiadiazinan-3-ylacetic acid + TX, 6-isopentenylaminopurine + TX, 8-hydroxyquinoline sulfate + TX, abamectin + TX, acequinocyl + TX, acetamiprid + TX, acetoprole + TX, acrinathrin + TX, acynonapyr + TX, Adoxophyes orana GV + TX, afidopyropen + TX, afoxolaner + TX, Agrobacterium radiobacter + TX, AKD-3088 + TX, alanycarb + TX, aldicarb + TX, aldoxycarb + TX, allethrin + TX, alpha-cypermethrin + TX, alphamethrin + TX, alpha-multistriatin + TX, Amblyseius spp. + TX, amidoflumet + TX, amino acids + TX, aminocarb + TX, Anagrapha falcifera NPV + TX, Anagrus atomus + TX, Aphelinus abdominalis + TX, Aphidius colemani + TX, Aphidoletes aphidimyza + TX, apholate + TX, Autographa californica NPV + TX, AZ 60541 + TX, azadirachtin + TX, azocyclotin + TX, Bacillus aizawai + TX, Bacillus chitinosporus AQ746 (NRRL Accession No B-21 618) + TX, Bacillus firmus + TX, Bacillus kurstaki + TX, Bacillus mycoides AQ726 (NRRL Accession No. B-21664) + TX, Bacillus pumilus (NRRL Accession No B-30087) + TX, Bacillus pumilus AQ717 (NRRL Accession No. B-21662) + TX, Bacillus sp. AQ175 (ATCC Accession No. 55608) + TX, Bacillus sp. AQ177 (ATCC Accession No. 55609) + TX, Bacillus sp. AQ178 (ATCC Accession No. 53522) + TX, Bacillus sphaericus Neide + TX, Bacillus subtilis AQ153 (ATCC Accession No. 55614) + TX, Bacillus subtilis AQ30002 (NRRL Accession No. B-50421) + TX, Bacillus subtilis AQ30004 (NRRL Accession No. B- 50455) + TX, Bacillus subtilis AQ713 (NRRL Accession No. B-21661) + TX, Bacillus subtilis AQ743 (NRRL Accession No. B-21665) + TX, Bacillus subtilis unspecified + TX, Bacillus thuringiensis AQ52 (NRRL Accession No. B-21619) + TX, Bacillus thuringiensis BD#32 (NRRL Accession No B-21530) + TX, Bacillus thuringiensis Berliner + TX, Bacillus thuringiensis subsp. Aizawai + TX, Bacillus thuringiensis subsp. Israelensis + TX, Bacillus thuringiensis subsp. Japonensis + TX, Bacillus thuringiensis subsp. Kurstaki + TX, Bacillus thuringiensis subsp. Tenebrionis + TX, Bacillus thuringiensis subspec. kurstaki BMP 123 + TX, Beauveria bassiana + TX, Beauveria brongniartii + TX, benclothiaz + TX, benomyl + TX, bensultap + TX, bentioflumin (CAS Number: 2566451-67-8) + TX, benzoximate + TX, benzpyrimoxan + TX, betacyfluthrin + TX, beta-cypermethrin + TX, bethoxazin + TX, bifenazate + TX, bifenthrin + TX, binapacryl + TX, bioallethrin + TX, bioresmethrin + TX, bis(tributyltin) oxide + TX, bisazir + TX, bistrifluron + TX, bisulflufen + TX, brevicomin + TX, broflanilide + TX, brofluthrinate + TX, bromoacetamide + TX, bromophos-ethyl + TX, bronopol + TX, busulfan + TX, butocarboxim + TX, butopyronoxyl + TX, butoxy(polypropylene glycol) + TX, butylpyridaben + TX, cadusafos + TX, calcium arsenate + TX, carbaryl + TX, carbofuran + TX, carbon disulfide + TX, carbosulfan + TX, cartap + TX, CAS number: 1594624-87-9 + TX, CAS number: 1922957-47-8 + TX, CAS number: 1255091-74-7 + TX, CAS number: 1365070-72-9 + TX, CAS number: 1445683-71-5 + TX, CAS number: 1445684-82-1 + TX, CAS Number: 158062-71-6 + TX, CAS number: 1594626-19-3 + TX, CAS number: 1594637-65-6 + TX, CAS number: 1632218-00-8 + TX, CAS number: 1808115-49-2 + TX, CAS number: 1922957-46-7 + TX, CAS number: 1922957-48-9 + TX, CAS number: 1956329-03-5 + TX, CAS number: 1990457-52-7 + TX, CAS number: 1990457-55-0 + TX, CAS number: 1990457-57-2 + TX, CAS number: 1990457-66-3 + TX, CAS number: 1990457-77-6 + TX, CAS number: 1990457-85-6 + TX, CAS number: 2032403-97-5 + TX, CAS number: 2044701-44-0 + TX, CAS number: 2095470-94-1 + TX, CAS number: 2128706-05-6 + TX, CAS number: 2133042-31-4 + TX, CAS number: 2133042-44-9 + TX, CAS number: 2171099-09-3 + TX, CAS number: 2220132-55-6 + TX, CAS number: 2396747-83-2 + TX, CAS number: 2408220-91-5 + TX, CAS number: 2408220-94-8 + TX, CAS number: 2415706-16-8 + TX, CAS number: 2719848-60-7 + TX, CAS number: RNA (Leptinotarsa decemlineata-specific recombinant double-stranded interfering GS2) + TX, chlorantraniliprole + TX, chlordane + TX, chlorfenapyr + TX, chloropicrin + TX, chloroprallethrin + TX, chlorpyrifos + TX, chromafenozide + TX, Chrysoperla carnea + TX, clenpirin + TX, cloethocarb + TX, clothianidin + TX, codlelure + TX, codlemone + TX, copper acetoarsenite + TX, copper dioctanoate + TX, copper hydroxide + TX, copper sulfate + TX, cresol + TX, crufomate + TX, Cryptolaemus montrouzieri + TX, cuelure + TX, cyanofenphos + TX, cyantraniliprole + TX, cybenzoxasulfyl (CAS Number: 2128706-04-5) + TX, cybutryne + TX, cyclaniliprole + TX, cyclobutrifluram + TX, cycloprothrin + TX, cycloxaprid + TX, Cydia pomonella GV + TX, cyenopyrafen + TX, cyetpyrafen + TX, cyflumetofen + TX, cyfluthrin + TX, cyhalodiamide + TX, cylohalothrin + TX, cypermethrin + TX, cyphenothrin + TX, cyproflanilide + TX, cyromazine + TX, cytokinins + TX, Dacnusa sibirica + TX, dazomet + TX, DBCP + TX, DCIP + TX, deltamethrin + TX, diafenthiuron + TX, dialifos + TX, diamidafos + TX, dibrom + TX, dibutyl adipate + TX, dibutyl phthalate + TX, dibutyl succinate + TX, dichlofenthion + TX, dichlone + TX, dichlorophen + TX, dicliphos + TX, dicloromezotiaz + TX, diethyltoluamide + TX, diflubenzuron + TX, Diglyphus isaea + TX, dimatif + TX, dimethoate + TX, dimethyl carbate + TX, dimethyl phthalate + TX, dimpropyridaz + TX, dinactin + TX, dinocap + TX, dinotefuran + TX, dioxabenzofos + TX, dipyrithione + TX, disparlure + TX, D-limonene + TX, dodec-8-en-1-yl acetate + TX, dodec-9-en-1-yl acetate + TX, dodeca-8,10-dien-1-yl acetate + TX, dodicin + TX, dominicalure + TX, doramectin + TX, emamectin + TX, emamectin benzoate + TX, empenthrin + TX, Encarsia formosa + TX, endothal + TX, endrin + TX, eprinomectin + TX, epsilon - momfluorothrin + TX, epsilon-metofluthrin + TX, Eretmocerus eremicus + TX, esfenvalerate + TX, ethion + TX, ethiprole + TX, ethoprophos + TX, ethyl 4-methyloctanoate + TX, ethyl hexanediol + TX, ethylene dibromide + TX, etofenprox + TX, etoxazole + TX, etpyrafen + TX, eugenol + TX, Extract of seaweed and fermentation product derived from melasse + TX, Extract of seaweed and fermentation product derived from melasse comprising urea + TX, Extract of seaweed and fermented plant products + TX, Extract of seaweed and fermented plant products comprising phytohormones, vitamins, EDTA-chelated copper, zinc, and iron + TX, famphur + TX, fenaminosulf + TX, fenamiphos + TX, fenazaquin + TX, fenfluthrin + TX, fenitrothion + TX, fenmezoditiaz + TX, fenobucarb + TX, fenothiocarb + TX, fenoxycarb + TX, fenpropathrin + TX, fenpyrad + TX, fenpyroximate + TX, fensulfothion + TX, fenthion + TX, fentin + TX, fentinacetate + TX, fenvalerate + TX, ferric phosphate + TX, fipronil + TX, flometoquin + TX, flonicamid + TX, fluacrypyrim + TX, fluazaindolizine + TX, fluazuron + TX, flubendiamide + TX, flubenzimine + TX, fluchlordiniliprole + TX, flucitrinate + TX, flucycloxuron + TX, flucythrinate + TX, fluensulfone [318290-98-1] + TX, fluensulfone + TX, flufenerim + TX, flufenprox + TX, flufiprole + TX, fluhexafon + TX, flumethrin + TX, fluopyram + TX, flupyradifurone + TX, flupyrimin + TX, flupyroxystrobin + TX, fluralaner + TX, fluvalinate + TX, fluxametamide + TX, formaldehyde + TX, fosthiazate + TX, fosthietan + TX, frontalin + TX, furfural + TX, galquin (CAS Number: 2644770-30-7) + TX, gamma-cyhalothrin + TX, Gossyplure^{®} (1:1 mixture of the (Z,E) and (Z,Z) isomers of hexadeca-7,11-dien-1-yl-acetate) + TX, grandlure + TX, grandlure I + TX, grandlure II + TX, grandlure III + TX, grandlure IV + TX, Granulovirus + TX, guadipyr + TX, GY-81 + TX, halfenprox + TX, halofenozide + TX, Harpin + TX, Helicoverpa armigera Nucleopolyhedrovirus + TX, Helicoverpa zea NPV + TX, Helicoverpa zea Nucleopolyhedrovirus + TX, Heliothis punctigera Nucleopolyhedrovirus + TX, Heliothis virescens Nucleopolyhedrovirus + TX, hemel + TX, hempa + TX, heptafluthrin + TX, heterophos + TX, Heterorhabditis bacteriophora and H. megidis + TX, hexalure + TX, hexamide + TX, hexythiazox + TX, Hippodamia convergens + TX, hydramethylnon + TX, hydrargaphen + TX, hydrated lime + TX, imicyafos + TX, imidacloprid + TX, imiprothrin + TX, Indazapyroxamet + TX, indoxacarb + TX, iodomethane + TX, iprodione + TX, ipsdienol + TX, ipsenol + TX, isamidofos + TX, isazofos + TX, isocycloseram + TX, Isoflualanam (CAS number: 2892524-05-7) + TX, isothioate + TX, ivermectin + TX, japonilure + TX, kappa-bifenthrin + TX, kappa-tefluthrin + TX, kasugamycin + TX, kasugamycin hydrochloride hydrate + TX, kinetin + TX, lambda-cyhalothrin + TX, ledprona + TX, lepimectin + TX, Leptomastix dactylopii + TX, lineatin + TX, litlure + TX, looplure + TX, lotilaner + TX, lufenuron + TX, Macrolophus caliginosus + TX, Mamestra brassicae NPV + TX, mecarphon + TX, medlure + TX, megatomoic acid + TX, metaflumizone + TX, metaldehyde + TX, metam + TX, metam-potassium + TX, metam-sodium + TX, Metaphycus helvolus + TX, Metarhizium anisopliae var. acridum + TX, Metarhizium anisopliae var. anisopliae + TX, Metarhizium spp. + TX, metepa + TX, methiocarb + TX, methiotepa + TX, methomyl + TX, methoquin-butyl + TX, methoxyfenozide + TX, methyl apholate + TX, methyl bromide + TX, methyl eugenol + TX, methyl isothiocyanate + TX, methylneodecanamide + TX, metofluthrin + TX, metolcarb + TX, mexacarbate + TX, milbemectin + TX, milbemycin oxime + TX, momfluorothrin + TX, morzid + TX, moxidectin + TX, muscalure + TX, Muscodor albus 620 (NRRL Accession No. 30547) + TX, Muscodor roseus A3-5 (NRRL Accession No. 30548) + TX, Myrothecium verrucaria composition + TX, nabam + TX, NC-184 + TX, Neem tree based products + TX, Neodiprion sertifer NPV and N. lecontei NPV + TX, nickel bis(dimethyldithiocarbamate) + TX, niclosamide + TX, niclosamide-olamine + TX, nicofluprole + TX, nitenpyram + TX, nithiazine + TX, nitrapyrin + TX, octadeca-2,13-dien-1-yl acetate + TX, octadeca-3,13-dien-1-yl acetate + TX, octhilinone + TX, omethoate + TX, orfralure + TX, Orius spp. + TX, oryctalure + TX, ostramone + TX, oxamate + TX, oxamyl + TX, oxazosulfyl + TX, oxolinic acid + TX, oxytetracycline + TX, Paecilomyces fumosoroseus + TX, Paecilomyces lilacinus + TX, parathion-ethyl + TX, Pasteuria nishizawae + TX, Pasteuria penetrans + TX, Pasteuria ramosa + TX, Pasteuria thornei + TX, Pasteuria usgae + TX, P-cymene + TX, penfluron + TX, pentachlorophenol + TX, permethrin + TX, phenothrin + TX, phorate + TX, phosphamidon + TX, phosphocarb + TX, Phytoseiulus persimilis + TX, picaridin + TX, pioxaniliprole + TX, piperazine + TX, piperflanilide (CAS number: 2615135-05-0) + TX, piperonylbutoxide + TX, pirimicarb + TX, pirimiphos-ethyl + TX, pirimiphos-methyl + TX, Plutella xylostella Granulosis virus + TX, Plutella xylostella Nucleopolyhedrovirus + TX, Polyhedrosis virus + TX, potassium and molybdenum and EDTA-chelated manganese + TX, potassium ethylxanthate + TX, potassium hydroxyquinoline sulfate + TX, prallethrin + TX, probenazole + TX, profenofos + TX, profluthrin + TX, propargite + TX, propetamphos + TX, propoxur + TX, prothiophos + TX, protrifenbute + TX, pyflubumide + TX, pymetrozine + TX, pyraclofos + TX, pyrafluprole + TX, pyrethrum + TX, pyridaben + TX, pyridalyl + TX, pyridin-4-amine + TX, pyrifluquinazon + TX, pyrimidifen + TX, pyriminostrobin + TX, pyriprole [394730-71-3] + TX, pyriprole + TX, pyriproxyfen + TX, QRD 420 (a terpenoid blend) + TX, QRD 452 (a terpenoid blend) + TX, QRD 460 (a terpenoid blend) + TX, Quillaja saponaria + TX, quinoclamine + TX, quinonamid + TX, resmethrin + TX, Rhodococcus globerulus AQ719 (NRRL Accession No B-21663) + TX, sarolaner + TX, S-bioallethrin + TX, sebufos + TX, selamectin + TX, siglure + TX, silafluofen + TX, simazine + TX, sodium pentachlorophenoxide + TX, sordidin + TX, spidoxamat + TX, spinetoram + TX, spinosad + TX, spirobudifen + TX, spirodiclofen + TX, spiromesifen + TX, spiropidion + TX, spirotetramat + TX, Spodoptera exigua multicapsid nuclear polyhedrosis virus + TX, Spodoptera frugiperda Nucleopolyhedrovirus + TX, Steinernema bibionis + TX, Steinernema carpocapsae + TX, Steinernema feltiae + TX, Steinernema glaseri + TX, Steinernema riobrave + TX, Steinernema riobravis + TX, Steinernema scapterisci + TX, Steinernema spp. + TX, Streptomyces galbus (NRRL Accession No. 30232) + TX, Streptomyces sp. (NRRL Accession No. B-30145) + TX, streptomycin + TX, streptomycin sesquisulfate + TX, strychnine + TX, sulcatol + TX, sulfiflumin (CAS number: 2377084-09-6) + TX, sulfoxaflor + TX, tazimcarb + TX, tebufenozide + TX, tebufenpyrad + TX, tebupirimiphos + TX, tecloftalam + TX, tefluthrin + TX, temephos + TX, tepa + TX, terbam + TX, terbufos + TX, terpenoid blend + TX, tetrachlorantraniliprole + TX, tetrachlorothiophene + TX, tetradec-11-en-1-yl acetate + TX, tetradiphon + TX, tetramethrin + TX, tetramethylfluthrin + TX, tetranactin + TX, tetraniliprole + TX, theta-cypermethrin + TX, thiacloprid + TX, thiafenox + TX, thiamethoxam + TX, thiocyclam + TX, thiodicarb + TX, thiofanox + TX, thiohempa + TX, thiomersal + TX, thiometon + TX, thionazin + TX, thiophanate + TX, thiosultap + TX, thiotepa + TX, tiapyrachlor (CAS Number: 1255091-74-7) + TX, tigolaner + TX, tiorantraniliprole + TX, tioxazafen + TX, tolfenpyrad + TX, toxaphene + TX, tralomethrin + TX, transfluthrin + TX, tretamine + TX, triazamate + TX, triazophos + TX, triazuron + TX, tributyltin oxide + TX, trichlorfon + TX, trichloronate + TX, trichlorphon + TX, Trichogramma spp. + TX, trifenmorph + TX, trifluenfuronate + TX, triflumezopyrim + TX, trimedlure + TX, trimedlure A + TX, trimedlure B1 + TX, trimedlure B2 + TX, trimedlure C + TX, trimethacarb + TX, triphenyltin acetate + TX, triphenyltin hydroxide + TX, trunc-call + TX, tyclopyrazoflor + TX, Typhlodromus occidentalis + TX, uredepa + TX, Verticillium lecanii + TX, Verticillium spp. + TX, xylenols + TX, YI-5302 + TX, zeatin + TX, zeta-Cypermethrin + TX;
N-[(1R)-1-benzyl-3-chloro-1-methyl-but-3-enyl]-8-fluoro-quinoline-3-carboxamide + TX, N-[(1S)-1-benzyl-3-chloro-1-methyl-but-3-enyl]-8-fluoro-quinoline-3-carboxamide + TX, N-ethyl-N'-[5-methoxy-2-methyl-4-[(2-trifuoromethyl)tetrahydrofuran-2-yl]phenyl]-N-methyl-formamidine (these compounds may be prepared from the methods described in WO2019/110427) + TX, (3',4',5'-trifluoro-biphenyl-2-yl)-amide + TX, (3-methylisoxazol-5-yl)-[4-[5-(trifluoromethyl)-1,2,4-oxadiazol-3-yl]phenyl]methanone (these compounds may be prepared from the methods described in WO 2017/220485) + TX, (4-phenoxyphenyl)methyl 2-amino-6-methyl-pyridine-3-carboxylate (this compound may be prepared from the methods described in WO 2014/006945) + TX, (5-methyl-2-pyridyl)-[4-[5-(trifluoromethyl)-1,2,4-oxadiazol-3-yl]phenyl]methanone + TX, (7E,9Z)-dodeca-7,9-dien-1-yl acetate + TX, (9Z,11E)-tetradeca-9,11-dien-1-yl acetate + TX, (9Z,12E)-tetradeca-9,12-dien-1-yl acetate + TX, (E)-6-methylhept-2-en-4-ol + TX, (E)-dec-5-en-1-yl acetate with (E)-dec-5-en-1-ol + TX, (E)-tridec-4-en-1-yl acetate + TX, (E,Z)-tetradeca-4,10-dien-1-yl acetate, + TX, (R)-3-(difluoromethyl)-1-methyl-N-[1,1,3-trimethylindan-4-yl]pyrazole-4-carboxamide + TX, (Z)-dodec-7-en-1-yl acetate + TX, (Z)-hexadec-11-en-1-yl acetate + TX, (Z)-hexadec-11 -enal + TX, (Z)-hexadec-13-en-11-yn-1-yl acetate + TX, (Z)-icos-13-en-10-one + TX, (Z)-tetradec-7-en-1-al + TX, (Z)-tetradec-9-en-1-ol + TX, (Z)-tetradec-9-en-1-yl acetate + TX, (Z,2E)-5-[1-(2,4-dichlorophenyl)pyrazol-3-yl]oxy-2-methoxyimino-N,3-dimethyl-pent-3-enamide (this compound may be prepared from the methods described in WO 2018/153707) + TX, (Z,2E)-5-[1-(4-chlorophenyl)pyrazol-3-yl]oxy-2-methoxyimino-N,3-dimethyl-pent-3-enamide + TX, , [2-[3-[2-[1-[2-[3,5-bis(difluoromethyl)pyrazol-1-yl]acetyl]-4-piperidyl]thiazol-4-yl]-4,5-dihydroisoxazol-5-yl]-3-chlorophenyl] methanesulfonate + TX, 1-(4,5-dimethylbenzimidazol-1-yl)-4,4,5-trifluoro-3,3-dimethyl-isoquinoline + TX, 1-(4,5-dimethylbenzimidazol-1-yl)-4,4-difluoro-3,3-dimethyl-isoquinoline + TX, 1-(6,7-dimethylpyrazolo[1,5-a]pyridin-3-yl)-4,4,5-trifluoro-3,3-dimethyl-isoquinoline + TX, 1-(6,7-dimethylpyrazolo[1,5-a]pyridin-3-yl)-4,4,6-trifluoro-3,3-dimethyl-isoquinoline + TX, 1-(6-chloro-7-methyl-pyrazolo[1,5-a]pyridin-3-yl)-4,4-difluoro-3,3-dimethyl-isoquinoline (these compounds may be prepared from the methods described in WO2017/025510) + TX, 1,1-bis(4-chlorophenyl)-2-ethoxyethanol + TX, 1,1-dichloro-2,2-bis(4-ethylphenyl)ethane + TX, 1,2-dibromo-3-chloropropane + TX, 1,2-dichloropropane with 1,3-dichloropropene + TX, 1,3-dichloropropene + TX, 1,3-dimethoxy-1-[[4-[5-(trifluoromethyl)-1,2,4-oxadiazol-3-yl]phenyl]methyl]urea + TX, 1-[2-[[1-(4-chlorophenyl)pyrazol-3-yl]oxymethyl]-3-methyl-phenyl]-4-methyl-tetrazol-5-one + TX, 10-dien-1-yl acetate + TX, 14-methyloctadec-1-ene + TX, 1-bromo-2-chloroethane + TX, 1-dichloro-1-nitroethane + TX, 1-hydroxy-1H-pyridine-2-thione + TX, 1-methoxy-3-methyl-1-[[4-[5-(trifluoromethyl)-1,2,4-oxadiazol-3-yl]phenyl]methyl]urea + TX, 1-methyl-4-[3-methyl-2-[[2-methyl-4-(3,4,5-trimethylpyrazol-1-yl)phenoxy]methyl]phenyl]tetrazol-5-one + TX, 2- (difluoromethyl) - N- ((3R) - 1, 1, 3- trimethylindan- 4-yl) pyridine- 3- carboxamide + TX, 2- (difluoromethyl) - N- ((3R) - 1, 1, 3- trimethylindan- 4-yl) pyridine-3- carboxamide + TX, 2-(1,3-dithiolan-2-yl)phenyl dimethylcarbamate + TX, 2-(2-butoxyethoxy)ethyl piperonylate + TX, 2-(2-butoxyethoxy)ethyl thiocyanate + TX, 2-(4,5-dimethyl-1,3-dioxolan-2-yl)phenyl methylcarbamate + TX, 2-(4-chloro-3,5-xylyloxy)ethanol + TX, 2-(difluoromethyl)-N-(3-ethyl-1,1-dimethyl-indan-4-yl)pyridine-3-carboxamide + TX, 2-(difluoromethyl)-N-[(3R)-3-ethyl-1,1-dimethyl-indan-4-yl]pyridine-3-carboxamide + TX, 2-(difluoromethyl)-N-[(3S)-3-ethyl-1,1-dimethyl-indan-4-yl]pyridine-3-carboxamide (this compound may be prepared from the methods described in WO 2014/095675) + TX, 2-(difluoromethyl)-N-[3-ethyl-1,1-dimethyl-indan-4-yl]pyridine-3-carboxamide + TX, 2-(octylthio)ethanol + TX, 2,2,2-trichloro-1-(3,4-dichlorophenyl)ethyl acetate + TX, 2,2-dichlorovinyl 2-ethylsulfinylethyl methyl phosphate + TX, 2,2-difluoro-N-methyl-2-[4-[5-(trifluoromethyl)-1,2,4-oxadiazol-3-yl]phenyl]acetamide + TX, 2,4-dichlorophenyl benzenesulfonate + TX, 2,6-Dimethyl-1H,5H-[1,4]dithiino[2,3-c:5,6-c']dipyrrole-1,3,5,7(2H,6H)-tetrone (this compound may be prepared from the methods described in WO 2011/138281) + TX, 2-[2-fluoro-6-[(8-fluoro-2-methyl-3-quinolyl)oxy]phenyl]propan-2-ol + TX, 2-[6-(4-bromophenoxy)-2-(trifluoromethyl)-3-pyridyl]-1-(1,2,4-triazol-1-yl)propan-2-ol (this compound may be prepared from the methods described in WO 2017/029179) + TX, 2-[6-(4-chlorophenoxy)-2-(trifluoromethyl)-3-pyridyl]-1-(1,2,4-triazol-1-yl)propan-2-ol (this compound may be prepared from the methods described in WO 2017/029179) + TX, 2-chlorovinyl diethyl phosphate + TX, 2-fluoro-N-methyl-N-1-naphthylacetamide + TX, 2-imidazolidone + TX, 2-isovalerylindan-1,3-dione + TX, 2-methyl(prop-2-ynyl)aminophenyl methylcarbamate + TX, 2-oxoN-propyl-2-[4-[5-(trifluoromethyl)-1,2,4-oxadiazol-3-yl]phenyl]acetamide (this compound may be prepared from the methods described in WO 2018/065414) + TX, 2-thiocyanatoethyl laurate + TX, 3-(4,4-difluoro-3,3-dimethyl-1-isoquinolyl)-7,8-dihydro-6H-cyclopenta[e]benzimidazole (these compounds may be prepared from the methods described in WO2016/156085) + TX, 3-(4,4-difluoro-3,4-dihydro-3,3-dimethylisoquinolin-1-yl)quinolone + TX, 3-(4-chlorophenyl)-5-methylrhodanine + TX, 3-(difluoromethyl)-1-methyl-N-[1,1,3-trimethylindan-4-yl]pyrazole-4-carboxamide + TX, 3,4-dichlorotetrahydrothiophene 1,1-dioxide + TX, 3-[2-(1-chlorocyclopropyl)-3-(2-fluorophenyl)-2-hydroxypropyl]imidazole-4-carbonitrile (this compound may be prepared from the methods described in WO 2016/156290) + TX, 3-[2-(1-chlorocyclopropyl)-3-(3-chloro-2-fluoro-phenyl)-2-hydroxypropyl]imidazole-4-carbonitrile (this compound may be prepared from the methods described in WO 2016/156290) + TX, 3-bromo-1-chloroprop-1-ene + TX, 3-chloro-6-methyl-5-phenyl-4-(2,4,6-trifluorophenyl)pyridazine + TX, 3-difluoromethyl-1-methyl-1H-pyrazole-4-carboxylic acid + TX, 3-ethyl-1-methoxy-1-[[4-[5-(trifluoromethyl)-1,2,4-oxadiazol-3-yl]phenyl]methyl]urea + TX, 3-methyl-1-phenylpyrazol-5-yl dimethylcarbamate + TX, 4- (2- bromo- 4- fluorophenyl) - N- (2- chloro- 6-fluorophenyl) - 1, 3- dimethyl- 1H- pyrazol- 5- amine + TX, 4-(2,6-difluorophenyl)-6-methyl-5-phenyl-pyridazine-3-carbonitrile + TX, 4-(2-bromo-4-fluoro-phenyl)-N-(2-chloro-6-fluoro-phenyl)-2,5-dimethylpyrazol-3-amine + TX, 4-(quinoxalin-2-ylamino)benzenesulfonamide + TX, 4,4-difluoro-1-(5-fluoro-4-methyl-benzimidazol-1-yl)-3,3-dimethyl-isoquinoline + TX, 4,4-difluoro-3,3-dimethyl-1-(6-methylpyrazolo[1,5-a]pyridin-3-yl)isoquinoline + TX, 4,4-difluoro-3,3-dimethyl-1-(7-methylpyrazolo[1,5-a]pyridin-3-yl)isoquinoline + TX, 4,4-dimethyl-2-[[4-[5-(trifluoromethyl)-1,2,4-oxadiazol-3-yl]phenyl]methyl]isoxazolidin-3-one + TX, 4-[[6-[2-(2,4-difluorophenyl)-1,1-difluoro-2-hydroxy-3-(1,2,4-triazol-1-yl)propyl]-3-pyridyl]oxy] benzonitrile + TX, 4-[[6-[2-(2,4-difluorophenyl)-1,1-difluoro-2-hydroxy-3-(5-sulfanyl-1,2,4-triazol-1-yl)propyl]-3-pyridyl]oxy] benzonitrile + TX, 4-[[6-[2-(2,4-difluorophenyl)-1,1-difluoro-2-hydroxy-3-(5-thioxo-4H-1,2,4-triazol-1-yl)propyl]-3-pyridyl]oxy] benzonitrile + TX, 4-chloro-2-(2-chloro-2-methyl-propyl)-5-[(6-iodo-3-pyridyl)methoxy]pyridazin-3-one + TX, 4-chlorophenyl phenyl sulfone + TX, 4-methyl(prop-2-ynyl)amino-3,5-xylyl methylcarbamate + TX, 4-methylnonan-5-ol with 4-methylnonan-5-one + TX, 5-(1,3-benzodioxol-5-yl)-3-hexylcyclohex-2-enone + TX, 5,5-dimethyl-2-[[4-[5-(trifluoromethyl)-1,2,4-oxadiazol-3-yl]phenyl]methyl]isoxazolidin-3-one + TX, 5,5-dimethyl-3-oxocyclohex-1-enyl dimethylcarbamate + TX, 5-amino-1,3,4-thiadiazole-2-thiol zinc salt (2:1) + TX, 5-methyl-6-thioxo-1,3,5-thiadiazinan-3-ylacetic acid + TX, 6-chloro-3-(3-cyclopropyl-2-fluoro-phenoxy)-N-[2-(2,4-dimethylphenyl)-2,2-difluoro-ethyl]-5-methyl-pyridazine-4-carboxamide (may be prepared from the methods described in WO 2020/109391) + TX, 6-chloro-3-(3-cyclopropyl-2-fluoro-phenoxy)-N-[2-(3,4-dimethylphenyl)-2,2-difluoro-ethyl]-5-methyl-pyridazine-4-carboxamide (may be prepared from the methods described in WO 2020/109391) + TX, 6-chloro-4,4-difluoro-3,3-dimethyl-1-(4-methylbenzimidazol-1-yl)isoquinoline + TX, 6-chloro-N-[2-(2-chloro-4-methyl-phenyl)-2,2-difluoroethyl]-3-(3-cyclopropyl-2-fluoro-phenoxy)-5-methyl-pyridazine-4-carboxamide (may be prepared from the methods described in WO 2020/109391) + TX, 6-ethyl-5,7-dioxo-pyrrolo[4,5][1,4]dithiino[1,2-c]isothiazole-3-carbonitrile + TX, 6-isopentenylaminopurine + TX, 8-fluoro-N-[(1R)-1-[(3-fluorophenyl)methyl]-1,3-dimethyl-butyl]quinoline-3-carboxamide + TX, 8-fluoro-N-[(1S)-1-[(3-fluorophenyl)methyl]-1,3-dimethyl-butyl]quinoline-3-carboxamide + TX, 8-hydroxyquinoline sulfate + TX, acethion + TX, acetoprole + TX, acibenzolar + TX, acibenzolar-S-methyl + TX, acrylonitrile + TX, Adoxophyes orana GV + TX, Agrobacterium radiobacter + TX, aldoxycarb + TX, aldrin + TX, allosamidin + TX, allyxycarb + TX, alpha-chlorohydrin + TX, alpha-ecdysone + TX, alpha-multistriatin + TX, aluminium phosphide + TX, Amblyseius spp. + TX, amectotractin + TX, ametoctradin + TX, amidithion + TX, amidothioate + TX, aminocarb + TX, aminopyrifen + TX, amisulbrom + TX, amiton + TX, amiton hydrogen oxalate + TX, amitraz + TX, anabasine + TX, Anagrapha falcifera NPV + TX, Anagrus atomus + TX, ancymidol + TX, anilazine + TX, anisiflupurin + TX, anthraquinone + TX, antu + TX, Aphelinus abdominalis + TX, Aphidius colemani + TX, Aphidoletes aphidimyza + TX, apholate + TX, aramite + TX, arsenous oxide + TX, athidathion + TX, Autographa californica NPV + TX, azaconazole + TX, azamethiphos + TX, azobenzene + TX, azothoate + TX, azoxystrobin + TX, Bacillus sphaericus Neide + TX, Bacillus thuringiensis delta endotoxins + TX, barium carbonate + TX, barium hexafluorosilicate + TX, barium polysulfide + TX, barthrin + TX, Bayer 22/190 + TX, Bayer 22408 + TX, Beauveria brongniartii + TX, benalaxyl + TX, benclothiaz + TX, benomyl + TX, benoxafos + TX, benthiavalicarb + TX, benzothiostrobin + TX, benzovindiflupyr + TX, benzyl benzoate + TX, beta-cyfluthrin + TX, beta-cypermethrin + TX, bethoxazin + TX, bioethanomethrin + TX, biopermethrin + TX, bis(2-chloroethyl) ether + TX, bis(tributyltin) oxide + TX, bisazir + TX, bisthiosemi + TX, bitertanol + TX, bixafen + TX, blasticidin-S + TX, borax + TX, bordeaux mixture + TX, boscalid + TX, brevicomin + TX, brodifacoum + TX, brofenvalerate + TX, bromadiolone + TX, bromethalin + TX, bromfenvinfos + TX, bromoacetamide + TX, bromocyclen + TX, bromo-DDT + TX, bromophos + TX, bromopropylate + TX, bromuconazole + TX, bronopol + TX, bufencarb + TX, bupirimate + TX, buprofezin + TX, busulfan + TX, but-3-ynyl N-[6-[[(Z)-[(1-methyltetrazol-5-yl)-phenyl-methylene]amino]oxymethyl]-2-pyridyl]carbamate + TX, butacarb + TX, butathiofos + TX, butocarboxim + TX, butonate + TX, butopyronoxyl + TX, butoxy(polypropylene glycol) + TX, butoxycarboxim + TX, butylpyridaben + TX, calcium arsenate + TX, calcium cyanide + TX, calcium polysulfide + TX, camphechlor + TX, captafol + TX, captan + TX, carbanolate + TX, carbendazim + TX, carbon disulfide + TX, carbon tetrachloride + TX, carbophenothion + TX, carboxin + TX, cartap hydrochloride + TX, CAS Number: 2454319-63-0 + TX, cevadine + TX, chinomethionat + TX, chloralose + TX, chlorbenside + TX, chlorbicyclen + TX, chlordane + TX, chlordecone + TX, chlordimeform + TX, chlordimeform hydrochloride + TX, chlorfenethol + TX, chlorfenson + TX, chlorfensulfide + TX, chlorobenzilate + TX, chloroform + TX, chloroinconazide + TX, chloromebuform + TX, chloromethiuron + TX, chloroneb + TX, chlorophacinone + TX, chloropicrin + TX, chloropropylate + TX, chlorothalonil + TX, chlorphoxim + TX, chlorprazophos + TX, chlorthiophos + TX, chlozolinate + TX, cholecalciferol + TX, Chrysoperla carnea + TX, cinerin I + TX, cinerin II + TX, cinerins + TX, cismethrin + TX, cis-resmethrin + TX, clocythrin + TX, closantel + TX, codlelure + TX, codlemone + TX, copper acetoarsenite + TX, copper arsenate + TX, copper dioctanoate + TX, copper hydroxide + TX, copper naphthenate + TX, copper oleate + TX, copper oxide + TX, copper oxychloride + TX, copper sulfate + TX, coumachlor + TX, coumafuryl + TX, coumaphos + TX, coumatetralyl + TX, coumethoxystrobin (jiaxiangjunzhi) + TX, coumithoate + TX, coumoxystrobin + TX, cresol + TX, crimidine + TX, crotamiton + TX, crotoxyphos + TX, crufomate + TX, cryolite + TX, Cryptolaemus montrouzieri + TX, CS 708 + TX, cuelure + TX, cufraneb + TX, cyanofenphos + TX, cyanophos + TX, cyanthoate + TX, cyazofamid + TX, cybutryne + TX, cyclethrin + TX, cyclobutrifluram + TX, Cydia pomonella GV + TX, cyflufenamid + TX, cymiazole + TX, cymoxanil + TX, cyproconazole + TX, cyprodinil + TX, cythioate + TX, cytokinins + TX, Dacnusa sibirica + TX, DAEP + TX, dazomet + TX, DCIP + TX, DCPM + TX, DDT + TX, debacarb + TX, decarbofuran + TX, demephion + TX, demephion-O + TX, demephion-S + TX, demeton-methyl + TX, demeton-O + TX, demeton-O-methyl + TX, demeton-S + TX, demeton-S-methyl + TX, demeton-S-methylsulfon + TX, diamidafos + TX, dibutyl adipate + TX, dibutyl phthalate + TX, dibutyl succinate + TX, dicapthon + TX, dichlobentiazox + TX, dichlofenthion + TX, dichlofluanid + TX, dichlone + TX, dichlorophen + TX, dichlorvos + TX, dichlozoline + TX, dicliphos + TX, diclocymet + TX, diclomezine + TX, dicloran + TX, dicresyl + TX, dicyclanil + TX, dicyclopentadiene + TX, dieldrin + TX, dienochlor + TX, diethofencarb + TX, diethyl 5-methylpyrazol-3-yl phosphate + TX, diethyltoluamide + TX, difenacoum + TX, difenoconazole + TX, difethialone + TX, diflovidazin + TX, Diglyphus isaea + TX, dilor + TX, dimatif + TX, dimefluthrin + TX, dimefox + TX, dimetan + TX, dimethirimol + TX, dimethomorph + TX, dimethrin + TX, dimethyl carbate + TX, dimethyl phthalate + TX, dimethylvinphos + TX, dimetilan + TX, dimoxystrobin + TX, dinex + TX, dinex-diclexine + TX, diniconazole + TX, dinocap-4 + TX, dinocap-6 + TX, dinocton + TX, dinopenton + TX, dinoprop + TX, dinosam + TX, dinoseb + TX, dinosulfon + TX, dinoterbon + TX, diofenolan + TX, dioxabenzofos + TX, dioxathion + TX, diphacinone + TX, diphenyl sulfone + TX, dipymetitrone + TX, dipyrithione + TX, disparlure + TX, disulfiram + TX, dithianon + TX, dithicrofos + TX, DNOC + TX, dodec-8-en-1-yl acetate + TX, dodec-9-en-1-yl acetate + TX, dodeca-8 + TX, dodemorph + TX, dodicin + TX, dodine + TX, dofenapyn + TX, dominicalure + TX, doramectin + TX, DSP + TX, d-tetramethrin + TX, ecdysterone + TX, edifenphos + TX, EI 1642 + TX, EMPC + TX, Encarsia formosa + TX, endothal + TX, endothion + TX, enestroburin + TX, enoxastrobin + TX, EPBP + TX, epoxiconazole + TX, eprinomectin + TX, Eretmocerus eremicus + TX, ergocalciferol + TX, etaphos + TX, ethaboxam + TX, ethiofencarb + TX, ethirimol + TX, ethoate-methyl + TX, ethyl 1-[[4-[(Z)-2-ethoxy-3,3,3-trifluoro-prop-1-enoxy]phenyl]methyl]pyrazole-3-carboxylate (may be prepared from the methods described in WO 2020/056090) + TX, ethyl 1-[[4-[[2-(trifluoromethyl)-1,3-dioxolan-2-yl]methoxy]phenyl]methyl]pyrazole-3-carboxylate (may be prepared from the methods described in WO 2020/056090) + TX, ethyl 1-[[4-[5-(trifluoromethyl)-1,2,4-oxadiazol-3-yl]phenyl]methyl]pyrazole-4-carboxylate + TX, ethyl 1-[[5-[5-(trifluoromethyl)-1,2,4-oxadiazol-3-yl]-2-thienyl]methyl]pyrazole-4-carboxylate (this compound may be prepared from the methods described in WO 2018/158365) + TX, ethyl 4-methyloctanoate + TX, ethyl formate + TX, ethyl hexanediol + TX, ethylene dibromide + TX, ethylene dichloride + TX, ethylene oxide + TX, etridiazole + TX, etrimfos + TX, eugenol + TX, EXD + TX, famoxadone + TX, farnesol + TX, farnesol with nerolidol + TX, fenamidone + TX, fenaminosulf + TX, fenaminstrobin + TX, fenarimol + TX, fenazaflor + TX, fenbuconazole + TX, fenbutatin oxide + TX, fenchlorphos + TX, feneptamidoquin (CAS Number: 2132414-04-9) + TX, fenethacarb + TX, fenfuram + TX, fenhexamid + TX, fenitrothion + TX, fenopyramid (CAS Number: 2344721-61-3) + TX, fenothiocarb + TX, fenoxacrim + TX, fenoxanil + TX, fenpiclonil + TX, fenpicoxamid + TX, fenpirithrin + TX, fenpropidin + TX, fenpropimorph + TX, fenpyrad + TX, fenpyrazamine + TX, fenpyroximate + TX, fenson + TX, fensulfothion + TX, fenthion + TX, fenthion-ethyl + TX, fentin + TX, fentrifanil + TX, ferbam + TX, ferimzone + TX, ferric phosphate + TX, flocoumafen + TX, florylpicoxamid + TX, fluazinam + TX, flubeneteram + TX, flubenzimine + TX, flucofuron + TX, flucycloxuron + TX, fludioxonil + TX, fluenetil + TX, flufenoxadiazam + TX, flufenoxystrobin + TX, fluindapyr + TX, flumetylsulforim + TX, flumorph + TX, fluopicolide + TX, fluopimomide + TX, fluopyram + TX, fluorbenside + TX, fluoroacetamide + TX, fluoroimide + TX, fluoxapiprolin + TX, fluoxastrobin + TX, fluoxytioconazole + TX, flupropadine + TX, flupropadine hydrochloride + TX, fluquinconazole + TX, flusilazole + TX, flusulfamide + TX, flutianil + TX, flutolanil + TX, flutriafol + TX, fluxapyroxad + TX, FMC 1137 + TX, folpet + TX, formaldehyde + TX, formetanate + TX, formetanate hydrochloride + TX, formparanate + TX, fosetyl-aluminium + TX, fosmethilan + TX, fospirate + TX, fosthietan + TX, frontalin + TX, fuberidazole + TX, furalaxyl + TX, furametpyr + TX, furathiocarb + TX, furethrin + TX, furfural + TX, gamma-HCH + TX, glyodin + TX, grandlure + TX, grandlure I + TX, grandlure II + TX, grandlure III + TX, grandlure IV + TX, guazatine + TX, guazatine acetates + TX, halfenprox + TX, HCH + TX, hemel + TX, hempa + TX, HEOD + TX, heptachlor + TX, heterophos + TX, Heterorhabditis bacteriophora and H. megidis + TX, hexaconazole + TX, hexadecyl cyclopropanecarboxylate + TX, hexalure + TX, hexamide + TX, HHDN + TX, Hippodamia convergens + TX, hydrargaphen + TX, hydrated lime + TX, hydrogen cyanide + TX, hymexazol + TX, hyquincarb + TX, imanin + TX, imazalil + TX, imibenconazole + TX, iminoctadine + TX, inpyrfluxam + TX, ipconazole + TX, ipfentrifluconazole + TX, ipflufenoquin + TX, iprobenphos + TX, iprodione + TX, iprovalicarb + TX, ipsdienol + TX, ipsenol + TX, IPSP + TX, isamidofos + TX, isazofos + TX, isobenzan + TX, isocarbophos + TX, isodrin + TX, isofenphos + TX, isofetamid + TX, isoflucypram + TX, isolane + TX, isoprothiolane + TX, isopyrazam + TX, isotianil + TX, isoxathion + TX, japonilure + TX, jasmolin I + TX, jasmolin II + TX, jodfenphos + TX, juvenile hormone I + TX, juvenile hormone II + TX, juvenile hormone III + TX, kadethrin + TX, kasugamycin + TX, kasugamycin hydrochloride hydrate + TX, kelevan + TX, kinetin + TX, kinoprene + TX, kresoxim-methyl + TX, lead arsenate + TX, Leptomastix dactylopii + TX, leptophos + TX, lindane + TX, lineatin + TX, lirimfos + TX, litlure + TX, looplure + TX, lvbenmixianan + TX, lythidathion + TX, Macrolophus caliginosus + TX, magnesium phosphide + TX, malonoben + TX, Mamestra brassicae NPV + TX, mancopper + TX, mancozeb + TX, mandestrobin + TX, mandipropamid + TX, maneb + TX, mazidox + TX, m-cumenyl methylcarbamate + TX, mecarbam + TX, mecarphon + TX, medlure + TX, mefentrifluconazole + TX, megatomoic acid + TX, menazon + TX, mepanipyrim + TX, meperfluthrin + TX, mephosfolan + TX, mepronil + TX, mercuric oxide + TX, mercurous chloride + TX, mesulfen + TX, mesulfenfos + TX, metalaxyl + TX, metam + TX, metam-potassium + TX, metam-sodium + TX, Metaphycus helvolus + TX, Metarhizium anisopliae var. acridum + TX, Metarhizium anisopliae var. anisopliae + TX, metarylpicoxamid + TX, metconazole + TX, metepa + TX, methacrifos + TX, methanesulfonyl fluoride + TX, methasulfocarb + TX, methiotepa + TX, methocrotophos + TX, methoprene + TX, methoquin-butyl + TX, methothrin + TX, methoxychlor + TX, methyl (Z)-2-(5-cyclohexyl-2-methyl-phenoxy)-3-methoxy-prop-2-enoate + TX, methyl (Z)-2-(5-cyclopentyl-2-methyl-phenoxy)-3-methoxy-prop-2-enoate (these compounds may be prepared from the methods described in WO2020/193387) + TX, methyl (Z)-2-[5-(3-isopropylpyrazol-1-yl)-2-methyl-phenoxy]-3-methoxy-prop-2-enoate + TX, methyl (Z)-3-methoxy-2-[2-methyl-5-(3-propylpyrazol-1-yl)phenoxy]prop-2-enoate + TX, methyl (Z)-3-methoxy-2-[2-methyl-5-(4-propyltriazol-2-yl)phenoxy]prop-2-enoate + TX, methyl (Z)-3-methoxy-2-[2-methyl-5-[3-(trifluoromethyl)pyrazol-1-yl]phenoxy]prop-2-enoate (these compounds may be prepared from the methods described in WO2020/079111) + TX, methyl (Z)-3-methoxy-2-[2-methyl-5-[4-(trifluoromethyl)triazol-2-yl]phenoxy]prop-2-enoate + TX, methyl apholate + TX, methyl bromide + TX, methyl eugenol + TX, methyl isothiocyanate + TX, methyl N-[[4-[1-(2,6-difluoro-4-isopropyl-phenyl)pyrazol-4-yl]-2-methylphenyl]methyl]carbamate (may be prepared from the methods described in WO 2020/097012) + TX, methyl N-[[4-[1-(4-cyclopropyl-2,6-difluoro-phenyl)pyrazol-4-yl]-2-methyl-phenyl]methyl]carbamate (may be prepared from the methods described in WO 2020/097012) + TX, methyl N-[[5-[4-(2,4-dimethylphenyl)triazol-2-yl]-2-methyl-phenyl]methyl]carbamate + TX, methylchloroform + TX, methylene chloride + TX, methylneodecanamide + TX, metiram + TX, metolcarb + TX, metominostrobin + TX, metoxadiazone + TX, metrafenone + TX, metyltetraprole + TX, MGK 264 + TX, milbemycin oxime + TX, mipafox + TX, mirex + TX, monocrotophos + TX, morphothion + TX, morzid + TX, moxidectin + TX, muscalure + TX, myclobutanil + TX, myclozoline + TX, Myrothecium verrucaria composition + TX, N-((1R)-1-benzyl-3-chloro-1-methyl-but-3-enyl)-8-fluoro-quinoline-3-carboxamide (these compounds may be prepared from the methods described in WO2017/153380) + TX, N-((1S)-1-benzyl-3-chloro-1-methyl-but-3-enyl)-8-fluoro-quinoline-3-carboxamide (these compounds may be prepared from the methods described in WO2017/153380) + TX, N'-(2,5-dimethyl-4-phenoxy-phenyl)-N-ethyl-N-methyl-formamidine + TX, N'-(2-chloro-5-methyl-4-phenoxy-phenyl)-N-ethyl-N-methyl-formamidine + TX, N,2-dimethoxy-N-[[4-[5-(trifluoromethyl)-1,2,4-oxadiazol-3-yl]phenyl]methyl]propanamide + TX, N,N-dimethyl-1-[[4-[5-(trifluoromethyl)-1,2,4-oxadiazol-3-yl]phenyl]methyl]-1,2,4-triazol-3-amine (THESE COMPOUNDS may be prepared from the methods described in WO 2017/055473, WO 2017/055469, WO 2017/093348 and WO 2017/118689) + TX, N-[(1R)-1-benzyl-1,3-dimethyl-butyl]-7,8-difluoro-quinoline-3-carboxamide + TX, N-[(1R)-1-benzyl-1,3-dimethyl-butyl]-8-fluoro-quinoline-3-carboxamide + TX, N-[(1R)-1-benzyl-3,3,3-trifluoro-1-methyl-propyl]-8-fluoro-quinoline-3-carboxamide + TX, N-[(1S)-1-benzyl-1,3-dimethyl-butyl]-7,8-difluoro-quinoline-3-carboxamide + TX, N-[(1S)-1-benzyl-1,3-dimethylbutyl]-8-fluoro-quinoline-3-carboxamide + TX, N-[(1S)-1-benzyl-3,3,3-trifluoro-1-methyl-propyl]-8-fluoro-quinoline-3-carboxamide + TX, N-[(E)-methoxyiminomethyl]-4-[5-(trifluoromethyl)-1,2,4-oxadiazol-3-yl]benzamide + TX, N-[(Z)-methoxyiminomethyl]-4-[5-(trifluoromethyl)-1,2,4-oxadiazol-3-yl]benzamide + TX, N-[[4-[5-(trifluoromethyl)-1,2,4-oxadiazol-3-yl]phenyl]methyl]propanamide + TX, N-[2-[2,4-dichloro-phenoxy]phenyl]-3-(difluoromethyl)-1-methyl-pyrazole-4-carboxamide + TX, N-[2-[2-chloro-4-(trifluoromethyl)phenoxy]phenyl]-3-(difluoromethyl)-1-methyl-pyrazole-4-carboxamide + TX, N'-[2-chloro-4-(2-fluorophenoxy)-5-methyl-phenyl]-N-ethyl-N-methyl-formamidine (this compound may be prepared from the methods described in WO 2016/202742) + TX, N'-[4-(4,5-dichlorothiazol-2-yl)oxy-2,5-dimethyl-phenyl]-N-ethyl-N-methyl-formamidine + TX, N'-[5-bromo-2-methyl-6-(1-methyl-2-propoxy-ethoxy)-3-pyridyl]-N-ethyl-N-methyl-formamidine + TX, N'-[5-bromo-2-methyl-6-(1-methyl-2-propoxy-ethoxy)-3-pyridyl]-N-isopropyl-N-methyl-formamidine (these compounds may be prepared from the methods described in WO2015/155075) + TX, N'-[5-bromo-2-methyl-6-(2-propoxypropoxy)-3-pyridyl]-N-ethyl-N-methyl-formamidine (this compound may be prepared from the methods described in IPCOM000249876D) + TX, N'-[5-bromo-2-methyl-6-[(1R)-1-methyl-2-propoxy-ethoxy]-3-pyridyl]-N-ethyl-N-methyl-formamidine + TX, N'-[5-bromo-2-methyl-6-[(1S)-1-methyl-2-propoxy-ethoxy]-3-pyridyl]-N-ethyl-N-methyl-formamidine + TX, N'-[5-chloro-2-methyl-6-(1-methyl-2-propoxy-ethoxy)-3-pyridyl]-N-ethyl-N-methyl-formamidine + TX, N-[N-methoxy-C-methyl-carbonimidoyl]-4-[5-(trifluoromethyl)-1,2,4-oxadiazol-3-yl]benzamide (these compounds may be prepared from the methods described in WO 2018/202428) + TX, N'-[4-(1-cyclopropyl-2,2,2-trifluoro-1-hydroxy-ethyl)-5-methoxy-2-methyl-phenyl]-N-isopropyl-N-methyl-formamidine (these compounds may be prepared from the methods described in WO2018/228896) + TX, nabam + TX, naftalofos + TX, naled + TX, naphthalene + TX, NC-170 + TX, Neodiprion sertifer NPV and N. lecontei NPV + TX, nerolidol + TX, N-ethyl-2-methyl-N-[[4-[5-(trifluoromethyl)-1,2,4-oxadiazol-3-yl]phenyl]methyl]propanamide + TX, N-ethyl-N'-[5-methoxy-2-methyl-4-[(2-trifluoromethyl)oxetan-2-yl]phenyl]-N-methyl-formamidine + TX, nickel bis(dimethyldithiocarbamate) + TX, niclosamide-olamine + TX, nicotine + TX, nicotine sulfate + TX, nifluridide + TX, nikkomycins + TX, N-isopropyl-N'-[5-methoxy-2-methyl-4-(2,2,2-trifluoro-1-hydroxy-1-phenyl-ethyl)phenyl]-N-methyl-formamidine + TX, nithiazine + TX, nitrapyrin + TX, nitrilacarb + TX, nitrilacarb 1:1 zinc chloride complex + TX, nitrothal-isopropyl + TX, N-methoxy-N-[[4-[5-(trifluoromethyl)-1,2,4-oxadiazol-3-yl]phenyl]methyl]cyclopropanecarboxamide + TX, N-methyl-4-[5-(trifluoromethyl)-1,2,4-oxadiazol-3-yl]benzamide + TX, N-methyl-4-[5-(trifluoromethyl)-1,2,4-oxadiazol-3-yl]benzenecarbothioamide + TX, norbormide + TX, nuarimol + TX, O,O,O',O'-tetrapropyl dithiopyrophosphate + TX, octadeca-2,13-dien-1-yl acetate + TX, octadeca-3,13-dien-1-yl acetate + TX, octhilinone + TX, ofurace + TX, oleic acid + TX, omethoate + TX, orfralure + TX, Orius spp. + TX, oryctalure + TX, orysastrobin + TX, ostramone + TX, oxadixyl + TX, oxamate + TX, oxathiapiprolin + TX, oxine-copper + TX, oxolinic acid + TX, oxycarboxin + TX, oxydeprofos + TX, oxydisulfoton + TX, oxytetracycline + TX, paclobutrazole + TX, Paecilomyces fumosoroseus + TX, para-dichlorobenzene + TX, parathion + TX, parathion-methyl + TX, pefurazoate + TX, penconazole + TX, pencycuron + TX, penflufen + TX, penfluron + TX, pentachlorophenol + TX, pentachlorophenyl laurate + TX, penthiopyrad + TX, permethrin + TX, PH 60-38 + TX, phenamacril + TX, phenkapton + TX, phosacetim + TX, phosalone + TX, phosdiphen + TX, phosfolan + TX, phosglycin + TX, phosnichlor + TX, phosphamidon + TX, phosphine + TX, phosphorus + TX, phoxim-methyl + TX, phthalide + TX, Phytoseiulus persimilis + TX, picarbutrazox + TX, picaridin + TX, picoxystrobin + TX, pindone + TX, piperazine + TX, piperonyl butoxide + TX, piprotal + TX, pirimetaphos + TX, polychlorodicyclopentadiene isomers + TX, polychloroterpenes + TX, polynactins + TX, polyoxins + TX, potassium arsenite + TX, potassium ethylxanthate + TX, potassium hydroxyquinoline sulfate + TX, potassium thiocyanate + TX, pp'-DDT + TX, precocene I + TX, precocene II + TX, precocene III + TX, primidophos + TX, probenazole + TX, prochloraz + TX, proclonol + TX, procymidone + TX, profluthrin + TX, promacyl + TX, promecarb + TX, propamocarb + TX, propiconazole + TX, propineb + TX, propoxur + TX, propyl isomer + TX, proquinazid + TX, prothidathion + TX, prothioconazole + TX, prothiofos + TX, prothoate + TX, pydiflumetofen + TX, pyraclostrobin + TX, pyrametostrobin + TX, pyraoxystrobin + TX, pyrapropoyne + TX, pyraziflumid + TX, pyrazophos + TX, pyresmethrin + TX, pyrethrin I + TX, pyrethrin II + TX, pyrethrins + TX, pyribencarb + TX, pyridachlometyl + TX, pyridaphenthion + TX, pyridin-4-amine + TX, pyrifenox + TX, pyrimethanil + TX, pyrimitate + TX, pyrimorph + TX, pyrinuron + TX, pyriofenone + TX, pyrisoxazole + TX, pyroquilon + TX, quassia + TX, quinalphos + TX, quinalphos-methyl + TX, quinoclamine + TX, quinofumelin + TX, quinonamid + TX, quinothion + TX, quinoxyfen + TX, quintiofos + TX, quintozene + TX, R-1492 + TX, rafoxanide + TX, resmethrin + TX, Reynoutria sachalinensis extract + TX, ribavirin + TX, Rmetalaxyl + TX, rotenone + TX, ryania + TX, ryanodine + TX, S421 + TX, sabadilla + TX, schradan + TX, scilliroside + TX, seboctylamine + TX, sebufos + TX, sedaxane + TX, selamectin + TX, sesamex + TX, sesasmolin + TX, SI-0009 + TX, siglure + TX, simazine + TX, simeconazole + TX, sodium arsenite + TX, sodium cyanide + TX, sodium fluoride + TX, sodium fluoroacetate + TX, sodium hexafluorosilicate + TX, sodium pentachlorophenoxide + TX, sodium selenate + TX, sodium tetrathiocarbonate + TX, sodium thiocyanate + TX, sophamide + TX, sordidin + TX, spiroxamine + TX, SSI-121 + TX, Steinernema bibionis + TX, Steinernema carpocapsae + TX, Steinernema feltiae + TX, Steinernema glaseri + TX, Steinernema riobrave + TX, Steinernema riobravis + TX, Steinernema scapterisci + TX, Steinernema spp. + TX, streptomycin + TX, streptomycin sesquisulfate + TX, strychnine + TX, sulcatol + TX, sulcofuron + TX, sulcofuron-sodium + TX, sulfiram + TX, sulfluramid + TX, sulfotep + TX, sulfoxide + TX, sulfur + TX, sulfuryl fluoride + TX, sulprofos + TX, tar oils + TX, tau-fluvalinate + TX, tazimcarb + TX, TDE + TX, tebuconazole + TX, tebufloquin + TX, tebupirimfos + TX, tecloftalam + TX, temephos + TX, tepa + TX, TEPP + TX, terallethrin + TX, terbam + TX, tert-butyl N-[6-[[[(1-methyltetrazol-5-yl)-phenyl-methylene]amino]oxymethyl]-2-pyridyl]carbamate + TX, tetrachloroethane + TX, tetrachlorothiophene + TX, tetraconazole + TX, tetradec-11-en-1-yl acetate + TX, tetradifon + TX, tetramethylfluthrin + TX, tetrasul + TX, thallium sulfate + TX, thiabendazole + TX, thiafenox + TX, thiapronil + TX, thicrofos + TX, thifluzamide + TX, thiocarboxime + TX, thiocyclam + TX, thiocyclam hydrogen oxalate + TX, thiodiazole copper + TX, thiofanox + TX, thiohempa + TX, thiomersal + TX, thiometon + TX, thionazin + TX, thiophanate + TX, thiophanate-methyl + TX, thioquinox + TX, thiosultap + TX, thiosultap-sodium + TX, thiotepa + TX, thiram + TX, thuringiensin + TX, tiadinil + TX, tolclofos-methyl + TX, tolprocarb + TX, tolylfluanid + TX, tralomethrin + TX, transpermethrin + TX, tretamine + TX, triadimefon + TX, triadimenol + TX, triamiphos + TX, triarathene + TX, triazamate + TX, triazophos + TX, triazoxide + TX, triazuron + TX, tributyltin oxide + TX, trichlormetaphos-3 + TX, trichloronat + TX, Trichogramma spp. + TX, triclopyricarb + TX, tricyclazole + TX, tridemorph + TX, trifenmorph + TX, trifenofos + TX, trifloxystrobin + TX, triflumizole + TX, triforine + TX, trimedlure + TX, trimedlure A + TX, trimedlure B1 + TX, trimedlure B2 + TX, trimedlure C + TX, trimethacarb + TX, trinactin + TX, trinexapac + TX, triphenyltin acetate + TX, triphenyltin hydroxide + TX, triprene + TX, triticonazole + TX, trunc-call + TX, Typhlodromus occidentalis + TX, uredepa + TX, validamycin + TX, valifenalate + TX, vamidothion + TX, vaniliprole + TX, veratridine + TX, veratrine + TX, verbutin + TX, Verticillium lecanii + TX, vinclozoline + TX, warfarin + TX, XMC + TX, xylenols + TX, zeatin + TX, zetamethrin + TX, zhongshengmycin + TX, zinc naphthenate + TX, zinc phosphide + TX, zinc thiazole + TX, zineb + TX, ziram + TX;
Acinetobacter lwoffii + TX, Acremonium alternatum + TX, Acremonium cephalosporium + TX, Acremonium diospyri + TX, Acremonium obclavatum + TX, Adoxophyes orana granulovirus (AdoxGV) (Capex^{®}) + TX, Agrobacterium radiobacter strain K84 (Galltrol-A^{®}) + TX, Alternaria alternate + TX, Alternaria cassia + TX, Alternaria destruens (Smolder^{®}) + TX, Ampelomyces quisqualis (AQ10^{®}) + TX, Aspergillus flavus AF36 (AF36^{®}) + TX, Aspergillus flavus NRRL 21882 (Aflaguard^{®}) + TX, Aspergillus spp. + TX, Aureobasidium pullulans + TX, Azospirillum (MicroAZ^{®}, TAZO B^{®}) + TX, Azotobacter + TX, Azotobacter chroocuccum (Azotomeal^{®}) + TX, Azotobacter cysts (Bionatural Blooming Blossoms^{®}) + TX, Bacillus amyloliquefaciens + TX, Bacillus cereus + TX, Bacillus chitinosporus strain AQ746 + TX, Bacillus chitinosporus strain CM-1 + TX, Bacillus circulans + TX, Bacillus firmus (BioSafe^{®}, BioNem-WP^{®}) in particular strain CNMC 1-1582 (e.g. VOTIVO^{®} from BASF SE) + TX, Bacillus licheniformis strain 3086 (EcoGuard^{®}, Green Releaf^{®}) + TX, Bacillus licheniformis strain HB-2 (Biostart^{™} formerly Rhizoboost^{®}) + TX, Bacillus macerans + TX, Bacillus marismortui + TX, Bacillus megaterium + TX, Bacillus mycoides strain AQ726 + TX, Bacillus papillae (Milky Spore Powder^{®}) + TX, Bacillus pumilus spp. + TX, Bacillus pumilus strain AQ717 + TX, Bacillus pumilus strain GB34 (Yield Shield^{®}) + TX, Bacillus pumilus strain QST 2808 (Sonata^{®}, Ballad Plus^{®}) + TX, Bacillus sphaericus (VectoLex^{®}) + TX, Bacillus spp. + TX, Bacillus spp. strain AQ175 + TX, Bacillus spp. strain AQ177 + TX, Bacillus spp. strain AQ178 + TX, Bacillus subtilis strain AQ153 + TX, Bacillus subtilis strain AQ743 + TX, Bacillus subtilis strain QST 713 (CEASE^{®}, Serenade^{®}, Rhapsody^{®}) + TX, Bacillus subtilis strain QST 714 (JAZZ^{®}) + TX, Bacillus subtilis strain QST3002 + TX, Bacillus subtilis strain QST3004 + TX, Bacillus subtilis var. amyloliquefaciens strain FZB24 (Taegro^{®}, Rhizopro^{®}) + TX, Bacillus thuringiensis aizawai GC 91 (Agree^{®}) + TX, Bacillus thuringiensis Cry 2Ae + TX, Bacillus thuringiensis Cry1Ab + TX, Bacillus thuringiensis israelensis (BMP123^{®}, Aquabac^{®}, VectoBac^{®}) + TX, Bacillus thuringiensis kurstaki (Javelin^{®}, Deliver^{®}, CryMax^{®}, Bonide^{®}, Scutella WP^{®}, Turilav WP ^{®}, Astuto^{®}, Dipel WP^{®}, Biobit^{®}, Foray^{®}) + TX, Bacillus thuringiensis kurstaki BMP 123 (Baritone^{®}) + TX, Bacillus thuringiensis kurstaki HD-1 (Bioprotec-CAF / 3P^{®}) + TX, Bacillus thuringiensis strain AQ52 + TX, Bacillus thuringiensis strain BD#32 + TX, Bacillus thuringiensis tenebrionis (Novodor^{®}, BtBooster) + TX, Bacillus thuringiensis var. aizawai (XenTari^{®}, DiPel^{®}) + TX, bacteria spp. (GROWMEND^{®}, GROWSWEET^{®}, Shootup^{®}) + TX, bacteriophage of Clavipacter michiganensis (AgriPhage^{®}, Bakflor^{®}) + TX, Beauveria bassiana (Beaugenic^{®}, Brocaril WP^{®}) + TX, Beauveria bassiana GHA (Mycotrol ES^{®}, Mycotrol O^{®}, BotaniGuard^{®}) + TX, Beauveria brongniartii (Engerlingspilz^{®}, Schweizer Beauveria^{®}, Melocont^{®}) + TX, Beauveria spp. + TX, Botrytis cineria + TX, Bradyrhizobium japonicum (TerraMax^{®}) + TX, Brevibacillus brevis + TX, Burkholderia cepacia (Deny^{®}, Intercept^{®}, Blue Circle^{®}) + TX, Burkholderia gladii + TX, Burkholderia gladioli + TX, Burkholderia spp. + TX, Canadian thistle fungus (CBH Canadian Bioherbicide^{®}) + TX, Candida butyri + TX, Candida famata + TX, Candida fructus + TX, Candida glabrata + TX, Candida guilliermondii + TX, Candida melibiosica + TX, Candida oleophila strain O + TX, Candida parapsilosis + TX, Candida pelliculosa + TX, Candida pulcherrima + TX, Candida reukaufii + TX, Candida saitoana (Bio-Coat^{®}, Biocure^{®}) + TX, Candida sake + TX, Candida spp. + TX, Candida tenius + TX, Cedecea davisae + TX, Cellulomonas flavigena + TX, Chaetomium cochliodes (Nova-Cide^{®}) + TX, Chaetomium globosum (Nova-Cide^{®}) + TX, Chromobacterium subtsugae strain PRAA4-1T (Grandevo^{®}) + TX, Cladosporium chlorocephalum + TX, Cladosporium cladosporioides + TX, Cladosporium oxysporum + TX, Cladosporium spp. + TX, Cladosporium tenuissimum + TX, Clonostachys rosea (EndoFine^{®}) + TX, Colletotrichum acutatum + TX, Coniothyrium minitans (Cotans WG^{®}) + TX, Coniothyrium spp. + TX, Cryptococcus albidus (YIELDPLUS^{®}) + TX, Cryptococcus humicola + TX, Cryptococcus infirmo-miniatus + TX, Cryptococcus laurentii + TX, Cryptophlebia leucotreta granulovirus (Cryptex^{®}) + TX, Cupriavidus campinensis + TX, Cydia pomonella granulovirus (CYD-X^{®}, Madex^{®}, Madex^{®} Plus, Madex Max, Carpovirusine^{®} + TX, Cylindrobasidium laeve (Stumpout^{®}) + TX, Cylindrocladium + TX, Debaryomyces hansenii + TX, Drechslera hawaiinensis + TX, Enterobacter cloacae + TX, Enterobacteriaceae + TX, Entomophtora virulenta (Vektor^{®}) + TX, Epicoccum nigrum + TX, Epicoccum purpurascens + TX, Epicoccum spp. + TX, Filobasidium floriforme + TX, Fusarium acuminatum + TX, Fusarium chlamydosporum + TX, Fusarium oxysporum (Fusaclean^{®}, Biofox C^{®}) + TX, Fusarium proliferatum + TX, Fusarium spp. + TX, Galactomyces geotrichum + TX, Gliocladium catenulatum (Primastop^{®}, Prestop^{®}) + TX, Gliocladium roseum + TX, Gliocladium spp. (SoilGard^{®}) + TX, Gliocladium virens (Soilgard^{®}) + TX, Granulovirus (Granupom^{®}) + TX, Halobacillus halophilus + TX, Halobacillus litoralis + TX, Halobacillus trueperi + TX, Halomonas spp. + TX, Halomonas subglaciescola + TX, Halovibrio variabilis + TX, Hanseniaspora uvarum + TX, Helicoverpa armigera nucleopolyhedrovirus (Helicovex^{®}) + TX, Helicoverpa zea nuclear polyhedrosis virus (Gemstar^{®}) + TX, Isaria fumosorosea (previously known as Paecilomyces fumosoroseus strain, PFR-97^{®}, PreFeRal^{®}) + TX, Isoflavone formononetin (Myconate^{®}) + TX, Kloeckera apiculata + TX, Kloeckera spp. + TX, Lagenidium giganteum (Laginex^{®}) + TX, Lecanicillium lecanii (formerly known as Verticillium lecanii (Mycotal^{®}) conidia of strain KV01 (e.g. Vertalec^{®} by Koppert/Arysta) + TX, Lecanicillium longisporum (Vertiblast^{®}) + TX, Lecanicillium muscarium (Vertikil^{®}) + TX, Lymantria Dispar nucleopolyhedrosis virus (Disparvirus^{®}) + TX, Marinococcus halophilus + TX, Meira geulakonigii + TX, Metarhizium anisopliae (Destruxin WP^{®}) + TX, Metarhizium anisopliae (Met52^{®}) + TX, Metschnikowia fruticola (Shemer^{®}) + TX, Metschnikowia pulcherrima + TX, Microdochium dimerum (Antibot^{®}) + TX, Micromonospora coerulea + TX, Microsphaeropsis ochracea + TX, Muscodor albus 620 (Muscudor^{®}) + TX, Muscodor roseus in particular strain A3-5 (Accession No. NRRL 30548) + TX, Mycorrhizae spp. (AMykor^{®}, Root Maximizer^{®}) + TX, Myrothecium verrucaria strain AARC-0255 (DiTera^{®}, BROS PLUS^{®}) + TX, Ophiostoma piliferum strain D97 (Sylvanex^{®}) + TX, Paecilomyces farinosus + TX, Paecilomyces lilacinus strain 251 (MeloCon WG^{®}) + TX, Paecilomyces linacinus (Biostat WP^{®}) + TX, Paenibacillus polymyxa + TX, Pantoea agglomerans (BlightBan C9-1^{®}) + TX, Pantoea spp. + TX, Pasteuria nishizawae in particular strain Pn1 (CLARIVA from Syngenta/ChemChina); + TX, Pasteuria spp. (Econem^{®}) + TX, Penicillium aurantiogriseum + TX, Penicillium billai (Jumpstart^{®}, TagTeam^{®}) + TX, Penicillium brevicompactum + TX, Penicillium frequentans + TX, Penicillium griseofulvum + TX, Penicillium purpurogenum + TX, Penicillium spp. + TX, Penicillium viridicatum + TX, Phlebiopsis gigantean (Rotstop^{®}) + TX, phosphate solubilizing bacteria (Phosphomeal^{®}) + TX, Phytophthora cryptogea + TX, Phytophthora palmivora (Devine^{®}) + TX, Pichia anomala + TX, Pichia guilliermondii + TX, Pichia membranaefaciens + TX, Pichia onychis + TX, Pichia stipites + TX, Pseudomonas aeruginosa + TX, Pseudomonas aureofasciens (Spot-Less Biofungicide^{®}) + TX, Pseudomonas cepacia + TX, Pseudomonas chlororaphis (AtEze^{®}) + TX, Pseudomonas corrugate + TX, Pseudomonas fluorescens (Zequanox^{®}) + TX, Pseudomonas fluorescens strain A506 (BlightBan A506^{®}) + TX, Pseudomonas putida + TX, Pseudomonas reactans + TX, Pseudomonas spp. + TX, Pseudomonas syringae (Bio-Save^{®}) + TX, Pseudomonas viridiflava + TX, Pseudozyma flocculosa strain PF-A22 UL (Sporodex L^{®}) + TX, Puccinia canaliculata + TX, Puccinia thlaspeos (Wood Warrior^{®}) + TX, Pythium paroecandrum + TX, Pythium oligandrum (Polygandron^{®}, Polyversum^{®}) + TX, Pythium periplocum + TX, Rhanella aquatilis + TX, Rhanella spp. + TX, Rhizobia (Dormal^{®}, Vault^{®}) + TX, Rhizoctonia + TX, Rhodococcus globerulus strain AQ719 + TX, Rhodosporidium diobovatum + TX, Rhodosporidium toruloides + TX, Rhodotorula glutinis + TX, Rhodotorula graminis + TX, Rhodotorula mucilagnosa + TX, Rhodotorula rubra + TX, Rhodotorula spp. + TX, Saccharomyces cerevisiae + TX, Salinococcus roseus + TX, Sclerotinia minor (SARRITOR^{®}) + TX, Sclerotinia minor + TX, Scytalidium spp. + TX, Scytalidium uredinicola + TX, Serratia marcescens + TX, Serratia plymuthica + TX, Serratia spp. + TX, Sordaria fimicola + TX, Spodoptera exigua nuclear polyhedrosis virus (Spod-X^{®}, Spexit^{®}) + TX, Spodoptera littoralis nucleopolyhedrovirus (Littovir^{®}) + TX, Sporobolomyces roseus + TX, Stenotrophomonas maltophilia + TX, Streptomyces albaduncus + TX, Streptomyces exfoliates + TX, Streptomyces galbus + TX, Streptomyces griseoplanus + TX, Streptomyces griseoviridis (Mycostop^{®}) + TX, Streptomyces hygroscopicus + TX, Streptomyces lydicus (Actinovate^{®}) + TX, Streptomyces lydicus WYEC-108 (ActinoGrow^{®}) + TX, Streptomyces violaceus + TX, Tilletiopsis minor + TX, Tilletiopsis spp. + TX, Trichoderma asperellum (T34 Biocontrol^{®}) + TX, Trichoderma atroviride (Plantmate^{®}) + TX, Trichoderma gamsii (Tenet^{®}) + TX, Trichoderma hamatum TH 382 + TX, Trichoderma harzianum rifai (Mycostar^{®}) + TX, Trichoderma harzianum T-22 (Trianum-P^{®}, PlantShield HC^{®}, RootShield^{®}, Trianum-G^{®} + TX, Trichoderma harzianum T-39 (Trichodex^{®}) + TX, Trichoderma inhamatum + TX, Trichoderma koningii + TX, Trichoderma lignorum + TX, Trichoderma longibrachiatum + TX, Trichoderma polysporum (Binab T^{®}) + TX, Trichoderma spp. LC 52 (Sentinel^{®}) + TX, Trichoderma taxi + TX, Trichoderma virens (formerly Gliocladium virens GL-21) (SoilGuard^{®}) + TX, Trichoderma virens + TX, Trichoderma viride + TX, Trichoderma viride strain ICC 080 (Remedier^{®}) + TX, Trichosporon pullulans + TX, Trichosporon spp. + TX, Trichothecium roseum + TX, Trichothecium spp. + TX, Typhula phacorrhiza strain 94670 + TX, Typhula phacorrhiza strain 94671 + TX, Ulocladium atrum + TX, Ulocladium oudemansii (Botry-Zen^{®}) + TX, Ustilago maydis + TX, various bacteria and supplementary micronutrients (Natural II^{®}) + TX, various fungi (Millennium Microbes^{®}) + TX, Verticillium chlamydosporium + TX, Vip3Aa20 (VIPtera^{®}) + TX, Virgibaclillus marismortui + TX, Xanthomonas campestris pv. Poae (Camperico^{®}) + TX, Xenorhabdus bovienii + TX, Xenorhabdus nematophilus + TX;
AGNIQUE^{®} MMF + TX, azadirachtin (Plasma Neem Oil^{®}, AzaGuard^{®}, MeemAzal^{®}, Molt-X^{®} e.g. AZATIN XL from Certis, US) + TX, Botanical IGR (Neemazad^{®}, Neemix^{®}) + TX, BugOil^{®} + TX, canola oil (Lilly Miller Vegol^{®}) + TX, Chenopodium ambrosioides near ambrosioides (Requiem^{®}) + TX, Chrysanthemum extract (Crisant^{®}) + TX, essentials oils of Labiatae (Botania^{®}) + TX, extract of neem oil (Trilogy^{®}) + TX, extracts of clove rosemary peppermint and thyme oil (Garden insect killer^{®}) + TX, garlic + TX, Glycinebetaine (Greenstim^{®}) + TX, kaolin (Screen^{®}) + TX, lemongrass oil (GreenMatch^{®}) + TX, Melaleuca alternifolia extract (also called tea tree oil) (Timorex Gold^{®}) + TX, mixture of clove pepermint garlic oil and mint (Soil Shot^{®}) + TX, mixture of clove rosemary and peppermint extract (EF 400^{®}) + TX, mixture of rosemary sesame pepermint thyme and cinnamon extracts (EF 300^{®}) + TX, neem oil + TX, Nepeta cataria (Catnip oil) + TX, Nepeta catarina + TX, nicotine + TX, oregano oil (MossBuster^{®}) + TX, Pedaliaceae oil (Nematon^{®}) + TX, pine oil (Retenol^{®}) + TX, pyrethrum + TX, Quillaja saponaria (NemaQ^{®}) + TX, Reynoutria sachalinensis (Regalia^{®}, Sakalia^{®}) + TX, rotenone (Eco Roten^{®}) + TX, Rutaceae plant extract (Soleo^{®}) + TX, soybean oil (Ortho ecosense^{®}) + TX, storage glucam of brown algae (Laminarin^{®}) + TX, thyme oil + TX;
(E,Z)-7,9-Dodecadien-1-yl acetate + TX, (E,Z,Z)-3,8,11 Tetradecatrienyl acetate + TX, (Z,Z,E)-7,11,13-Hexadecatrienal + TX, 2-Methyl-1-butanol + TX, Biolure^{®} + TX, blackheaded fireworm pheromone (3M Sprayable Blackheaded Fireworm Pheromone^{®}) + TX, Calcium acetate + TX, Check-Mate^{®} + TX, Codling Moth Pheromone (Paramount dispenser-(CM)/ Isomate C-Plus^{®}) + TX, Entostat powder (extract from palm tree) (Exosex CM^{®}) + TX, Grape Berry Moth Pheromone (3M MEC-GBM Sprayable Pheromone^{®}) + TX, Lavandulyl senecioate + TX, Leafroller pheromone (3M MEC - LR Sprayable Pheromone^{®}) + TX, Muscamone (Snip7 Fly Bait^{®} + TX, Oriental Fruit Moth Pheromone (3M oriental fruit moth sprayable pheromone^{®}) + TX, Peachtree Borer Pheromone (Isomate-P^{®}) + TX, Scenturion^{®} + TX, Starbar Premium Fly Bait^{®}) + TX, Tomato Pinworm Pheromone (3M Sprayable pheromone^{®}) + TX;
Acerophagus papaya + TX, Adalia bipunctata (Adalia-System^{®}) + TX, Adalia bipunctata (Adaline^{®}) + TX, Adalia bipunctata (Aphidalia^{®}) + TX, Ageniaspis citricola + TX, Ageniaspis fuscicollis + TX, Amblyseius andersoni (Anderline^{®}, Andersoni-System^{®}) + TX, Amblyseius californicus (Amblyline^{®}, Spical^{®}) + TX, Amblyseius cucumeris (Thripex^{®}, Bugline cucumeris^{®}) + TX, Amblyseius fallacis (Fallacis^{®}) + TX, Amblyseius swirskii (Bugline swirskii^{®}, Swirskii-Mite^{®}) + TX, Amblyseius womersleyi (WomerMite^{®}) + TX, Amitus hesperidum + TX, Anagrus atomus + TX, Anagyrus fusciventris + TX, Anagyrus kamali + TX, Anagyrus loecki + TX, Anagyrus pseudococci (Citripar^{®}) + TX, Anicetus benefices + TX, Anisopteromalus calandrae + TX, Anthocoris nemoralis (Anthocoris-System^{®}) + TX, Aphelinus abdominalis (Apheline^{®}, Aphiline^{®}), + TX, Aphelinus asychis + TX, Aphidius colemani (Aphipar^{®}) + TX, Aphidius ervi (Aphelinus-System^{®}) + TX, Aphidius ervi (Ervipar^{®}) + TX, Aphidius gifuensis + TX, Aphidius matricariae (Aphipar-M^{®}) + TX, Aphidoletes aphidimyza (Aphidend^{®}, Aphidoline^{®}) + TX, Aphytis lingnanensis + TX, Aphytis melinus + TX, Aprostocetus hagenowii + TX, Atheta coriaria (Staphyline^{®}) + TX, Bombus spp. + TX, Bombus terrestris (Beeline^{®}, Tripol^{®}) + TX, Bombus terrestris (Natupol Beehive^{®}) + TX, Cephalonomia stephanoderis + TX, Chilocorus nigritus + TX, Chrysoperla carnea (Chrysoline^{®}, Chrysopa^{®}) + TX, Chrysoperla rufilabris + TX, Cirrospilus ingenuus + TX, Cirrospilus quadristriatus + TX, Citrostichus phyllocnistoides + TX, Closterocerus chamaeleon + TX, Closterocerus spp. + TX, Coccidoxenoides perminutus (Planopar^{®}) + TX, Coccophagus cowperi + TX, Coccophagus lycimnia + TX, Cotesia flavipes + TX, Cotesia plutellae + TX, Cryptolaemus montrouzieri (Cryptobug^{®}, Cryptoline^{®}) + TX, Cybocephalus nipponicus + TX, Dacnusa sibirica (Minusa^{®}, DacDigline^{®}, Minex^{®}) + TX, Delphastus catalinae (Delphastus^{®}) + TX, Delphastus pusillus + TX, Diachasmimorpha krausii + TX, Diachasmimorpha longicaudata + TX, Diaparsis jucunda + TX, Diaphorencyrtus aligarhensis + TX, Diglyphus isaea (Diminex^{®}, Miglyphus^{®}, Digline^{®}) + TX, Diversinervus spp. + TX, Encarsia citrina + TX, Encarsia formosa (Encarsia max^{®}, Encarline^{®}, En-Strip^{®}) + TX, Encarsia guadeloupae + TX, Encarsia haitiensis + TX, Episyrphus balteatus (Syrphidend^{®}) + TX, Eretmoceris siphonini + TX, Eretmocerus californicus + TX, Eretmocerus eremicus (Enermix^{®}, Ercal^{®}, Eretline e^{®}, Bemimix^{®}) + TX, Eretmocerus hayati + TX, Eretmocerus mundus (Bemipar^{®}, Eretline m^{®}) + TX, Eretmocerus siphonini + TX, Exochomus quadripustulatus + TX, Feltiella acarisuga (Feltiline^{®}) + TX, Feltiella acarisuga (Spidend^{®}) + TX, Fopius arisanus + TX, Fopius ceratitivorus + TX, Formononetin (Wirless Beehome^{®}) + TX, Franklinothrips vespiformis (Vespop^{®}) + TX, Galendromus occidentalis + TX, Goniozus legneri + TX, Habrobracon hebetor + TX, Harmonia axyridis (HarmoBeetle^{®}) + TX, Heterorhabditis bacteriophora (NemaShield HB^{®}, Nemaseek^{®}, Terranem-Nam^{®}, Terranem^{®}, Larvanem^{®}, B-Green^{®}, NemAttack^{®}, Nematop^{®}) + TX, Heterorhabditis megidis (Nemasys H^{®}, BioNem H^{®}, Exhibitline hm^{®}, Larvanem-M^{®}) + TX, Heterorhabditis spp. (Lawn Patrol^{®}) + TX, Hippodamia convergens + TX, Hypoaspis aculeifer (Aculeifer-System^{®}, Entomite-A^{®}) + TX, Hypoaspis miles (Hypoline m^{®}, Entomite-M^{®}) + TX, Lbalia leucospoides + TX, Lecanoideus floccissimus + TX, Lemophagus errabundus + TX, Leptomastidea abnormis + TX, Leptomastix dactylopii (Leptopar^{®}) + TX, Leptomastix epona + TX, Lindorus lophanthae + TX, Lipolexis oregmae + TX, Lucilia caesar (Natufly^{®}) + TX, Lysiphlebus testaceipes + TX, Macrolophus caliginosus (Mirical-NO, Macroline c^{®}, Mirical^{®}) + TX, Mesoseiulus longipes + TX, Metaphycus flavus + TX, Metaphycus lounsburyi + TX, Micromus angulatus (Milacewing^{®}) + TX, Microterys flavus + TX, Muscidifurax raptorellus and Spalangia cameroni (Biopar^{®}) + TX, Neodryinus typhlocybae + TX, Neoseiulus californicus + TX, Neoseiulus cucumeris (THRYPEX^{®}) + TX, Neoseiulus fallacis + TX, Nesideocoris tenuis (NesidioBug^{®}, Nesibug^{®}) + TX, Ophyra aenescens (Biofly^{®}) + TX, Orius insidiosus (Thripor-I^{®}, Oriline i^{®}) + TX, Orius laevigatus (Thripor-L^{®}, Oriline l^{®}) + TX, Orius majusculus (Oriline m^{®}) + TX, Orius strigicollis (Thripor-S^{®}) + TX, Pauesia juniperorum + TX, Pediobius foveolatus + TX, Phasmarhabditis hermaphrodita (Nemaslug^{®}) + TX, Phymastichus coffea + TX, Phytoseiulus macropilus + TX, Phytoseiulus persimilis (Spidex^{®}, Phytoline p^{®}) + TX, Podisus maculiventris (Podisus^{®}) + TX, Pseudacteon curvatus + TX, Pseudacteon obtusus + TX, Pseudacteon tricuspis + TX, Pseudaphycus maculipennis + TX, Pseudleptomastix mexicana + TX, Psyllaephagus pilosus + TX, Psyttalia concolor (complex) + TX, Quadrastichus spp. + TX, Rhyzobius lophanthae + TX, Rodolia cardinalis + TX, Rumina decollate + TX, Semielacher petiolatus + TX, Sitobion avenae (Ervibank^{®}) + TX, Steinernema carpocapsae (Nematac C^{®}, Millenium^{®}, BioNem C^{®}, NemAttack^{®}, Nemastar^{®}, Capsanem^{®}) + TX, Steinernema feltiae (NemaShield^{®}, Nemasys F^{®}, BioNem F^{®}, Steinernema-System^{®}, NemAttack^{®}, Nemaplus^{®}, Exhibitline sf^{®}, Scia-rid^{®}, Entonem^{®}) + TX, Steinernema kraussei (Nemasys L^{®}, BioNem L^{®}, Exhibitline srb^{®}) + TX, Steinernema riobrave (BioVector^{®}, BioVektor^{®}) + TX, Steinernema scapterisci (Nematac S^{®}) + TX, Steinernema spp. + TX, Steinernematid spp. (Guardian Nematodes^{®}) + TX, Stethorus punctillum (Stethorus^{®}) + TX, Tamarixia radiate + TX, Tetrastichus setifer + TX, Thripobius semiluteus + TX, Torymus sinensis + TX, Trichogramma brassicae (Tricholine b^{®}) + TX, Trichogramma brassicae (Tricho-Strip^{®}) + TX, Trichogramma evanescens + TX, Trichogramma minutum + TX, Trichogramma ostriniae + TX, Trichogramma platneri + TX, Trichogramma pretiosum + TX, Xanthopimpla stemmator + TX;
abscisic acid + TX, Aminomite^{®} + TX, BioGain^{®} + TX, bioSea^{®} + TX, Chondrostereum purpureum (Chontrol Paste^{®}) + TX, Colletotrichum gloeosporioides (Collego^{®}) + TX, Copper Octanoate (Cueva^{®}) + TX, Delta traps (Trapline d^{®}) + TX, Erwinia amylovora (Harpin) (ProAct^{®}, Ni-HIBIT Gold CST^{®}) + TX, fatty acids derived from a natural by-product of extra virgin olive oil (FLIPPER^{®}) + TX, Ferri-phosphate (Ferramol^{®}) + TX, Funnel traps (Trapline y^{®}) + TX, Gallex^{®} + TX, Grower's Secret^{®} + TX, Homo-brassonolide + TX, Iron Phosphate (Lilly Miller Worry Free Ferramol Slug & Snail Bait^{®}) + TX, MCP hail trap (Trapline f^{®}) + TX, Microctonus hyperodae + TX, Mycoleptodiscus terrestris (Des-X^{®}) + TX, Nosema locustae (Semaspore Organic Grasshopper Control^{®}) + TX, Pheromone trap (Thripline ams^{®}) + TX, potassium bicarbonate (MilStop^{®}) + TX, potassium iodide + potassiumthiocyanate (Enzicur^{®}) + TX, potassium salts of fatty acids (Sanova^{®}) + TX, potassium silicate solution (Sil-Matrix^{®}) + TX, Spider venom + TX, Sticky traps (Trapline YF^{®}, Rebell Amarillo^{®}) + TX, SuffOil-X^{®} + TX, Traps (Takitrapline y + b^{®}) + TX, vadescana (CAS Number: 2643947-26-4) + TX;
Bacillus mojavensis strain R3B (Accession No. NCAIM (P) B001389) (WO 2013/034938) from Certis USA LLC + TX, Bacillus pumilus, in particular strain BU F-33, having NRRL Accession No. 50185 (CARTISSA^{®} from BASF, EPA Reg. No. 71840-19) + TX, Bacillus subtilis CX-9060 from Certis USA LLC, Bacillus sp., in particular strain D747 (available as DOUBLE NICKEL^{®} from Kumiai Chemical Industry Co., Ltd.), having Accession No. FERM BP-8234, U.S. Patent No. 7,094,592 + TX, Bacillus subtilis strain BU1814, (VELONDIS^{®} PLUS, VELONDIS^{®} FLEX and VELONDIS^{®} EXTRA from BASF SE) + TX, Bacillus subtilis var. amyloliquefaciens strain FZB24 having Accession No. DSM 10271 (available from Novozymes as TAEGRO@ or TAEGRO@ ECO (EPA Registration No. 70127-5)) + TX, Bacillus subtilis, in particular strain QST713/AQ713 (having NRRL Accession No. B-21661 and described in U.S. Patent No. 6,060,051, available as SERENADE^{®} OPTI or SERENADE^{®} ASO from Bayer CropScience LP, US) + TX, Paenibacillus polymyxa, in particular strain AC-1 (e.g. TOPSEED^{®} from Green Biotech Company Ltd.) + TX, Paenibacillus sp. strain having Accession No. NRRL B-50972 or Accession No. NRRL B-67129, WO 2016/154297 + TX, Pantoea agglomerans, in particular strain E325 (Accession No. NRRL B-21856) (available as BLOOMTIME BIOLOGICAL^{™} FD BIOPESTICIDE from Northwest Agri Products) + TX, Pseudomonas proradix (e.g. PRORADIX^{®} from Sourcon Padena) + TX;
Aureobasidium pullulans, in particular blastospores of strain DSM14940, blastospores of strain DSM 14941 or mixtures of blastospores of strains DSM14940 and DSM14941 (e.g., BOTECTOR^{®} and BLOSSOM PROTECT^{®} from bio-ferm, CH) + TX, Pseudozyma aphidis (as disclosed in WO2011/151819 by Yissum Research Development Company of the Hebrew University of Jerusalem) + TX, Saccharomyces cerevisiae, in particular strains CNCM No. 1-3936, CNCM No. 1-3937, CNCM No. 1-3938 or CNCM No. 1-3939 (WO 2010/086790) from Lesaffre et Compagnie, FR + TX;
Agrobacterium radiobacter strain K84 (e.g. GALLTROL-A^{®} from AgBioChem, CA) + TX, Bacillus amyloliquefaciens isolate B246 (e.g. AVOGREEN^{™} from University of Pretoria) + TX, Bacillus amyloliquefaciens strain F727 (also known as strain MBI110) (NRRL Accession No. B-50768, WO 2014/028521) (STARGUS^{®} from Marrone Bio Innovations) + TX, Bacillus amyloliquefaciens strain FZB42, Accession No. DSM 23117 (available as RHIZOVITAL^{®} from ABiTEP, DE) + TX, Bacillus amyloliquefaciens, in particular strain D747 (available as Double Nickel^{™} from Kumiai Chemical Industry Co., Ltd., having accession number FERM BP-8234, US Patent No. 7,094,592) + TX, Bacillus licheniformis FMCH001 and Bacillus subtilis FMCH002 (QUARTZO^{®} (WG) and PRESENCE^{®} (WP) from FMC Corporation) + TX, Bacillus licheniformis, in particular strain SB3086, having Accession No. ATCC 55406, WO 2003/000051 (available as ECOGUARD^{®} Biofungicide and GREEN RELEAF^{™} from Novozymes) + TX, Bacillus methylotrophicus strain BAC-9912 (from Chinese Academy of Sciences' Institute of Applied Ecology) + TX, Bacillus mycoides, isolate, having Accession No. B-30890 (available as BMJ TGAI^{®} or WG and LifeGard^{™} from Certis USA LLC) + TX, Bacillus pumilus, in particular strain GB34 (available as Yield Shield^{®} from Bayer AG, DE) + TX, Bacillus pumilus, in particular strain QST2808 (available as SONATA^{®} from Bayer CropScience LP, US, having Accession No. NRRL B-30087 and described in U.S. Patent No. 6,245,551) + TX, Bacillus subtilis CX-9060 from Certis USA LLC + TX, Bacillus subtilis IAB/BS03 (AVIV^{™} from STK Bio-Ag Technologies, PORTENTO^{®} from Idai Nature) + TX, Bacillus subtilis KTSB strain (FOLIACTIVE^{®} from Donaghys) + TX, Bacillus subtilis strain BU1814, (available as VELONDIS^{®} PLUS, VELONDIS^{®} FLEX and VELONDIS^{®} EXTRA from BASF SE) + TX, Bacillus subtilis strain GB03 (available as Kodiak^{®} from Bayer AG, DE) + TX, Bacillus subtilis strain MBI 600 (available as SUBTILEX from BASF SE), having Accession Number NRRL B-50595, U.S. Patent No. 5,061,495 + TX, Bacillus subtilis strain Y1336 (available as BIOBAC^{®} WP from Bion-Tech, Taiwan, registered as a biological fungicide in Taiwan under Registration Nos. 4764, 5454, 5096 and 5277) + TX, Bacillus subtilis var. amyloliquefaciens strain FZB24 having Accession No. DSM 10271 (available from Novozymes as TAEGRO@ or TAEGRO@ ECO (EPA Registration No. 70127-5)) + TX, Bacillus subtilis Y1336 (available as BIOBAC^{®} WP from Bion-Tech, Taiwan, registered as a biological fungicide in Taiwan under Registration Nos. 4764, 5454, 5096 and 5277) + TX, Paenibacillus epiphyticus (WO 2016/020371) from BASF SE + TX, Paenibacillus polymyxa ssp. plantarum (WO 2016/020371) from BASF SE + TX, Paenibacillus sp. strain having Accession No. NRRL B-50972 or Accession No. NRRL B-67129, WO 2016/154297 + TX, Pseudomonas chlororaphis strain AFS009, having Accession No. NRRL B-50897, WO 2017/019448 (e.g., HOWLER^{™} and ZIO^{®} from AgBiome Innovations, US) + TX, Pseudomonas chlororaphis, in particular strain MA342 (e.g. CEDOMON^{®}, CERALL^{®}, and CEDRESS^{®} by Bioagri and Koppert) + TX, Pseudomonas fluorescens strain A506 (e.g. BLIGHTBAN^{®} A506 by NuFarm) + TX, Pseudomonas proradix (e.g. PRORADIX^{®} from Sourcon Padena) + TX, Streptomyces griseoviridis strain K61 (also known as Streptomyces galbus strain K61) (Accession No. DSM 7206) (MYCOSTOP^{®} from Verdera, PREFENCE^{®} from BioWorks, cf. Crop Protection 2006, 25, 468-475) + TX, Streptomyces lydicus strain WYEC108 (also known as Streptomyces lydicus strain WYCD108US) (ACTINO-IRON^{®} and ACTINOVATEO from Novozymes) + TX;
Trichoderma atroviride strain T11 (IMI352941/ CECT20498) + TX, Ampelomyces quisqualis strain AQ10, having Accession No. CNCM 1-807 (e.g., AQ 100 by IntrachemBio Italia) + TX, Ampelomyces quisqualis, in particular strain AQ 10 (e.g. AQ 100 by IntrachemBio Italia) + TX, Aspergillus flavus strain NRRL 21882 (products known as AFLA-GUARD^{®} from Syngenta/ChemChina) + TX, Aureobasidium pullulans, in particular blastospores of strain DSM 14941 + TX, Aureobasidium pullulans, in particular blastospores of strain DSM14940 + TX, Aureobasidium pullulans, in particular mixtures of blastospores of strains DSM14940 and DSM 14941 (e.g. Botector^{®} by bio-ferm, CH) + TX, Chaetomium cupreum (Accession No. CABI 353812) (e.g. BIOKUPRUM^{™} by AgriLife) + TX, Chaetomium globosum (available as RIVADIOM^{®} by Rivale) + TX, Cladosporium cladosporioides, strain H39, having Accession No. CBS122244, US 2010/0291039 (by Stichting Dienst Landbouwkundig Onderzoek) + TX, Coniothyrium minitans, in particular strain CON/M/91-8 (Accession No. DSM9660, e.g. Contans ^{®} from Bayer CropScience Biologics GmbH) + TX, Cryptococcus flavescens, strain 3C (NRRL Y-50378), + TX, Dactylaria candida, Dilophosphora alopecuri (available as TWIST FUNGUS^{®}), Fusarium oxysporum, strain Fo47 (available as FUSACLEAN^{®} by Natural Plant Protection) + TX, Gliocladium catenulatum (Synonym: Clonostachys rosea f. catenulate) strain J1446 (e.g. Prestop ^{®} by Lallemand) + TX, Gliocladium roseum (also known as Clonostachys rosea f rosea) strain IK726 (Jensen DF, et al. Development of a biocontrol agent for plant disease control with special emphasis on the near commercial fungal antagonist Clonostachys rosea strain 'IK726', Australasian Plant Pathol. 2007,36(2):95-101) + TX, Gliocladium roseum (also known as Clonostachys rosea f rosea), in particular strain 321U from Adjuvants Plus, strain ACM941 as disclosed in Xue A.G. (Efficacy of Clonostachys rosea strain ACM941 and fungicide seed treatments for controlling the root tot complex of field pea, Can Jour Plant Sci 2003, 83(3): 519-524) + TX, Metschnikowia fructicola, in particular strain NRRL Y-30752 + TX, Microsphaeropsis ochracea, Penicillium steckii (DSM 27859, WO 2015/067800) from BASF SE + TX, mixtures of Trichoderma asperellum strain ICC 012 (also known as Trichoderma harzianum ICC012), having Accession No. CABI CC IMI 392716 and Trichoderma gamsii (formerly T. viride) strain ICC 080, having Accession No. IMI 392151 (e.g., BIO-TAM^{™} from Isagro USA, Inc. or BIODERMA^{®} by Agrobiosol de Mexico, S.A. de C.V.) + TX, Penicillium vermiculatum + TX, Phlebiopsis gigantea strain VRA 1992 (ROTSTOP^{®} C from Danstar Ferment) + TX, Pseudozyma flocculosa, strain PF-A22 UL (available as SPORODEX^{®} L by Plant Products Co., CA) + TX, Saccharomyces cerevisiae strain LAS117 cell walls (CEREVISANE^{®} from Lesaffre, ROMEO^{®} from BASF SE) + TX, Saccharomyces cerevisiae strains CNCM No. 1-3936, CNCM No. 1-3937, CNCM No. 1-3938, CNCM No. 1-3939 (WO 2010/086790) from Lesaffre et Compagnie, FR + TX, Saccharomyces cerevisiae, in particular strain LASO2 (from Agro-Levures et Dérivés) + TX, Simplicillium lanosoniveum + TX, strain T34 (e.g. T34 Biocontrol by Biocontrol Technologies S.L., ES) or strain ICC 012 from Isagro + TX, strain WRL-076 (NRRL Y-30842), U.S. Patent No. 7,579,183 + TX, Talaromyces flavus, strain V117b + TX, Trichoderma asperelloides JM41R (Accession No. NRRL B-50759) (TRICHO PLUS^{®} from BASF SE) + TX, Trichoderma asperellum, in particular strain SKT-1, having Accession No. FERM P-16510 (e.g. ECO-HOPE^{®} from Kumiai Chemical Industry) + TX, Trichoderma asperellum, in particular, strain kd (e.g. T-Gro from Andermatt Biocontrol) + TX, Trichoderma atroviride strain 77B (T77 from Andermatt Biocontrol) + TX, Trichoderma atroviride strain ATCC 20476 (IMI 206040) + TX, Trichoderma atroviride strain LC52 (e.g. Tenet by Agrimm Technologies Limited) + TX, Trichoderma atroviride strain LU132 (e.g. Sentinel from Agrimm Technologies Limited) + TX, Trichoderma atroviride strain NMI no. V08/002388 + TX, Trichoderma atroviride strain NMI no. V08/002389 + TX, Trichoderma atroviride strain NMI no. V08/002390 + TX, Trichoderma atroviride strain no. V08/002387 + TX, Trichoderma atroviride strain SKT-1 (FERM P-16510), JP Patent Publication (Kokai) 11-253151 A + TX, Trichoderma atroviride strain SKT-2 (FERM P-16511), JP Patent Publication (Kokai) 11-253151 A + TX, Trichoderma atroviride strain SKT-3 (FERM P-17021), JP Patent Publication (Kokai) 11-253151 A + TX, Trichoderma atroviride, in particular strain SC1 (Accession No. CBS 122089, WO 2009/116106 and U.S. Patent No. 8,431,120 (from Bi-PA)) + TX, Trichoderma atroviride,strain CNCM 1-1237 (e.g. Esquive^{®} WP from Agrauxine, FR) + TX, Trichoderma fertile (e.g. product TrichoPlus from BASF) + TX, Trichoderma gamsii (formerly T. viride) + TX, Trichoderma gamsii (formerly T. viride) strain ICC 080 (IMI CC 392151 CABI) (available as BIODERMA^{®} by AGROBIOSOL DE MEXICO, S.A. DE C.V.), + TX, Trichoderma gamsii strain ICC080 (IMI CC 392151 CABI, e.g. BioDerma by AGROBIOSOL DE MEXICO, S.A. DE C.V.), + TX, Trichoderma harmatum + TX, Trichoderma harmatum, having Accession No. ATCC 28012 + TX, Trichoderma harzianum + TX, Trichoderma harzianum rifai T39 (e.g. Trichodex^{®} from Makhteshim, US) + TX, Trichoderma harzianum strain Cepa SimbT5 (from Simbiose Agro), + TX, Trichoderma harzianum strain DB 103 (available as T-GRO^{®} 7456 by Dagutat Biolab) + TX, Trichoderma harzianum strain ITEM 908 (e.g. Trianum-P from Koppert) + TX, Trichoderma harzianum strain T-22 (e.g. Trianum-P from Andermatt Biocontrol or Koppert) + TX, Trichoderma harzianum strain TH35 (e.g. Root-Pro by Mycontrol) + TX, Trichoderma polysporum strain IMI 206039 (e.g. Binab TF WP by BINAB Bio-Innovation AB, Sweden) + TX, Trichoderma stromaticum having Accession No. Ts3550 (e.g. Tricovab by CEPLAC, Brazil) + TX, Trichoderma virens (also known as Gliocladium virens) in particular strain GL-21 (e.g. SoilGard by Certis, US) + TX, Trichoderma virens strain G-41, formerly known as Gliocladium virens (Accession No. ATCC 20906) (e.g., ROOTSHIELD^{®} PLUS WP and TURFSHIELD^{®} PLUS WP from BioWorks, US) + TX, Trichoderma viride in particular strain B35 (Pietr et al., 1993, Zesz. Nauk. A R w Szczecinie 161: 125-137) + TX, Trichoderma viride strain TV1 (e.g. Trianum-P by Koppert) + TX, Ulocladium oudemansii strain U3, having Accession No. NM 99/06216 (e.g., BOTRY-ZEN^{®} by Botry-Zen Ltd, New Zealand and BOTRYSTOP^{®} from BioWorks, Inc.) + TX, Verticillium albo-atrum (formerly V. dahliae) strain WCS850 having Accession No. WCS850, deposited at the Central Bureau for Fungi Cultures (e.g., DUTCH TRIG^{®} by Tree Care Innovations) + TX, Verticillium chlamydosporium + TX;
a mixture of Azotobacter vinelandii and Clostridium pasteurianum (available as INVIGORATE^{®} from Agrinos) + TX, a mixture of Bacillus licheniformis FMCH001 and Bacillus subtilis FMCH002 (available as QUARTZO^{®} (WG), PRESENCE^{®} (WP) from FMC Corporation) + TX, Azorhizobium caulinodans, in particular strain ZB-SK-5 + TX, Azospirillum brasilense (e.g., VIGOR^{®} from KALO, Inc.) + TX, Azospirillum lipoferum (e.g., VERTEX-IF^{™} from TerraMax, Inc.) + TX, Azotobacter chroococcum, in particular strain H23 + TX, Azotobacter vinelandii, in particular strain ATCC 12837 + TX, Bacillus amyloliquefaciens BS27 (Accession No. NRRL B-5015) + TX, Bacillus amyloliquefaciens in particular strain FZB42 (e.g. RHIZOVITAL^{®} from ABiTEP, DE) + TX, Bacillus amyloliquefaciens in particular strain IN937a + TX, Bacillus amyloliquefaciens pm414 (LOLI-PEPTA^{®} from Biofilm Crop Protection) + TX, Bacillus amyloliquefaciens SB3281 (ATCC # PTA-7542, WO 2017/205258) + TX, Bacillus amyloliquefaciens TJ1000 (available as QUIKROOTS^{®} from Novozymes) + TX, Bacillus cereus family member EE128 (NRRL No. B-50917) + TX, Bacillus cereus family member EE349 (NRRL No. B-50928) + TX, Bacillus cereus in particular strain BP01 (ATCC 55675, e.g. MEPICHLOR^{®} from Arysta Lifescience, US) + TX, Bacillus mycoides BT155 (NRRL No. B-50921) + TX, Bacillus mycoides BT46-3 (NRRL No. B-50922) + TX, Bacillus mycoides EE118 (NRRL No. B-50918) + TX, Bacillus mycoides EE141 (NRRL No. B-50916) + TX, Bacillus pumilus in particular strain GB34 (e.g. YIELD SHIELD^{®} from Bayer Crop Science, DE), + TX, Bacillus pumilus in particular strain QST2808 (Accession No. NRRL No. B-30087) + TX, Bacillus siamensis in particular strain KCTC 13613T + TX, Bacillus subtilis in particular strain AQ30002 (Accession No. NRRL No. B-50421 and described in U.S. Patent Application No. 13/330,576) + TX, Bacillus subtilis in particular strain AQ30004 (NRRL No. B-50455 and described in U.S. Patent Application No. 13/330,576) + TX, Bacillus subtilis in particular strain MBI 600 (e.g. SUBTILEX^{®} from BASF SE) + TX, Bacillus subtilis rm303 (RHIZOMAX^{®} from Biofilm Crop Protection) + TX, Bacillus subtilis strain BU1814 (available as TEQUALIS^{®} from BASF SE) + TX, Bacillus tequilensis in particular strain NII-0943 + TX, Bacillus thuringiensis BT013A (NRRL No. B-50924) also known as Bacillus thuringiensis 4Q7 + TX, Bradyrhizobium japonicum (e.g. OPTIMIZE^{®} from Novozymes) + TX, Delftia acidovorans in particular strain RAY209 (e.g. BIOBOOST^{®} from Brett Young Seeds) + TX, Lactobacillus sp. (e.g. LACTOPLANT^{®} from LactoPAFI) + TX, Mesorhizobium cicer (e.g., NODULATOR from BASF SE) + TX, Paenibacillus polymyxa in particular strain AC-1 (e.g. TOPSEED^{®} from Green Biotech Company Ltd.) + TX, Pseudomonas aeruginosa in particular strain PN1 + TX, Pseudomonas proradix (e.g. PRORADIX^{®} from Sourcon Padena) + TX, Rhizobium leguminosarium biovar viciae (e.g., NODULATOR from BASF SE) + TX, Rhizobium leguminosarum in particular bv. viceae strain Z25 (Accession No. CECT 4585) + TX, Serratia marcescens in particular strain SRM (Accession No. MTCC 8708), + TX, Sinorhizobium meliloti strain NRG-185-1 (NITRAGIN^{®} GOLD from Bayer CropScience) + TX, Thiobacillus sp. (e.g. CROPAID^{®} from Cropaid Ltd UK) + TX;
Myrothecium verrucaria strain AARC-0255 (e.g. DiTera^{™} from Valent Biosciences) + TX, Penicillium bilaii strain ATCC 22348 (e.g. JumpStart^{®} from Acceleron BioAg) + TX, Penicillium bilaii strain ATCC ATCC20851 + TX, Purpureocillium lilacinum (previously known as Paecilomyces lilacinus) strain 251 (AGAL 89/030550, e.g. BioAct from Bayer CropScience Biologics GmbH) + TX, Pythium oligandrum strain DV74 + TX, Pythium oligandrum strain M1 (ATCC 38472 e.g. Polyversum from Bioprepraty, CZ) + TX, Rhizopogon amylopogon (Myco-Sol from Agri-Enterprise, LLC, formerly Helena Chemical Company) + TX, Rhizopogon fulvigleba (e.g. Myco-Sol from Agri-Enterprise, LLC, formerly Helena Chemical Company) + TX, Talaromyces flavus strain V117b + TX, Trichoderma asperellum strain (Eco-T from Plant Health Products, ZA) + TX, Trichoderma asperellum strain kd (e.g. T-Gro from Andermatt Biocontrol) + TX, Trichoderma atroviride in particular strain no. V08/002387 + TX, Trichoderma atroviride strain CNCM 1-1237 (e.g. Esquive^{®} WP from Agrauxine, FR) + TX, Trichoderma atroviride strain LC52 (also known as Trichoderma atroviride strain LU132, e.g. Sentinel from Agrimm Technologies Limited) + TX, Trichoderma atroviride strain no. NMI No. V08/002388 + TX, Trichoderma atroviride strain no. NMI No. V08/002389 + TX, Trichoderma atroviride strain no. NMI No. V08/002390 + TX, Trichoderma atroviride strain SC1 (described in WO2009/116106) + TX, Trichoderma harzianum strain 1295-22 + TX, Trichoderma harzianum strain ITEM 908 + TX, Trichoderma harzianum strain T-22 (e.g. Trianum-P from Andermatt Biocontrol or Koppert) + TX, Trichoderma harzianum strain TSTh20, + TX, Trichoderma virens strain GI-3 + TX, Trichoderma virens strain GL-21 (e.g. SoilGard^{®} from Certis, USA) + TX, Trichoderma viride strain B35 (Pietr et al., 1993, Zesz. Nauk. A R w Szczecinie 161: 125-137) + TX, Verticillium albo-atrum (formerly V. dahliae) strain WCS850 (CBS 276.92, e.g. Dutch Trig from Tree Care Innovations) + TX;
Agrobacterium radiobacter strain K84 (Galltrol from AgBiochem Inc.), + TX, Bacillus amyloliquefaciens in particular strain PTS-4838 (e.g. AVEO from Valent Biosciences, US), + TX, Bacillus mycoides, isolate J. (e.g. BmJ from Certis USA LLC), + TX, Bacillus sphaericus in particular Serotype H5a5b strain 2362 (strain ABTS-1743) (e.g. VECTOLEX^{®} from Valent BioSciences, US), + TX, Bacillus thuringiensis israelensis strain BMP 144 (e.g. AQUABAC^{®} by Becker Microbial Products IL) + TX, Bacillus thuringiensis subsp. aizawai strain GC-91 + TX, Bacillus thuringiensis subsp. aizawai, in particular serotype H-7 (e.g. FLORBAC^{®} WG from Valent BioSciences, US) + TX, Bacillus thuringiensis subsp. aizawai, in particular strain ABTS-1857 (SD-1372, e.g. XENTARI^{®} from Valent BioSciences) + TX, Bacillus thuringiensis subsp. israelensis (serotype H-14) strain AM65-52 (Accession No. ATCC 1276) (e.g. VECTOBAC^{®} by Valent BioSciences, US) + TX, Bacillus thuringiensis subsp. kurstaki strain ABTS 351 + TX, Bacillus thuringiensis subsp. kurstaki strain BMP 123 (from Becker Microbial Products, IL, BARITONE from Bayer CropScience) + TX, Bacillus thuringiensis subsp. kurstaki strain EG 2348 (LEPINOX from Certis, US) + TX, Bacillus thuringiensis subsp. kurstaki strain EG 7841 (CRYMAX from Certis, US) + TX, Bacillus thuringiensis subsp. kurstaki strain HD-1 (e.g. DIPEL^{®} ES from Valent BioSciences, US) + TX, Bacillus thuringiensis subsp. kurstaki strain PB 54 + TX, Bacillus thuringiensis subsp. kurstaki strain SA 11 (JAVELIN from Certis, US) + TX, Bacillus thuringiensis subsp. kurstaki strain SA 12 (THURICIDE from Certis, US) + TX, Bacillus thuringiensis subsp. tenebrionis strain NB 176 (SD-5428, e.g. NOVODOR^{®} FC from BioFa DE) + TX, Bacillus thuringiensis var. Colmeri (e.g. TIANBAOBTC by Changzhou Jianghai Chemical Factory) + TX, Bacillus thuringiensis var. japonensis strain Buibui + TX, Bacillus thuringiensis var. kurstaki strain EVB-113-19 (e.g., BIOPROTEC^{®} from AEF Global) + TX, Brevibacillus laterosporus + TX, Burkholderia spp. in particular Burkholderia rinojensis strain A396 (also known as Burkholderia rinojensis strain MBI 305) (Accession No. NRRL B-50319, WO 2011/106491 and WO 2013/032693, e.g. MBI206 TGAI and ZELTO^{®} from Marrone Bio Innovations), + TX, Chromobacterium subtsugae in particular strain PRAA4-1T (e.g. MBI-203, e.g. GRANDEVO^{®} from Marrone Bio Innovations) + TX, Lecanicillium muscarium Ve6 (MYCOTAL from Koppert) + TX, Paenibacillus popilliae (formerly Bacillus popilliae, e.g. MILKY SPORE POWDER^{™} or MILKY SPORE GRANULAR^{™} from St. Gabriel Laboratories) + TX, Serratia entomophila (e.g. INVADE^{®} by Wrightson Seeds) + TX, Serratia marcescens in particular strain SRM (Accession No. MTCC 8708) + TX, Trichoderma asperellum (TRICHODERMAX from Novozymes) + TX, Wolbachia pipientis ZAP strain (e.g., ZAP MALES^{®} from MosquitoMate) + TX;
Beauveria bassiana strain ATCC 74040 (e.g. NATURALISO from Intrachem Bio Italia) + TX, Beauveria bassiana strain ATP02 (Accession No. DSM 24665), Apopka 97 (PREFERAL from SePRO) + TX, Beauveria bassiana strain GHA (Accession No. ATCC74250, e.g. BOTANIGUARD^{®} ES and MYCONTROL-O^{®} from Laverlam International Corporation) + TX, Metarhizium anisopliae 3213-1 (deposited under NRRL accession number 67074 disclosed in WO 2017/066094, Pioneer Hi-Bred International) + TX, Metarhizium robertsii 15013-1 (deposited under NRRL accession number 67073) + TX, Metarhizium robertsii 23013-3 (deposited under NRRL accession number 67075) + TX, Paecilomyces lilacinus strain 251 (MELOCON from Certis, US) + TX;
Cydia pomonella (codling moth) granulosis virus (GV) + TX, Helicoverpa armigera (cotton bollworm) nuclear polyhedrosis virus (NPV) + TX, of Adoxophyes orana (summer fruit tortrix) granulosis virus (GV) + TX, Spodoptera exigua (beet armyworm) mNPV + TX, Spodoptera frugiperda (fall armyworm) mNPV + TX;
Burkholderia spp. in particular Burkholderia cepacia (formerly known as Pseudomonas cepacia) + TX, Gigaspora spp. + TX, Glomus spp. + TX, Laccaria spp. + TX, LactoBacillus buchneri + TX, Paraglomus spp. + TX, Pisolithus tinctorus + TX, Pseudomonas spp. + TX, Rhizobium spp. in particular Rhizobium trifolii + TX, Rhizopogon spp. + TX, Scleroderma spp. + TX, Streptomyces spp. + TX, Suillus spp. + TX, Agrobacterium spp. + TX, Azorhizobium caulinodans + TX, Azospirillum spp. + TX, Azotobacter spp. + TX, Bradyrhizobium spp. + TX, Gigaspora monosporum + TX;
Allium sativum (NEMGUARD from Eco-Spray, BRALIC from ADAMA) + TX, Armour-Zen + TX, Artemisia absinthium + TX, Biokeeper WP + TX, Brassicaceae extract in particular oilseed rape powder or mustard powder + TX, Cassia nigricans + TX, Celastrus angulatus + TX, Chenopodium anthelminticum + TX, Chenopodium quinoa saponin extract from quinoa seeds (e.g. Heads Up^{®} (Saponins of Quinoa) from Heads Up plant Protectants, CA) + TX, Chitin + TX, Dryopteris filix-mas + TX, Equisetum arvense + TX, Fortune Aza + TX, Fungastop + TX, Melaleuca alternifolia extract (TIMOREX GOLD from STK) + TX, naturally occurring Blad polypeptide extracted from Lupin seeds (FRACTURE^{®} from FMC) + TX, naturally occurring Blad polypeptide extracted from Lupin seeds (PROBLAD^{®} from Certis EU) + TX, Pyrethrins + TX, Quassia amara + TX, Quercus + TX, Quillaja extract (QL AGRI 35 from BASF) + TX, REGALIA MAXX from Marrone Bio) + TX, Requiem^{™} Insecticide + TX, Reynoutria sachalinensis extract (REGALLIA + TX, ryania/ryanodine + TX, Symphytum officinale + TX, Tanacetum vulgare + TX, Thymol + TX, Thymol mixed with Geraniol (CEDROZ from Eden Research) + TX, Thymol mixed with Geraniol and Eugenol (MEVALONE from Eden Research) + TX, Triact 70 + TX, TriCon + TX, Tropaeulum majus + TX, Urtica dioica + TX, Veratrin + TX, Viscum album + TX;
mercuric oxide + TX, octhilinone + TX, thiophanate-methyl + TX;
MGK 264 + TX, 2-(2-butoxyethoxy)ethyl piperonylate + TX, 2-isovalerylindan-1,3-dione + TX, 4-(quinoxalin-2-ylamino)benzenesulfonamide + TX, 5-(1,3-benzodioxol-5-yl)-3-hexylcyclohex-2-enone + TX, acibenzolar + TX, acibenzolar-S-methyl + TX, alpha-bromadiolone + TX, alpha-chlorohydrin + TX, aluminium phosphide + TX, anthraquinone + TX, antu + TX, arsenous oxide + TX, barium carbonate + TX, benoxacor + TX, bisthiosemi + TX, brodifacoum + TX, bromadiolone + TX, bromethalin + TX, calcium cyanide + TX, chloralose + TX, chlorophacinone + TX, cholecalciferol + TX, cloquintocet (including cloquintocet-mexyl) + TX, copper naphthenate + TX, copper oxychloride + TX, coumachlor + TX, coumafuryl + TX, coumatetralyl + TX, crimidine + TX, cyprosulfamide + TX, diazinon + TX, dichlormid + TX, dicyclopentadiene + TX, difenacoum + TX, difethialone + TX, diphacinone + TX, ergocalciferol + TX, farnesol + TX, farnesol with nerolidol + TX, fenchlorazole (including fenchlorazole-ethyl) + TX, fenclorim + TX, flocoumafen + TX, fluoroacetamide + TX, flupropadine + TX, flupropadine hydrochloride + TX, fluxofenim + TX, furilazole + TX, gamma-HCH + TX, guazatine + TX, guazatine acetates + TX, HCH + TX, hydrogen cyanide + TX, imanin + TX, iodomethane + TX, isoxadifen (including isoxadifen-ethyl) + TX, lindane + TX, magnesium phosphide + TX, MB-599 + TX, mefenpyr (including mefenpyr-diethyl) + TX, metcamifen + TX, methiocarb + TX, methyl bromide + TX, nerolidol + TX, norbormide + TX, petroleum oils + TX, phosacetim + TX, phosphine + TX, phosphorus + TX, pindone + TX, piperonyl butoxide + TX, piprotal + TX, potassium arsenite + TX, probenazole + TX, propyl isomer + TX, pyridin-4-amine + TX, pyrinuron + TX, Reynoutria sachalinensis extract + TX, ribavirin + TX, S421 + TX, scilliroside + TX, sesamex + TX, sesasmolin + TX, sodium arsenite + TX, sodium cyanide + TX, sodium fluoroacetate + TX, strychnine + TX, sulfoxide + TX, thallium sulfate + TX, thiram + TX, trimethacarb + TX, warfarin + TX, zinc naphthenate + TX, zinc phosphide + TX, ziram + TX.

The references in brackets behind the active ingredients, e.g. [3878-19-1] refer to the Chemical Abstracts Registry number. The above-described mixing partners are known. Where the active ingredients are included in "The Pesticide Manual" [The Pesticide Manual - A World Compendium, Thirteenth Edition, Editor: C. D. S. Tomlin, The British Crop Protection Council], they are described therein under the entry number given in round brackets hereinabove for the particular compound, for example, the compound "abamectin" is described under entry number (1). Where "[CCN]" is added hereinabove to the particular compound, the compound in question is included in the "Compendium of Pesticide Common Names", which is accessible on the internet [A. Wood, Compendium of Pesticide Common Names, Copyright © 1995-2004], for example, the compound "acetoprole" is described under the internet address http://www.alanwood.net/pesticides/acetoprole.html.

Most of the active ingredients described above are referred to hereinabove by a so-called "common name", the relevant "ISO common name" or another "common name" being used in individual cases. If the designation is not a "common name", the nature of the designation used instead is given in round brackets for the particular compound, in that case, the IUPAC name, the IUPAC/Chemical Abstracts name, a "chemical name", a "traditional name", a "compound name" or a "development code" is used. "CAS Reg. No" means the Chemical Abstracts Registry Number.

The ratio (by weight) of active ingredient mixture of the compounds of formula (I) selected from a compound A1 to A41 listed in Table A (below) or a specific compound listed in Tables 1 to 13 with active ingredients described above is from 100:1 to 1:6000, especially from 50:1 to 1:50, more especially in a ratio of from 20:1 to 1:20, even more especially from 10:1 to 1:10, very especially from 5:1 and 1:5, special preference being given to a ratio of from 2:1 to 1:2, and a ratio of from 4:1 to 2:1 being likewise preferred, above all in a ratio of 1:1, or 5:1, or 5:2, or 5:3, or 5:4, or 4:1, or 4:2, or 4:3, or 3:1, or 3:2, or 2:1, or 1:5, or 2:5, or 3:5, or 4:5, or 1:4, or 2:4, or 3:4, or 1:3, or 2:3, or 1:2, or 1:600, or 1:300, or 1:150, or 1:35, or 2:35, or 4:35, or 1:75, or 2:75, or 4:75, or 1:6000, or 1:3000, or 1:1500, or 1:350, or 2:350, or 4:350, or 1 :750, or 2:750, or 4:750.

The mixtures as described above can be used in a method for controlling pests, which comprises applying a composition comprising a mixture as described above to the pests or their environment, with the exception of a method for treatment of the human or animal body by surgery or therapy and diagnostic methods practiced on the human or animal body.

The mixtures comprising a compound of formula (I) selected from a compound A1 to A41 listed in Table A (below) or a specific compound listed in Tables 1 to 13 (below) and one or more active ingredients as described above can be applied, for example, in a single "ready-mix" form, in a combined spray mixture composed from separate formulations of the single active ingredient components, such as a "tank-mix", and in a combined use of the single active ingredients when applied in a sequential manner, i.e. one after the other with a reasonably short period, such as a few hours or days. The order of applying the compounds of formula (I) selected from a compound A1 to A41 listed in Table A (below) or a specific compound listed in Tables 1 to 13 (below) and the active ingredients as described above is not essential for working the present invention.

In a further aspect, the present invention provides a combination of active ingredients comprising a compound defined in the first aspect, and one or more further active ingredients (whether chemical or biological).

The compositions according to the invention can also comprise further solid or liquid auxiliaries, such as stabilizers, for example unepoxidized or epoxidized vegetable oils (for example epoxidized coconut oil, rapeseed oil or soya oil), antifoams, for example silicone oil, preservatives, viscosity regulators, binders and/or tackifiers, fertilizers or other active ingredients for achieving specific effects, for example bactericides, fungicides, nematocides, plant activators, molluscicides or herbicides.

The compositions according to the invention are prepared in a manner known per se, in the absence of auxiliaries for example by grinding, screening and/or compressing a solid active ingredient and in the presence of at least one auxiliary for example by intimately mixing and/or grinding the active ingredient with the auxiliary (auxiliaries). These processes for the preparation of the compositions and the use of the compounds (I) for the preparation of these compositions are also a subject of the invention.

The application methods for the compositions, that is the methods of controlling pests of the abovementioned type, such as spraying, atomizing, dusting, brushing on, dressing, scattering or pouring - which are to be selected to suit the intended aims of the prevailing circumstances - and the use of the compositions for controlling pests of the abovementioned type are other subjects of the invention. Typical rates of concentration are between 0.1 and 1000 ppm, preferably between 0.1 and 500 ppm, of active ingredient. The rate of application per hectare is generally 1 to 2000 g of active ingredient per hectare, in particular 10 to 1000 g/ha, preferably 10 to 600 g/ha.

A preferred method of application in the field of crop protection is application to the foliage of the plants (foliar application), it being possible to select frequency and rate of application to match the danger of infestation with the pest in question. Alternatively, the active ingredient can reach the plants via the root system (systemic action), by drenching the locus of the plants with a liquid composition or by incorporating the active ingredient in solid form into the locus of the plants, for example into the soil, for example in the form of granules (soil application). In the case of paddy rice crops, such granules can be metered into the flooded paddy-field.

The compounds of the invention and compositions thereof are also suitable for the protection of plant propagation material, for example seeds, such as fruit, tubers or kernels, or nursery plants, against pests of the abovementioned type. The propagation material can be treated with the compound prior to planting, for example seed can be treated prior to sowing. Alternatively, the compound can be applied to seed kernels (coating), either by soaking the kernels in a liquid composition or by applying a layer of a solid composition. It is also possible to apply the compositions when the propagation material is planted to the site of application, for example into the seed furrow during drilling. These treatment methods for plant propagation material and the plant propagation material thus treated are further subjects of the invention. Typical treatment rates would depend on the plant and pest/fungi to be controlled and are generally between 1 to 200 grams per 100 kg of seeds, preferably between 5 to 150 grams per 100 kg of seeds, such as between 10 to 100 grams per 100 kg of seeds.

The term seed embraces seeds and plant propagules of all kinds including but not limited to true seeds, seed pieces, suckers, corns, bulbs, fruit, tubers, grains, rhizomes, cuttings, cut shoots and the like and means in a preferred embodiment true seeds.

The present invention also comprises seeds coated or treated with or containing a compound of formula (I). The term "coated or treated with and/or containing" generally signifies that the active ingredient is for the most part on the surface of the seed at the time of application, although a greater or lesser part of the ingredient may penetrate into the seed material, depending on the method of application. When the said seed product is (re)planted, it may absorb the active ingredient. In an embodiment, the present invention makes available a plant propagation material adhered thereto with a compound of formula (I). Further, it is hereby made available, a composition comprising a plant propagation material treated with a compound of formula (I).

Seed treatment comprises all suitable seed treatment techniques known in the art, such as seed dressing, seed coating, seed dusting, seed soaking and seed pelleting. The seed treatment application of the compound formula (I) can be carried out by any known methods, such as spraying or by dusting the seeds before sowing or during the sowing/planting of the seeds.

A further aspect is a plant propagation material comprising by way of treatment or coating one or more compounds of formula (I) according to the invention, optionally also comprising a colour pigment.

In each aspect and embodiment of the invention, "consisting essentially" and inflections thereof are a preferred embodiment of "comprising" and its inflections, and "consisting of" and inflections thereof are a preferred embodiment of "consisting essentially of" and its inflections.

The disclosure in the present application makes available each and every combination of embodiments disclosed herein.
Table 1 provides 19 specific compounds of formula (I), (compounds 1.01 to 1.19) 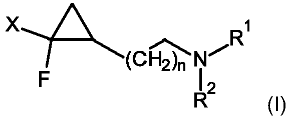 Wherein X is F, n is 3, and NR¹R² is as defined in the below Table Z1.

**Table Z1**

| **Compound** | **NR¹R²** | **Compound** | **NR¹R²** |
|---|---|---|---|
| 1.01 | amino | 1.11 | 2-pyridylamino |
| 1.02 | hydroxylamino | 1.12 | (5-chloro-2-pyridyl)amino |
| 1.03 | 2-benzylidenehydrazino | 1.13 | 2-chloro-3-fluoro-anilino |
| 1.04 | -N(CH₃)₂ | 1.14 | (6-chloro-3-pyridyl)amino |
| 1.05 | cyclobutylamino | 1.15 | pyrimidin-2-ylamino |
| 1.06 | cyclopentylamino | 1.16 | 2-(5-chloro-2-pyridyl)ethylamino |
| 1.07 | ethylamino | 1.17 | benzylamino |
| 1.08 | hydrazino | 1.18 | (5-chloro-2-pyridyl)methylamino |
| 1.09 | ethoxyamino | 1.19 | prop-2-ynylamino |
| 1.10 | 2,2-difluoroethylamino | | |

Table 2 provides 19 specific compounds of formula (I) (compounds 2.01 to 2.19), wherein X is F, n is 5, and NR¹R² is as defined in the below Table Z1.
Table 3 provides 19 specific compounds of formula (I) (compounds 3.01 to 3.19), wherein X is Cl, n is 3, and NR¹R² is as defined in the below Table Z1.
Table 4 provides 19 specific compounds of formula (I) (compounds 4.01 to 4.19), wherein X is Cl, n is 5, and NR¹R² is as defined in the below Table Z1.
Table 5 provides 16 specific compounds of formula (Id), (compounds 5.01 to 5.16), 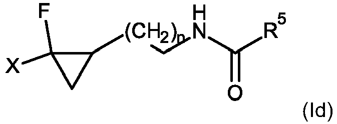 wherein X is F, n is 3, and R⁵ is as defined in the below Table Z2.

**Table Z2**

| **Compound** | **R⁵** | **Compound** | **R⁵** |
|---|---|---|---|
| 5.01 | phenyl | 5.09 | 2,6-dichlorophenyl |
| 5.02 | p-tolyl | 5.10 | 5-methyl-2-pyridyl |
| 5.03 | 4-methoxyphenyl | 5.11 | 1-piperidyl |
| 5.04 | 4-fluorophenyl | 5.12 | isopropyl |
| 5.05 | 4-(trifluoromethyl)phenyl | 5.13 | ethyl |
| 5.06 | 2,6-dimethylphenyl | 5.14 | pyrrolidin-1-yl |
| 5.07 | 2-fluorophenyl | 5.15 | 2,2-difluoropyrrolidin-1-yl |
| 5.08 | 3-fluorophenyl | 5.16 | (2-ethylpyrrolidin-1-yl) |

Table 6 provides 16 specific compounds of formula (Id) (compounds 6.01 to 6.16), wherein X is F, n is 5, and R⁵ is as defined in the below Table Z2.
Table 7 provides 16 specific compounds of formula (Id) (compounds 7.01 to 7.16), wherein X is Cl, n is 3, and R⁵ is as defined in the below Table Z2.
Table 8 provides 16 specific compounds of formula (Id) (compounds 8.01 to 8.16), wherein X is Cl, n is 5, and R⁵ is as defined in the below Table Z2.
Table 9 provides 11 specific compounds of formula (Ig) (compounds 9.01 to 9.11) 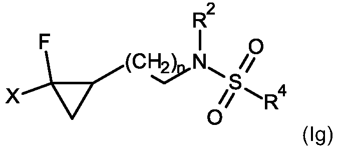 wherein X is F, n is 3; and R² and R⁴ are as defined below in Table Z3.

**Table Z3**

| **Compound** | **R²** | **R⁴** |
|---|---|---|
| 9.01 | hydrogen | ethyl |
| 9.02 | hydrogen | phenyl |
| 9.03 | hydrogen | 2-fluoro-3-pyridyl |
| 9.04 | hydrogen | 2-methoxy-3-pyridyl |
| 9.05 | hydrogen | 2-chloro-3-pyridyl |
| 9.06 | hydrogen | (5-chloro-2-pyridyl)-methyl |
| 9.07 | cyanomethyl | (5-chloro-2-pyridyl)methyl |
| 9.08 | methyl | (5-chloro-2-pyridyl)methyl |
| 9.09 | hydrogen | methylamino |
| 9.10 | hydrogen | pyrrolidin-1-yl |
| 9.11 | hydrogen | 1-piperidyl |

Table 10 provides 11 specific compounds of formula (Ig) (compounds 10.01 to 10.11), wherein X is F, n is 5, and R² and R⁴ is as defined in the below Table Z3.
Table 11 provides 11 specific compounds of formula (Ig) (compounds 11.01 to 11.11), wherein X is Cl, n is 3, and R² and R⁴ is as defined in the below Table Z3.
Table 12 provides 11 specific compounds of formula (Ig) (compounds 12.01 to 12.11), wherein X is Cl, n is 5, and R² and R⁴ is as defined in the below Table Z3.

**Table 13 provides 8 specific compounds (compounds 13.01 to 13.08) according to Formula (I) as follows:**

| **Compound** | **Structure** |
|---|---|
| 13.01 | 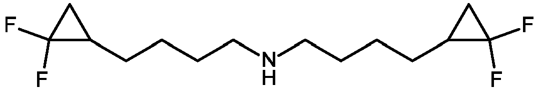 |
| 13.02 | 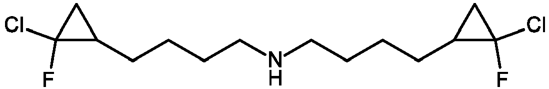 |
| 13.03 | 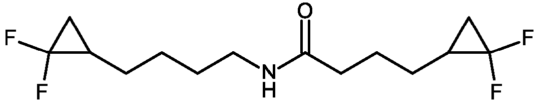 |
| 13.04 | 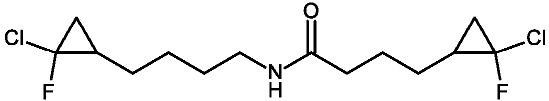 |
| 13.05 | 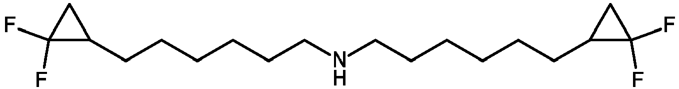 |
| 13.06 | 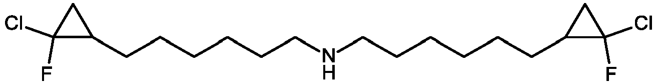 |
| 13.07 | 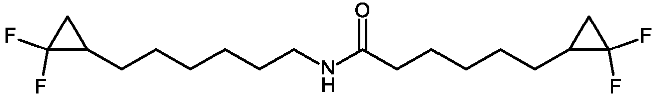 |
| 13.08 | 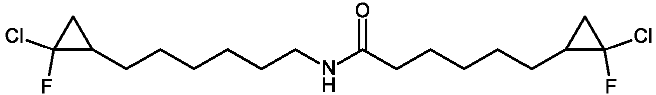 |

### EXAMPLES

The Examples which follow serve to illustrate the invention.

The compounds of the invention can be distinguished from known compounds by virtue of greater efficacy at low application rates, which can be verified by the person skilled in the art using the experimental procedures outlined in the Examples, using lower application rates if necessary, for example 50 ppm, 12.5 ppm, 6 ppm, 3 ppm, 1.5 ppm, 0.8 ppm or 0.2 ppm, or lower application rates, such as 300, 200 or 100 mg of Al per m².

Compounds of Formula (I) may possess any number of benefits including, *inter alia,* advantageous levels of biological activity for protecting plants against insects or superior properties for use as agrochemical active ingredients (for example, greater biological activity, an advantageous spectrum of activity, an increased safety profile (including improved crop tolerance, or environmental safety), improved physico-chemical properties, or increased biodegradability).

Throughout this description, temperatures are given in degrees Celsius (°C) and "mp" means melting point.

LC/MS means Liquid Chromatography Mass Spectrometry and the description of the apparatus and the methods A and B is outlined below. The characteristic LC/MS values obtained for each compound were the retention time ("Rt", recorded in minutes (min)) and the measured molecular ion (M+H)⁺ and/or (M-H)⁻.

¹ H NMR measurements were recorded on Brucker 400 MHz or 300 MHz spectrometers, chemical shifts are given in ppm relevant to a tetramethylsilane (TMS) standard. Spectra are measured in deuterated solvents (eg, dimethyl sulfoxide (DMSO-*d₆*)) as indicated.

### LCMS Methods:

### Method A (HSS): HSS QC method

Spectra were recorded on a ACQUITY Mass Spectrometer from Waters Corporations (SQD or SQDII Single quadrupole mass spectrometer) equipped with an electrospray source (Polarity: positive or negative ions, Capillary: 3.0 kV, Cone: 30V, Extractor: 3.00 V, Source Temperature: 150°C, Desolvation Temperature: 400°C, Cone Gas Flow: 60 L/hr, Desolvation Gas Flow: 700 L/hr, Mass range: 140 to 800 Da) and an ACQUITY UPLC from Waters Corporations with solvent degasser, binary pump, heated column compartment and diode-array detector. Column: Waters UPLC HSS T3, 1.8 µm, 30 × 2.1 mm, Temp: 60 °C, DAD Wavelength range (nm): 210 to 400, Solvent Gradient: A = Water/Methanol 9:1 + 0.1% formic acid, B= Acetonitrile + 0.1% formic acid, gradient: 0-100% B in 2.5 min; Flow (ml/min) 0.75.

### Example 1: Preparation of 6-(2,2-difluorocyclopropyl)hexan-1-amine hydrochloride

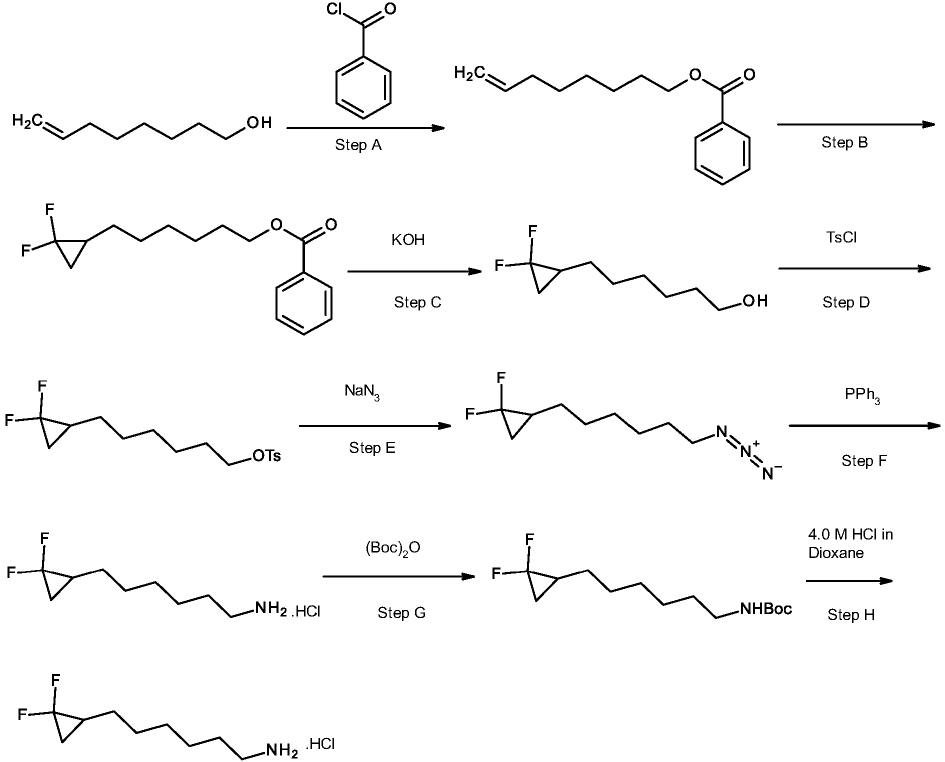

### Step A : Preparation of oct-7-en-1-yl benzoate

A flame-dried flask was charged with oct-7-en-1-ol (5.00 g, 39.9 mmol) and 2-methyl tetrahydrofuran (50 mL) and cooled to 0 °C. Triethylamine (6.76 mL, 46.8 mmol) was added dropwise and the resultant mixture was stirred for 5 min. Benzoyl chloride (4.53 mL, 39.0 mmol) in 2-methyl tetrahydrofuran (50 mL) was added, and the mixture was warmed to room temperature and stirred for 4 h. The reaction progress was monitored by TLC (20% Ethyl acetate/hexanes) and it showed no starting material. The reaction mixture was quenched with water (150 mL) and then extracted with methyl tert-butyl ether (2 × 200 mL). The combined organic layers were washed with brine (300 mL), dried over sodium sulfate and concentrated under reduced pressure. The crude compound was purified by Combi Flash column chromatography (Combi Flash, 0 to 100% Hexanes/Ethyl acetate). Pure fractions were combined and evaporated under reduced pressure to afford oct-7-en-1-yl benzoate (7.50 g, 32.3 mmol, yield: 82.8%) as a colorless liquid.

¹H NMR (400 MHz, CDCl₃): δ 8.05 - 8.03 (m, 2H), 7.55 - 7.53 (m, 1H), 7.45 - 7.42 (m, 2H), 5.86 - 5.76 (m, 1H), 5.02 - 4.92 (m, 2H), 4.31 (t, 2H), 2.07 - 2.05 (m, 2H), 1.79 - 1.75 (m, 2H), 1.54 - 1.39 (m, 6H).

### Step B : Preparation of 6-(2,2-difluorocyclopropyl)hexyl benzoate

A three-neck flask equipped with argon balloon was charged with oct-7-enyl benzoate (21.0 g, 90.4 mmol) and sodium fluoride (0.380 g, 9.04 mmol). The resulting reaction mixture was heated to 120 °C. At this temperature trimethylsilyl 2, 2-difluoro-2-fluorosulfonyl-acetate (49.8 g, 199 mmol) was added dropwise by syringe in two portions. The resulting reaction mixture was stirred at 115-120 °C for 16 h. The reaction progress was monitored by TLC (10% ethyl acetate/hexanes). The reaction mixture was quenched with ice (300 mL) and then extracted with ethyl acetate (2 × 200 mL). The combined organic layers were washed with brine (400 mL), dried over sodium sulfate and concentrated under reduced pressure. The crude compound was purified by Combi Flash column chromatography (Combi Flash, 0 to 100% Hexanes/Ethyl acetate). Pure fractions were combined and evaporated under reduced pressure to afford 6-(2,2-difluorocyclopropyl)hexyl benzoate (8.70 g, 30.8 mmol, yield: 34.1%) as a colorless liquid.

¹H NMR (400 MHz, DMSO-*d*₆): δ 7.97 - 7.95 (m, 2H), 7.66 - 7.64 (m, 1H), 7.55 - 7.51 (m, 2H), 4.27 (t, 2H), 1.73 - 1.38 (m, 12H), 1.05 - 1.01 (m, 1H).

¹⁹F NMR (376 MHz, DMSO-*d*₆): δ -126.38 (d, 0.91F), -142.65 (d, 1F).

### Step C : Preparation of 6-(2,2-difluorocyclopropyl)hexan-1-ol

A three-neck flask equipped with argon balloon was charged with 6-(2,2-difluorocyclopropyl)hexyl benzoate (10.4 g, 36.8 mmol) in dichloromethane (96 mL) and methanol (24 mL) at 0 °C was added potassium hydroxide (2.07 g, 36.8 mmol). The resulting reaction mixture was stirred at room temperature for 16 h. The reaction progress was monitored by TLC (30% ethyl acetate/hexanes) and it showed no starting material. The reaction mixture was diluted with water (300 mL) and then extracted with dichloromethane (2 × 300 mL). The combined organic layers were washed with brine (500 mL), dried over sodium sulfate and concentrated under reduced pressure. The crude residue was purified by combi-flash column chromatography using (0 to 100%) ethyl acetate in hexanes. Pure fractions were combined and evaporated under reduced pressure to afford 6-(2, 2-difluorocyclopropyl)hexan-1-ol (4.50 g, 25.2 mmol, yield: 68.5%) as a colour less liquid.

¹H NMR (400 MHz, DMSO-*d*₆): δ 4.33 (t, *J* = 4.8 Hz, 1H), 3.39 - 3.33 (m, 2H), 1.65 - 1.28 (m, 12H), 1.05 - 1.01 (m, 1H).

¹⁹F NMR (376 MHz, DMSO-*d*₆): δ -126.36 (d, 1F), -142.67 (d, 0.99F).

### Step D : Preparation of 6-(2,2-difluorocyclopropyl)hexvl 4-methylbenzenesulfonate :

A three-neck flask equipped with argon balloon and charged with 6-(2,2-difluorocyclopropyl)hexan-1-ol (4.50 g, 25.2 mmol) in dichloromethane (80 mL) at 0 °C were added *N,N-*diethylethanamine (7.67 g, 75.7 mmol) and 4-toluenesulfonyl chloride (7.22 g, 37.9 mmol). The resulting reaction mixture was stirred at room temperature for 16 h. The reaction progress was monitored by TLC (30% Ethyl acetate/Hexanes) and it showed no starting material. The reaction mixture was quenched at 0 °C with saturated sodium bicarbonate solution (100 mL) and then extracted with dichloromethane (100 mL). The combined organic layers were washed with water (100 mL) and brine (100 mL) dried over sodium sulfate and concentrated under reduced pressure. The crude residue was purified by combi-flash column chromatography using (0 to 100%) ethyl acetate in hexanes. Pure fractions were combined and evaporated under reduced pressure to afford 6-(2,2-difluorocyclopropyl)hexyl 4-methylbenzenesulfonate (5.50 g, 16.5 mmol, yield: 65.5%) as a colorless liquid.

¹H NMR (400 MHz, DMSO-*d*₆): δ 7.79 - 7.77 (m, 2H), 7.48 (d, 2H), 4.00 (t, 2H), 2.42 (s, 3H), 1.57 - 1.18 (m, 12H),1.04 - 0.99 (m, 1H).

¹⁹F NMR (376 MHz, DMSO-*d*₆): δ -126.38 (d, 1F), -142.68 (d, 0.99F).

### Step E : Preparation of 2-(6-azidohexyl)-1,1-difluorocyclopropane :

A three-neck flask equipped with argon balloon and charged 6-(2,2-difluorocyclopropyl)hexyl 4-methylbenzenesulfonate (8.00 g, 24.1 mmol) in dimethylformamide (80 mL) was added sodium azide (3.13 g, 48.1 mmol) at 0 °C and stirred at room temperature for 16 h. The reaction progress was monitored by TLC (30% Ethyl acetate : Hexanes) and it showed no starting material. The reaction mixture was diluted with water (100 mL) and extracted with methyl tert-butyl ether (2 × 100 mL). The combined organic layers were washed with brine (100 mL), dried over anhydrous sodium sulfate and evaporated under reduced pressure, with a bath temperature of 25 °C, to afford 2-(6-azidohexyl)-1,1-difluoro-cyclopropane (6.50 g, 19.2 mmol, crude) as a colorless liquid. This crude product was directly used for the next step without purification.

### Step F : Preparation of 6-(2,2-difluorocyclopropyl)hexan-1-amine hydrochloride :

A three-neck flask equipped with argon balloon and charged with 2-(6-azidohexyl)-1,1-difluorocyclopropane (6.50 g, 19.2 mmol) in ethyl acetate (60.0 mL) was added triphenylphosphine (10.1 g, 38.4 mmol) at room temperature portion wise and stirred for 12 h. Then the reaction mixture was cooled to 0 °C and then hydrochloric acid (4 M) in 1,4-dioxane (4.00 mol/L, 12.0 mL, 48.0 mmol) was added dropwise. The resulting reaction mixture was stirred at room temperature for 5 h. The reaction progress was monitored by TLC (50% Ethyl acetate/Hexanes) and it showed no starting material. The solvent was evaporated and the crude was triturated with methyl tert-butyl ether (25 mL). The resulting residue was washed with methyl tert-butyl ether to afford a crude 6-(2,2-difluorocyclopropyl)hexan-1-amine;hydrochloride (8.00 g, 16.8 mmol, crude) as a colorless gum. This crude product was directly used for the next step without purification.

### Step G : Preparation of tert-butyl (6-(2,2-difluorocyclopropyl)hexyl)carbamate:

A three-neck flask equipped with argon balloon and charged with 6-(2,2-difluorocyclopropyl)hexan-1-amine;hydrochloride (8.00 g, 16.8 mmol, crude) in dichloromethane (100 mL) at 0 °C were added *N,N*-diethylethanamine (5.11 g, 50.5 mmol) followed by di-tert-butyl dicarbonate (3.68 g, 16.8 mmol) dropwise. The resulting reaction mixture was stirred at room temperature for 16 h. The reaction progress was monitored by TLC (50% Ethyl acetate/Hexanes) and it showed no starting material. The solvent was removed under vacuum. The resulting crude residue was purified by combi flash column chromatography using (0 to 100%) ethyl acetate in hexanes. Pure fractions were combined and evaporated under reduced pressure to afford *tert*-butyl N-[6-(2,2-difluorocyclopropyl)hexyl] carbamate (4.50 g, 16.2 mmol, yield: 96.3%) as a colorless liquid.

¹H NMR (400 MHz, DMSO-*d*₆): δ 6.87 - 6.76 (m, 1H), 2.89 - 2.86 (m, 2H), 1.61 - 1.23 (m, 21H), 1.05 - 1.00 (m, 1H).

### Step H : Preparation of 6-(2,2-difluorocyclopropyl)hexan-1-amine hydrochloride :

A three-neck flask equipped with argon balloon and charged with *tert*-butyl N-[6-(2,2-difluorocyclopropyl)hexyl]carbamate (4.50 g, 16.2 mmol) in dry dichloromethane (50 mL) at 0 °C was added hydrochloric acid (4 M) in 1,4-dioxane (16.2 mL, 64.9 mmol) dropwise. The resulting reaction mixture was stirred at room temperature for 16 h. The reaction progress was monitored by TLC (50% Ethyl acetate /Hexanes) and it showed no starting material. The solvent was evaporated and the crude was triturated with methyl tert-butyl ether (2 × 25 mL) to afford 6-(2,2-difluorocyclopropyl)hexan-1-amine;hydrochloride (2.50 g, 11.7 mmol, yield: 72.1 %) as an off white solid.

¹H NMR (400 MHz, DMSO-*d*₆): δ 8.06 (brs, 3H), 2.76 - 2.70 (m, 2H), 1.66 - 1.30 (m, 12H), 1.06 - 1.02 (m, 1H).

¹⁹F NMR (376 MHz, DMSO-*d*₆): δ -126.35 (d, 1F), -142.58 (d, 0.98F).

### Example 2: Preparation of N-(6-(2,2-difluorocyclopropyl)hexyl)ethane sulfonamide

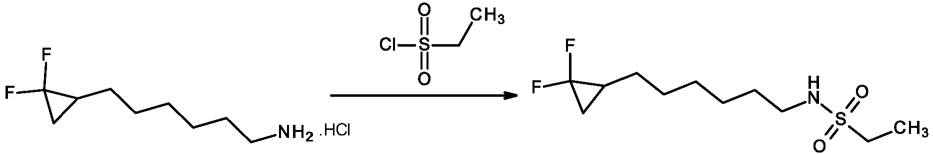

A flask equipped with argon balloon and charged with 6-(2,2-difluorocyclopropyl)hexan-1-amine;hydrochloride (100 mg, 0.468 mmol) in dichloromethane (3.0 mL) at 0 °C were added triethylamine (142 mg, 1.40 mmol) followed by ethane sulfonyl chloride (66.2 mg, 0.515 mmol) in dichloromethane (2.00 mL) drop wise. The resulting reaction mixture was stirred at room temperature for 16 h. The reaction progress was monitored by TLC (30% Ethyl acetate /Hexanes) and it showed no starting material. The reaction mixture was quenched with saturated sodium bicarbonate solution and extracted with dichloromethane (2 × 10 mL). The combined organic layers were washed with brine (10 mL), dried over sodium sulfate and concentrated under reduced pressure to afford crude reaction mixture. The resulting crude residue was purified by combi-flash column chromatography using (0 to 100%) ethyl acetate in hexanes. Pure fractions were combined and evaporated under reduced pressure to afford N-[6-(2,2-difluorocyclopropyl)hexyl]ethane sulfonamide (60.0 mg, 0.223 mmol, yield: 47.6%) as a light-yellow liquid.

¹H NMR (400 MHz, DMSO-*d*₆): δ 6.99 - 6.96(m, 1H), 2.97 - 2.93 (m, 2H), 2.90 - 2.85 (m, 2H), 1.46 - 1.28 (m, 12H), 1.17 (t, 3H),1.08 - 1.00 (m, 1H).

¹⁹F NMR (376 MHz, DMSO-*d*₆): δ -126.34 (d, 1F), -142.62 (d, 0.91F).

### Example 3: Preparation of N-(6-(2,2-difluorocyclopropyl)hexvl)benzamide:

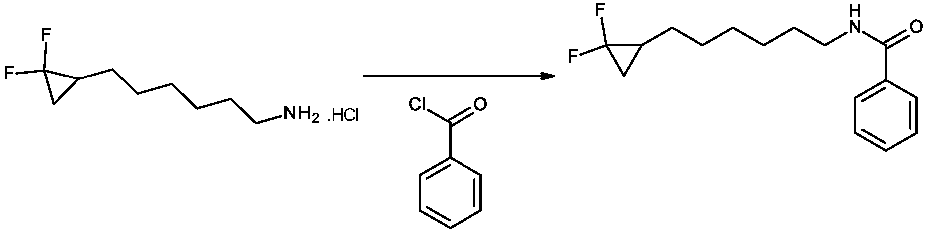

A flask equipped with argon balloon and charged with 6-(2,2-difluorocyclopropyl)hexan-1-amine;hydrochloride (100 mg, 0.468 mmol) in dichloromethane (3.0 mL) at 0 °C were added triethylamine (142 mg, 1.40 mmol) followed by benzoyl chloride (72.4 mg, 0.515 mmol) in dichloromethane (2.00 mL) drop wise. The resulting reaction mixture was stirred at room temperature for 16h. The reaction progress was monitored by TLC (50% Ethyl acetate/Hexanes) and it showed no starting material. The reaction mixture was quenched with saturated sodium bicarbonate solution and extracted with dichloromethane (2 × 10 mL). The combined organic layers were washed with brine (10 mL), dried over sodium sulfate and concentrated under reduced pressure to afford crude reaction mixture. The resulting crude residue was purified by combi flash column chromatography using (0 to 100%) ethyl acetate in hexanes. Pure fractions were combined and evaporated under reduced pressure to afford N-[6-(2,2-difluorocyclopropyl)hexyl]benzamide (65.0 mg, 0.231 mmol, yield: 49.4%) as an off-white solid.

¹H NMR (400 MHz, DMSO-*d*₆): δ 8.48 - 8.44(m, 1H), 7.84 - 7.62 (m, 2H), 7.51 - 7.42 (m, 3H), 3.34 - 3,22 (m, 2H), 1.60 - 1.31 (m, 12H),1.04 - 1.01 (m, 1H).

¹⁹F NMR (376 MHz, DMSO-*d*₆): δ -126.35 (d, 1F), -146.63 (d, 0.98).

### Example 4: Preparation of N-(6-(2,2-difluorocyclopropyl)hexvl)cyclobutene sulfonamide

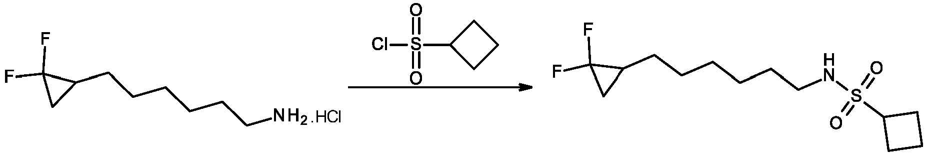

A flask equipped with argon balloon and charged with 6-(2,2-difluorocyclopropyl)hexan-1-amine;hydrochloride (100 mg, 0.468 mmol) in Dichloromethane (3.0 mL) at 0 °C were added Triethylamine (142 mg, 1.40 mmol) followed by cyclobutanesulfonyl chloride (79.6 mg, 0.515 mmol) in dichloromethane (2.00 mL) drop wise. The resulting reaction mixture was stirred at room temperature for 16 h. The reaction progress was monitored by TLC (50% Ethyl acetate /Hexanes) and it showed no starting material. The reaction mixture was quenched with saturated sodium bicarbonate solution and extracted with dichloromethane (2 × 10 mL). The combined organic layers were washed with brine (10 mL), dried over sodium sulfate and concentrated under reduced pressure to afford crude reaction mixture. The resulting crude residue was purified by combi-flash column chromatography using (0 to 100%) ethyl acetate in hexanes. Pure fractions were combined and evaporated under reduced pressure to afford N-[6-(2,2-difluorocyclopropyl)hexyl]cyclobutene sulfonamide (65.0 mg, 0.220 mmol, yield: 47.0% as a light-yellow gum.

¹H NMR (400 MHz, DMSO-*d*₆): δ 6.99 - 6.91 (m, 1H), 3.88 - 3.79 (m, 1H), 2.91 - 2.87 (m, 2H), 2.31 - 2.12 (m, 4H), 1.96 - 1.81 (m, 2H), 1.69 - 1.21 (m, 12H), 1.08 - 1.00 (m, 1H).

¹⁹F NMR (376 MHz, DMSO-*d*₆): δ -126.37 (d, 1F), -142.66 (d, 0.91F).

### Example 5: Preparation of Synthesis of N-(6-(2,2-difluorocyclopropyl)hexyl)pyridin-2-amine

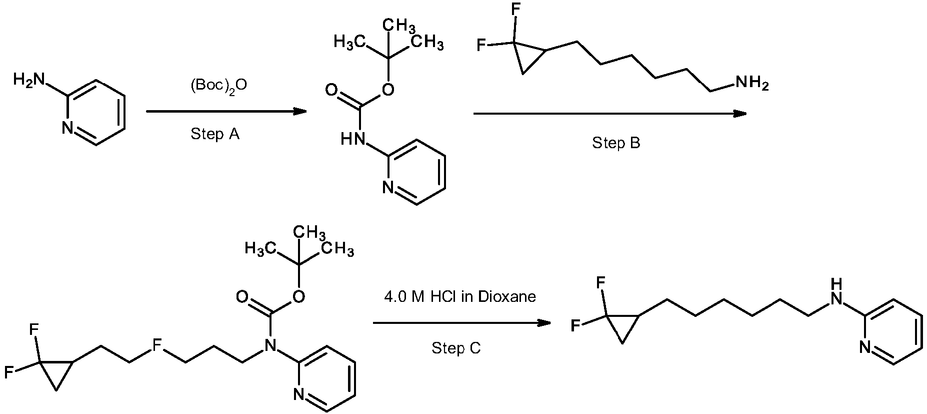

### Step A : Preparation of tert-butyl pvridin-2-vlcarbamate

A 30 mL sealed tube charged with pyridin-2-amine (200 mg, 2.13 mmol) and *tert*-butyl alcohol (4.00 mL) was added di-*tert*-butyl dicarbonate (464 mg, 2.13 mmol) at room temperature. The resulting reaction mixture was stirred under microwave irradiation at 100 °C for 30 min. The reaction progress was monitored by TLC (50% Ethyl acetate /Hexanes) and it showed no starting material. The reaction mixture was diluted with water (10 mL) and extracted with ethyl acetate (2 × 10 mL). The combined organic layers were washed with brine (10 mL), dried over sodium sulfate and concentrated under reduced pressure to afford crude reaction mixture. The solvent was removed under vacuum. The resulting crude residue was purified by combi flash column chromatography using (0 to 100%) ethyl acetate in hexanes. Pure fractions were combined and evaporated under reduced pressure to afford *tert*-butyl N-(2-pyridyl)carbamate (250 mg, 1.29 mmol, yield: 60.6%) as a colorless oil.

¹H NMR (400 MHz, DMSO-*d*₆): δ 9.73 (s, 1H), 8.24 - 8.22 (m, 1H), 7.79 - 7.69 (m, 2H), 7.02 - 6.99 (m, 1H), 1.47 (s, 9H).

### Step B : Preparation of tert-butyl (6-(2,2-difluorocyclopropyl)hexyl)(pyridin-2-yl)carbamate :

A 30 mL sealed tube charged with 6-(2,2-difluorocyclopropyl)hexyl 4-methylbenzenesulfonate (300 mg, 0.903 mmol), *tert*-butyl N-(2-pyridyl)carbamate (193 mg, 0.993 mmol) in dimethylformamide (5.0 mL) at room temperature was added cesium carbonate (441 mg, 1.35 mmol). The resulting reaction mixture was stirred under microwave irradiation at 110 °C for 30 min. The reaction progress was monitored by TLC (30% Ethyl acetate /Hexanes) and it showed no starting material. The reaction mixture was diluted with water (20 mL) and extracted with ethyl acetate (2 × 20 mL). The combined organic layers were washed with brine (30 mL), dried over sodium sulfate and concentrated under reduced pressure to afford crude reaction mixture. The resulting crude residue was purified by combi flash column chromatography using (0 to 100%) ethyl acetate in hexanes. Pure fractions were combined and evaporated under reduced pressure to afford *tert*-butyl N-[6-(2,2-difluorocyclopropyl)hexyl]-N-(2-pyridyl)carbamate (170 mg, 0.480 mmol, yield: 53.1%) as a colorless oil.

¹H NMR (400 MHz, DMSO-*d*₆): δ 8.37 - 8.36 (m, 1H), 7.75 - 7.53 (m, 1H), 7.53 - 7.51 (m, 1H), 7.14 - 7.12 (m, 1H), 3.84 - 3.80 (m, 2H), 1.62 - 1.23 (m, 21H), 1.08 - 1.00 (m, 1H).

### Step C : Preparation of N-(6-(2,2-difluorocyclopropyl)hexyl)pyridin-2-amine

A flask equipped with argon balloon and charged with *tert*-butyl N-[6-(2,2-difluorocyclopropyl)hexyl]-N-(2-pyridyl)carbamate (170 mg, 0.480 mmol) in dry dichloromethane (5.0 mL) at 0°C was added hydrochloric acid (4 M) in 1,4-dioxane (0.360 mL, 1.44 mmol) dropwise. The resulting reaction mixture was stirred at room temperature for 16 h. The reaction progress was monitored by TLC (50% Ethyl acetate/Hexanes) and it showed no starting material. The solvent was removed under vacuum. The resulting crude residue was purified by combi-flash column chromatography using (0 to 100%) ethyl acetate in hexanes. Pure fractions were combined and evaporated under reduced pressure to afford N-[6-(2,2-difluorocyclopropyl)hexyl]pyridin-2-amine (70.0 mg, 0.275 mmol, yield: 57.4%) as a colorless liquid.

¹H NMR (400 MHz, DMSO-*d*₆): δ 7.93 - 7.91 (m, 1H), 7.33 - 7.31 (m, 1H), 6.42 - 6.39 (m, 3H), 3.21 - 3.16 (m, 2H),1.52 - 1.32 (m, 12H), 1.05 - 1.00 (m, 1H).

¹⁹F NMR (376 MHz, DMSO-*d*₆): δ -126.33 (d, 1F), -142.63 (d, 0.94F).

### Example 6: Preparation of Synthesis of 4-(2,2-difluorocyclopropyl)butan-1-amine hydrochloride

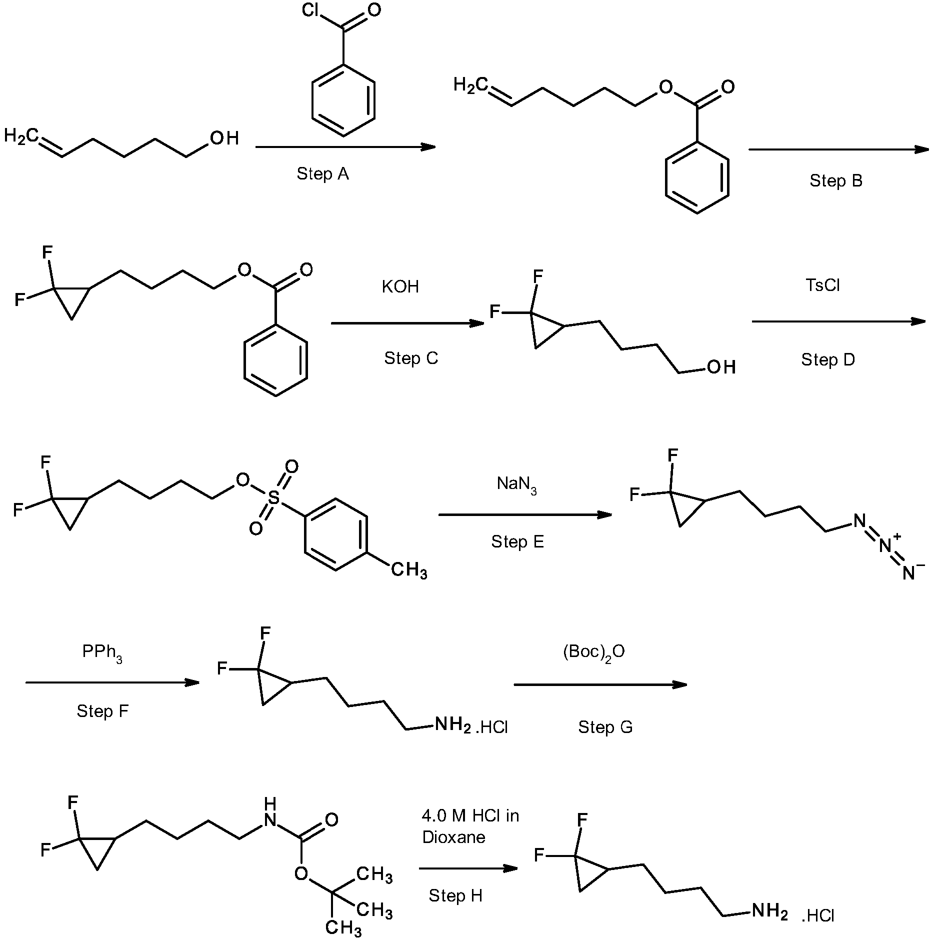

### Step A : Preparation of hex-5-en-1-vl benzoate :

A flask equipped with argon balloon was charged with hex-5-en-1-ol (25.0 g, 250 mmol) in 2-methyl tetrahydrofuran (250 mL) at 0°C were added triethylamine (41.7 mL, 300 mmol) followed by benzoyl chloride (29.0 mL, 250 mmol) in 2-methyl tetrahydrofuran (50 mL) drop wise. The resulting reaction mixture was stirred at room temperature for 4 h. The reaction progress was monitored by TLC (20% ethyl acetate/hexanes) and it showed no starting material. Reaction mixture was quenched with water (300 mL) and then extracted with methyl tert-butyl ether (2 × 300 mL). The combined organic layers were washed with brine (300 mL), dried over sodium sulfate and concentrated under reduced pressure. The crude compound was purified by Combi Flash column chromatography (Combi Flash, 0 to 100% Hexanes/Ethyl acetate). Pure fractions were combined and evaporated under reduced pressure to afford hex-5-enyl benzoate (37.0 g, 181.1 mmol, yield: 72.6%) as a colourless liquid.

¹H NMR (400 MHz, CDCl₃): 7.98 - 7.96 (m, 2H), 7.50 - 7.46 (m, 1H), 7.38 - 7.34 (m, 2H), 5.78 - 5.71 (m, 1H), 4.99- 4.89 (m, 2H), 4.25(t, 2H), 2.09 - 2.03 (m, 2H), 1.75 - 1.68 (m, 2H), 1.52 - 1.44 (m, 2H).

### Step B : Preparation of 4-(2,2-difluorocyclopropyl)butyl benzoate :

A flask equipped with argon balloon was charged with hex-5-enyl benzoate (5.00 g, 24.5 mmol) and sodium fluoride (103 mg, 2.45 mmol). The resulting reaction mixture was heated to 120 °C. At this temperature trimethylsilyl 2,2-difluoro-2-fluorosulfonyl-acetate (10.6 mL, 53.9 mmol) was added dropwise by syringe in two portions. The resulting reaction mixture was stirred at 115-120 °C for 16 h. The reaction progress was monitored by TLC (10% Ethyl acetate/hexanes). Reaction mixture was quenched with ice (300 mL) and then extracted with ethyl acetate (2 × 200 mL). The combined organic layers were washed with brine (400 mL), dried over sodium sulfate and concentrated under reduced pressure. The crude compound was purified by Combi Flash column chromatography (Combi Flash, 0 to 100% Hexanes/Ethyl acetate). Pure fractions were combined and evaporated under reduced pressure to afford 4-(2,2-difluorocyclopropyl)butyl benzoate (2.10 g, 33.7%) as a colorless liquid.

¹H NMR (400 MHz, DMSO-*d*₆): 7.98 - 7.95 (m, 2H), 7.66 - 7.64 (m, 1H), 7.55 - 7.51 (m, 2H), 4.28 (t, *J* = 6.4 Hz, 2H), 1.81 - 1.73 (m, 3H), 1.54 - 1.44 (m, 5H), 1.09 - 1.05 (m, 1H).

### Step C : Preparation of 4-(2,2-difluorocvclopropyl)butan-1-ol :

A flask equipped with argon balloon was charged with 4-(2,2-difluorocyclopropyl)butyl benzoate (6.10 g, 24.0 mmol) in dichloromethane (55 mL) and methanol (6 mL) at 0°C was added potassium hydroxide (1.48 g, 26.4 mmol). The resulting reaction mixture was stirred at room temperature for 16 h. The reaction progress was monitored by TLC (30% Ethyl acetate/hexanes) and it showed no starting material. Reaction mixture was diluted with water (200 mL) and then extracted with dichloromethane (2 × 200 mL). The combined organic layers were washed with brine (200 mL), dried over sodium sulfate and concentrated under reduced pressure to afford 4-(2,2-difluorocyclopropyl)butan-1-ol (5.50 g, 36.6 mmol, crude) as a colour less liquid. This crude product was directly used for the next step without purification.

### Step D : Preparation of 4-(2,2-difluorocyclopropyl)butyl 4-methylbenzenesulfonate :

A flask equipped with argon balloon and charged with 4-(2,2-difluorocyclopropyl)butan-1-ol (5.50 g, 36.6 mmol) in dichloromethane (50 mL) at 0 °C were added triethylamine (15.3 mL, 110 mmol) and toluene-4-sulfonyl chloride (10.5 g, 54.9 mmol). The resulting reaction mixture was stirred at room temperature for 16 h. The reaction progress was monitored by TLC (30% Ethyl acetate/Hexanes) and it showed no starting material. The reaction mixture was quenched with saturated sodium bicarbonate solution (50 mL) and extracted with dichloromethane (2 × 50 mL). The combined organic layers were washed with water (50 mL) and brine (50 mL), dried over sodium sulfate and concentrated under reduced pressure to afford crude reaction mixture. The crude residue was purified by combi-flash column chromatography using (0 to 100%) ethyl acetate in hexanes. Pure fractions were combined and evaporated under reduced pressure to afford 4-(2,2-difluorocyclopropyl)butyl 4-methylbenzenesulfonate (5.00 g, 16.4 mmol, yield: 44.9%) as a colorless liquid.

¹H NMR (400 MHz, CDCl₃): δ 7.72 (d, *J* = 8.0 Hz, 2H), 7.28 (d, *J* = 8.0 Hz, 2H), 3.96 (t, *J* = 6.4 Hz, 2H), 2.38 (s, 3H), 1.63 - 1.59 (m, 2H),1.49 - 1.27 (m, 6H), 0.82 - 0.78 (m, 1H).

¹⁹F NMR (376 MHz, DMSO-*d*₆): δ -128.22 (d,1F), -144.45 (d, 0.89F).

### Step E : Preparation of 2-(4-azidobutyl)-1,1-difluorocyclopropane :

A flask equipped with argon balloon and charged 4-(2,2-difluorocyclopropyl)butyl 4-methylbenzenesulfonate (5.00 g, 16.4 mmol) in dimethylformamide (50 ml) was added sodium azide (2.14 g, 32.9 mmol) at 0°C, and stirred at room temperature for 16 h. Reaction progress was monitored by TLC (30% Ethyl acetate : Hexanes) and it showed no starting material. The reaction mixture was diluted with water (200 mL) and extracted with methyl tert-butyl ether (2 × 200 mL). The combined organic layers were washed with brine (200 mL), dried over anhydrous sodium sulfate and evaporated under reduced pressure, with a bath temperature of 25 °C, to afford 2-(4-azidobutyl)-1,1-difluorocyclopropane (4.00 g, 22.8 mmol, crude) as a colorless liquid. This crude product was directly used for the next step without purification.

### Step F : Preparation of 4-(2,2-difluorocyclopropyl)butan-1-amine hydrochloride :

A flask equipped with argon balloon and charged with 2-(4-azidobutyl)-1,1-difluorocyclopropane (4.00 g, 22.8 mmol) in ethyl acetate (40 mL) was added triphenylphosphine (12.0 g, 45.7 mmol) at room temperature portion wise and stirred for 12 h. Then the reaction mixture was cooled to 0 °C and then hydrochloric acid (4 M) in 1,4-dioxane (40.0 mL) dropwise. The resulting reaction mixture was stirred at room temperature for 5 h. The reaction progress was monitored by TLC (50% Ethyl acetate /Hexanes) and it showed no starting material. The solvent was evaporated and the crude was triturated with 10% dichloromethane / methyl tert-butyl ether. The resulting residue was washed with methyl tert-butyl ether to afford 4-(2,2-difluorocyclopropyl)butan-1-amine;hydrochloride (0.80 g, 4.58 mmol, crude) as a colorless gum.

### Step G : Preparation of tert-butyl (4-(2,2-difluorocyclopropyl)butyl)carbamate :

A flask equipped with argon balloon and charged with 4-(2,2-difluorocyclopropyl)butan-1-amine;hydrochloride (3.70 g, 23.4 mmol, crude) in dichloromethane (50 mL) at 0 °C were added *N,N-*diethylethanamine (10.8 mL, 74.4 mmol) followed by Di-*tert*-butyl dicarbonate (5.95 g, 27.3 mmol) dropwise. The resulting reaction mixture was stirred at room temperature for 16 h. The reaction progress was monitored by TLC (60% Ethyl acetate/Hexanes) and it showed no starting material. The solvent was removed under vacuum. The resulting crude residue was purified by combi flash column chromatography using (0 to 100%) ethyl acetate in hexanes. Pure fractions were combined and evaporated under reduced pressure to afford *tert*-butyl N-[4-(2,2-difluorocyclopropyl)butyl]carbamate (1.80 g, 7.22 mmol, yield: 29.1%) as a colorless liquid.

¹H NMR (400 MHz, DMSO-*d*₆): δ 6.80 (t, *J =* 5.2 Hz,1H), 2.90 - 2.87 (m, 2H), 1.62 - 1.46 (m, 6H), 1.42 (s, 9H), 1.41 - 1.32 (m, 2H), 1.06 - 1.02 (m, 1H).

¹⁹F NMR (376 MHz, DMSO-*d*₆): δ -126.35 (d, 1F), -142.59 (d, 0.0.71F).

### Step H : Preparation of 4-(2,2-difluorocyclopropyl)butan-1-amine hydrochloride :

A flask equipped with argon balloon and charged with *tert*-butyl N-[4-(2,2-difluorocyclopropyl)butyl]carbamate (3.70 g, 14.8 mmol) in dry dichloromethane (40 mL) at 0 °C was added hydrochloric acid (4 M) in 1,4-dioxane (14.8 mL, 59.4 mmol) dropwise. The resulting reaction mixture was stirred at room temperature for 16 h. The reaction progress was monitored by TLC (20% Ethyl acetate /Hexanes) and it showed no starting material. The solvent was evaporated and the crude was triturated with methyl tert-butyl ether (2 × 25 mL) to afford 4-(2,2-difluorocyclopropyl)butan-1-amine;hydrochloride (1.80 g, 9.70 mmol, yield: 65%) as an off white solid.

¹H NMR (400 MHz, DMSO-*d*₆): δ 8.07 (brs, 3H), 2.78 - 2.72 (m, 2H),1.63 - 1.41 (m, 8H), 1.10 - 1.06 (m, 1H).

¹⁹F NMR (376 MHz, DMSO-*d*₆): δ -126.42 (d, 1F), -142.48 (d, 0.97F).

### Example 7: Preparation of Synthesis of 4-(2-chloro-2-fluorocyclopropyl)butan-1-amine hydrochloride

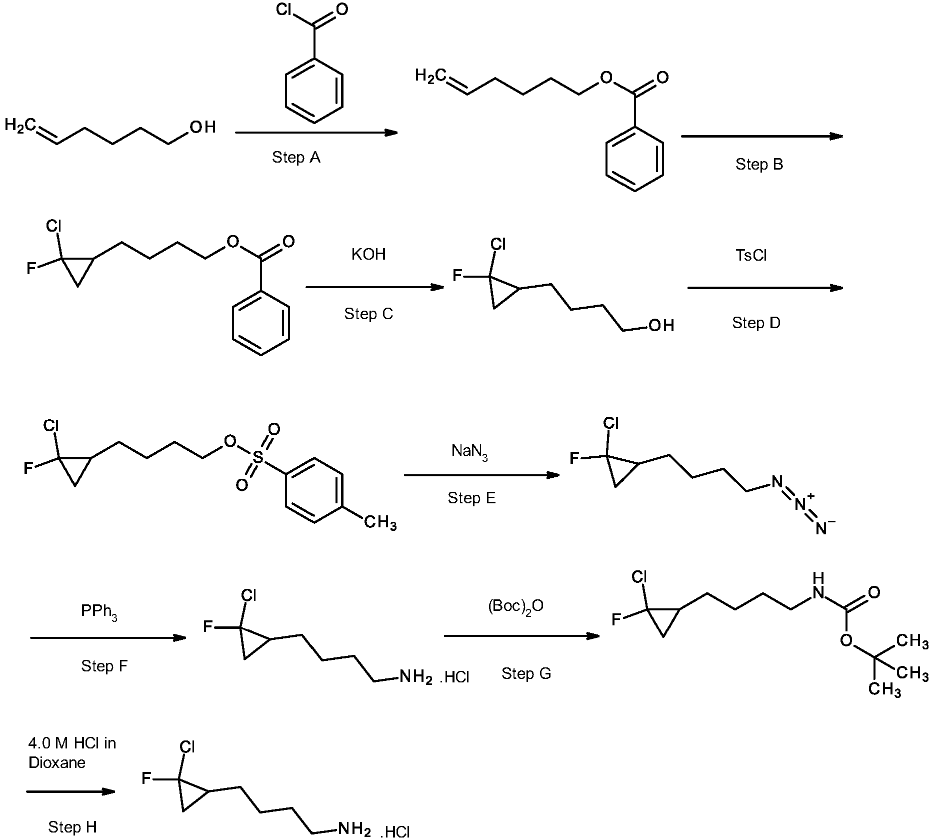

### Step A : Preparation of hex-5-en-1-vl benzoate :

A flask equipped with argon balloon was charged with hex-5-en-1-ol (25.0 g, 250 mmol) in 2-methyl tetrahydrofuran (250 mL) at 0 °C were added triethylamine (41.7 mL, 300 mmol) followed by benzoyl chloride (29.0 mL, 250 mmol) in 2-methyl tetrahydrofuran (50 mL) drop wise. The resulting reaction mixture was stirred at room temperature for 4 h. The reaction progress was monitored by TLC (20% ethyl acetate/hexanes) and it showed no starting material. Reaction mixture was quenched with water (300 mL) and then extracted with methyl *tert*-butyl ether (2 × 300 mL). The combined organic layers were washed with brine (300 mL), dried over sodium sulfate and concentrated under reduced pressure. The crude compound was purified by Combi Flash column chromatography (Combi Flash, 0 to 100% Hexanes/Ethyl acetate). Pure fractions were combined and evaporated under reduced pressure to afford hex-5-enyl benzoate (37.0 g, 181.1 mmol, yield: 72.6%) as a colourless liquid.

¹H NMR (400 MHz, CDCl₃): 7.98 - 7.96 (m, 2H), 7.50 - 7.46 (m, 1H), 7.38 - 7.34 (m, 2H), 5.78 - 5.71 (m, 1H), 4.99 - 4.89 (m, 2H), 4.25(t, 2H), 2.09 - 2.03 (m, 2H), 1.75 - 1.68 (m, 2H), 1.52 - 1.44 (m, 2H).

### Step B : Preparation of 4-(2-chloro-2-fluorocyclopropyl)butyl benzoate :

A flask equipped with argon balloon was charged with hex-5-enyl benzoate (10.2 g, 44.9 mmol) in diglyme (100.0 mL) was added sodium 2,2-dichloro-2-fluoro acetate (18.2 g, 108 mmol) dropwise by syringe at 165 °C for 6 h. The resulting reaction mixture was stirred at 165 °C another for 2 h. The reaction progress was monitored by TLC (10% Ethyl acetate/hexanes), and it showed no starting material. The reaction mixture was filtered and filtrate was concentrated under reduced pressure to afford crude reaction mixture. The crude compound was purified by Combi Flash column chromatography (Combi Flash, 0 to 100% Hexanes/Ethyl acetate). Pure fractions were combined and evaporated under reduced pressure to afford 4-(2-chloro-2-fluoro-cyclopropyl)butyl benzoate (3.50 g, 12.8 mmol, yield: 28.5%) as a colorless liquid.

¹H NMR (400 MHz, DMSO-*d*₆): 8.05 - 8.04 (m, 2H), 7.56 - 7.52 (m, 1H), 7.46 - 7.42 (m, 2H), 4.34 (t, *J* = 6.8 Hz, 2H), 1.85 - 1.80 (m, 2H), 1.66 - 0.84 (m, 7H).

### Step C : Preparation of 4-(2-chloro-2-fluorocyclopropyl)butan-1-ol :

A flask equipped with argon balloon was charged with 4-(2-chloro-2-fluoro-cyclopropyl)butyl benzoate ( 6.00 g, 19.9 mmol) in dichloromethane (30 mL) and methanol (10 mL) at 0 °C was added potassium hydroxide (1.12 g, 19.9 mmol). The resulting reaction mixture was stirred at room temperature for 16 h. The reaction progress was monitored by TLC (30% Ethyl acetate/hexanes) and it showed no starting material. The reaction mixture was diluted with water (100 mL) and then extracted with dichloromethane (2 × 100 mL). The combined organic layers were washed with brine (100 mL), dried over sodium sulfate and concentrated under reduced pressure to afford 4-(2-chloro-2-fluoro-cyclopropyl)butan-1-ol (4.50 g, 13.5 mmol, crude) as a colour less liquid.

### Step D : Preparation of 4-(2-chloro-2-fluorocyclopropyl)butyl 4-methylbenzenesulfonate :

A flask equipped with argon balloon and charged with 4-(2-chloro-2-fluoro-cyclopropyl)butan-1-ol (5.50 g, 33.1 mmol) in dichloromethane (100 mL) at 0 °C were added triethylamine (13.9 mL, 99.3 mmol) and toluene-4-sulfonyl chloride (9.44 g, 49.6 mmol). The resulting reaction mixture was stirred at room temperature for 4 h. The reaction progress was monitored by TLC (30% Ethyl acetate/Hexanes) and it showed no starting material. The reaction mixture was quenched with saturated sodium bicarbonate solution (150 mL) and extracted with dichloromethane (2 × 150 mL). The combined organic layers were washed with water (150 mL) and brine (150 mL), dried over sodium sulfate and concentrated under reduced pressure to afford crude reaction mixture. The crude residue was purified by combi-flash column chromatography using (0 to 100%) ethyl acetate in hexanes. Pure fractions were combined and evaporated under reduced pressure to afford 4-(2-chloro-2-fluoro-cyclopropyl)butyl 4-methylbenzenesulfonate (3.50 g, 9.82 mmol, yield: 29.7%) as a colorless liquid.

¹H NMR (400 MHz, CDCl₃): δ 7.81 - 7.79 (m, 2H),7.37 - 7.35 (m, 2H), 4.09 - 4.01 (m, 2H), 2.49 (s, 3H), 1.75 - 1.68 (m, 2H),1.54 - 1.45 (m, 6H), 1.19 - 0.80 (m, 1H).

### Step E : Preparation of 2-(4-azidobutyl)-1 ,1-difluorocyclopropane :

A flask equipped with argon balloon and charged 4-(2-chloro-2-fluoro-cyclopropyl)butyl 4-methoxybenzenesulfonate (3.30 g, 9.80 mmol) in dimethylformamide (30 ml) was added sodium azide (1.27 g, 19.6 mmol) at 0°C, and stirred at room temperature for 12 h. Reaction progress was monitored by TLC (20% Ethyl acetate : Hexanes) and it showed no starting material. The reaction mixture was diluted with water (100 mL) and extracted with methyl *tert*-butyl ether (2 × 100 mL). The combined organic layers were washed with brine (200 mL), dried over anhydrous sodium sulfate and evaporated under reduced pressure, with a bath temperature at 25 °C, to afford 2-(4-azidobutyl)-1-chloro-1-fluoro-cyclopropane (2.20 g, 8.04 mmol, crude) as a colorless liquid. This crude product was directly used for the next step without purification.

### Step F : Preparation of 4-(2-chloro-2-fluorocyclopropyl)butan-1-amine hydrochloride :

A flask equipped with argon balloon and charged with 2-(4-azidobutyl)-1-chloro-1-fluoro-cyclopropane (60.0%, 2.20 g, 6.89 mmol) in ethyl acetate (25 mL) was added triphenylphosphine (2.17 g, 8.27 mmol) at room temperature portion wise and stirred for 12 h. Then the reaction mixture was cooled to 0°C and then hydrochloric acid (4 M) in 1,4-dioxane (20.0 mL) dropwise. The resulting reaction mixture was stirred at room temperature for 5 h. The reaction progress was monitored by TLC (50% Ethyl acetate /Hexanes) and it showed no starting material. The solvent was evaporated and the crude was triturated with methyl *tert*-butyl ether. The resulting residue was washed with methyl tert-butyl ether to afford 4-(2-chloro-2-fluoro-cyclopropyl)butan-1-amine;hydrochloride (1.80 g, 4.45 mmol, crude) as a brown liquid.

### Step G : Preparation of tert-butyl (4-(2-chloro-2-fluorocyclopropyl)butyl)carbamate :

A flask equipped with argon balloon and charged with 4-(2-chloro-2-fluoro-cyclopropyl)butan-1-amine (1.80 g, 6.52 mmol, crude) in dichloromethane (10 mL) at 0°C were added *N*,*N*-diethylethanamine (2.75 mL, 19.6 mmol) followed by di-*tert*-butyl dicarbonate (1.42 g, 6.52 mmol) dropwise. The resulting reaction mixture was stirred at room temperature for 12 h. The reaction progress was monitored by TLC (60% Ethyl acetate/Hexanes) and it showed no starting material. The solvent was removed under vacuum. The resulting crude residue was purified by combi flash column chromatography using (0 to 100%) ethyl acetate in hexanes. Pure fractions were combined and evaporated under reduced pressure to afford *tert*-butyl N-[4-(2-chloro-2-fluoro-cyclopropyl)butyl]carbamate (2.82 g, 6.37 mmol, crude) as a brown color gum.

### Step H : Preparation of 4-(2-chloro-2-fluorocyclopropyl)butan-1-amine hydrochloride :

A flask equipped with argon balloon and charged with *tert*-butyl N-[4-(2-chloro-2-fluoro-cyclopropyl)butyl]carbamate (90.0 %, 2.80 g, 9.48 mmol in dry dichloromethane (10 mL) at 0°C was added hydrochloric acid (4 M) in 1,4-dioxane (2.37 mL, 9.48 mmol) dropwise. The resulting reaction mixture was stirred at room temperature for 5 h. The reaction progress was monitored by TLC (30% Ethyl acetate /Hexanes) and it showed no starting material. The solvent was evaporated and the crude was triturated with methyl *tert*-butyl ether (2 × 25 mL) to afford 4-(2-chloro-2-fluorocyclopropyl)butan-1-amine hydrochloride (1.60 g, 10.5 mmol, yield: 75%) as an off white solid.

¹H NMR (400 MHz, DMSO-*d*₆): δ 8.13 (brs, 3H), 2.76 - 2.73 (m, 2H),1.71 - 1.00 (m, 9H).

¹⁹F NMR (376 MHz, DMSO-*d*₆): δ -128.64 (s, 1F), -149.23 (s, 0.73F).

### Example 8: Preparation of N-(4-(2-chloro-2-fluorocyclopropyl)butyl)benzamide :

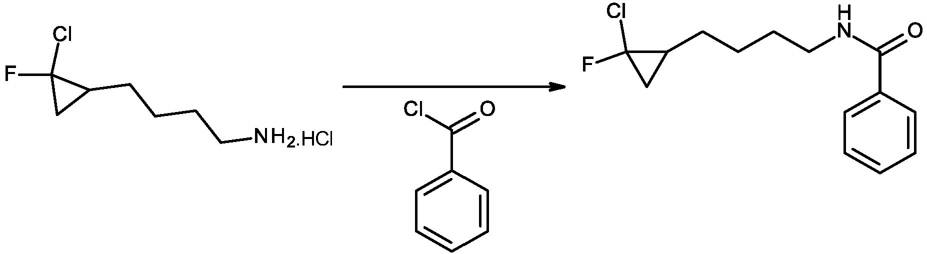

A flask equipped with argon balloon and charged with 4-(2-chloro-2-fluoro-cyclopropyl)butan-1-amine;hydrochloride (256 mg, 1.27 mmol) in dichloromethane (10 ml) at 0 °C were added triethylamine (0.535 mL, 3.81 mmol) followed by benzoyl chloride (178 mg, 1.27 mmol) drop wise. The resulting reaction mixture was stirred at room temperature for 3 h. The reaction progress was monitored by TLC (80% Ethyl acetate/Hexanes) and it showed no starting material, reaction mixture was diluted with water (50 mL) and extracted with dichloromethane (2 × 50 mL). The combined organic layers were washed with brine (50 mL), dried over sodium sulfate and concentrated under reduced pressure to afford crude reaction mixture. The resulting crude residue was purified by combi flash column chromatography using (0 to 100%) ethyl acetate in hexanes. Pure fractions were combined and evaporated under reduced pressure to afford N-[4-(2-chloro-2-fluoro-cyclopropyl)butyl]benzamide (120 mg, 0.400 mmol, yield: 31.5%) as a light-yellow liquid.

¹H NMR (400 MHz, CDCl₃): δ 7.77 - 7.75 (m, 2H), 7.51 - 7.48 (m, 3H), 6.13 (brs, 1H), 1.71-1.48 (m, 5H), 3.51 - 3.48 (m, 2H), 1.84 - 1.56 (m, 2H), 0.88 - 0.78 (m, 2H).

¹⁹F NMR (376 MHz, CDCl₃): δ -130.31 (s, 1F), -151.25 (s, 0.96F).

### Example 9: Preparation of N-(4-(2-chloro-2-fluorocyclopropyl)butyl)ethane sulfonamide:

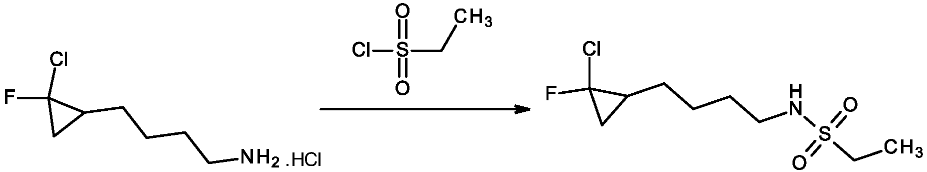

A flask equipped with argon balloon and charged with 4-(2-chloro-2-fluoro-cyclopropyl)butan-1-amine;hydrochloride (70.0 mg, 0.346 mmol) in dichloromethane (10 mL) at 0 °C were added triethylamine (0.178 mL, 1.27 mmol) followed by ethane sulfonyl chloride (0.0418 mL, 0.423 mmol) drop wise. The resulting reaction mixture was stirred at room temperature for 5 h. The reaction progress was monitored by TLC (80% Ethyl acetate /Hexanes) and it showed no starting material. Reaction mixture was diluted with water (10 mL) and extracted with dichloromethane (2 × 10 mL). The combined organic layers were washed with brine (10 mL), dried over sodium sulfate and concentrated under reduced pressure to afford crude reaction mixture. The resulting crude residue was purified by combi-flash column chromatography using (0 to 100%) ethyl acetate in hexanes. Pure fractions were combined and evaporated under reduced pressure to afford N-[4-(2-chloro-2-fluoro-cyclopropyl)butyl]ethane sulfonamide (0.125 g, 0.412 mmol, yield: 97.5%) as a light-yellow liquid.

¹H NMR (400 MHz, CDCl₃): δ 4.14 - 4.09 (m, 1H), 3.15 - 3.11 (m, 2H),3.07 - 3.01(m, 2H), 1.63 - 1.35 (m, 9H), 1.12 - 1.09 (m, 1H), 0.86 - 0.82 (m, 2H).

¹⁹F NMR (376 MHz, CDCl₃): δ -130.40 (s, 1F), -151.19 (s, 1F).

### Example 10: Preparation of N-(4-(2-chloro-2-fluorocyclopropyl)butyl)pyridin-2-amine :

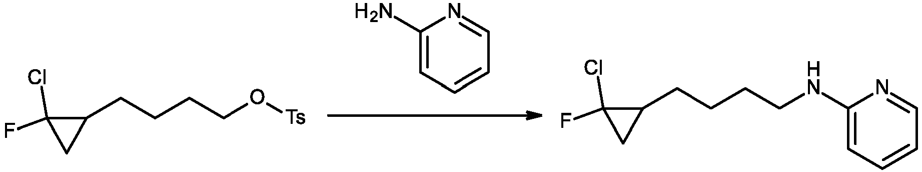

A flask equipped with argon balloon and charged with 4-(2-chloro-2-fluoro-cyclopropyl)butyl 4-methylbenzenesulfonate (200 mg, 0.623 mmol) in dimethylformamide (10.0 mL) were added pyridin-2-amine (0.0587 g, 0.623 mmol) and cesium carbonate (0.203 g, 0.623 mmol)) at 0 °C. The resulting reaction mixture was stirred at 130 °C for 16h. The reaction progress was monitored by TLC (30% Ethyl acetate /Hexanes) and it showed no starting material. Reaction mixture was diluted with water (30 mL) and extracted with ethyl acetate (2 × 15 mL). The combined organic layers were washed with brine (10 mL), dried over sodium sulfate and concentrated under reduced pressure to afford crude reaction mixture. The resulting crude residue was purified by combi flash column chromatography using (0 to 100%) ethyl acetate in hexanes. Pure fractions were combined and evaporated under reduced pressure to afford N-[4-(2-chloro-2-fluoro-cyclopropyl)butyl]pyridin-2-amine (0.0800 g, 0.297 mmol, yield: 47.6%) as a colorless oil.

¹H NMR (400 MHz, CDCl₃): δ 8.08 - 8.06 (m,1H), 7.43 - 7.39 (m, 1H), 6.57- 6.54 (m, 1H), 6.37 (d, 1H), 4.69 (brs, 1H), 3.31 - 3.26 (m, 2H), 1.78 - 1.44 (m, 8H), 1.10 - 1.06 (m, 0.5H), 0.83 - 0.82 (m, 0.5H).

¹⁹F NMR (376 MHz, CDCl₃): δ -130.29 (s, 1F), -151.2 (s, 0.90F).

The compounds A1 to A41 may be prepared by analogy with the reactions above.

**Table A: This table discloses compounds of the formula (I):**

| Entry | IUPAC name | Molecule | RT (min) | [M+H] (measured) | Method |
|---|---|---|---|---|---|
| A1 | 4-(2,2-difluorocyclopropyl)butylam monium;chloride | 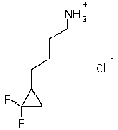 | See NMR below | | |
| A2 | N-[6-(2,2-difluorocyclopropyl)hexyl]pyr idin-2-amine | 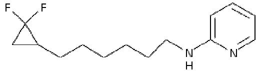 | See NMR below | | |
| A3 | N-[6-(2,2-difluorocyclopropyl)hexyl]be nzamide | 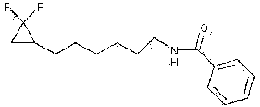 | See NMR below | | |
| A4 | N-[6-(2,2-difluorocyclopropyl)hexyl]cyc lobutanesulfonamide | 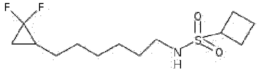 | See NMR below | | |
| A5 | N-[6-(2,2-difluorocyclopropyl)hexyl]eth anesulfonamide | 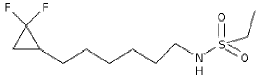 | See NMR below | | |
| A6 | 6-(2,2-difluorocyclopropyl)hexylam monium;chloride | 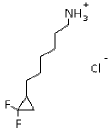 | See NMR below | | |
| A7 | N-[4-(2-chloro-2-fluoro-cyclopropyl)butyl]-5-methyl-isoxazole-3-carboxamide | 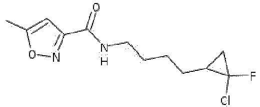 | 1.41 | 275.08 | A |
| A8 | N-[4-(2-chloro-2-fluoro-cyclopropyl)butyl]-2-fluoro-acetamide | 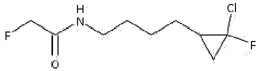 | 1.14 | 226.11 | A |
| A9 | N-[4-(2-chloro-2-fluoro-cyclopropyl)butyl]-2,6-difluoro-benzamide | 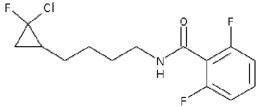 | 1.45 | 306.07 | A |
| A10 | N-[4-(2-chloro-2-fluoro-cyclopropyl)butyl]naphthalen e-2-carboxamide | 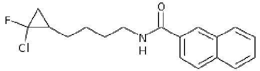 | 1.68 | 320.11 | A |
| A11 | 2-tert-butoxy-N-[4-(2-chloro-2-fluoro-cyclopropyl)butyl]acetamide | 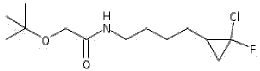 | 1.54 | 280.13 | A |
| A12 | N-[4-(2-chloro-2-fluoro-cyclopropyl)butyl]thiazole-2-carboxamide | 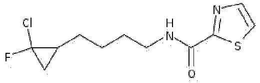 | 1.41 | 277.05 | A |
| A13 | (E)-N-[4-(2-chloro-2-fluoro-cyclopropyl)butyl]-2-methyl-but-2-enamide | 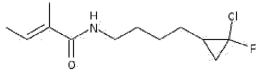 | 1.39 | 248.11 | A |
| A14 | N-[4-(2-chloro-2-fluoro-cyclopropyl)butyl]-6,6,6-trifluoro-hexanamide | 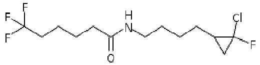 | 1.54 | 318.12 | A |
| A15 | N-[4-(2-chloro-2-fluoro-cyclopropyl)butyl]-4-methoxy-benzamide | 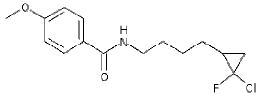 | 1.46 | 300.11 | A |
| A16 | N-[4-(2-chloro-2-fluoro-cyclopropyl)butyl]-2-(4-fluorophenyl)acetamide | 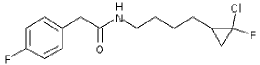 | 1.48 | 302.09 | A |
| A17 | N-[4-(2-chloro-2-fluoro-cyclopropyl)butyl]-6-methyl-pyridine-3-sulfonamide | 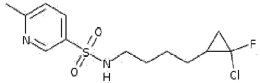 | 1.36 | 321.09 | A |
| A18 | N-[4-(2-chloro-2-fluoro-cyclopropyl)butyl]quinoline-2-carboxamide | 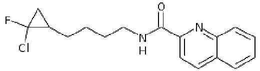 | 1.77 | 321.12 | A |
| A19 | N-[4-(2-chloro-2-fluoro-cyclopropyl)butyl]-2-phenoxy-acetamide | 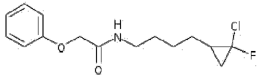 | 1.55 | 300.11 | A |
| A20 | 4-(2-chloro-2-fluoro-cyclopropyl)-N-(methylsulfamoyl)butan-1-amine | 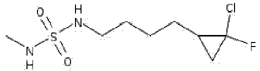 | 1.15 | 259.07 | A |
| A21 | N-[4-(2-chloro-2-fluoro-cyclopropyl)butyl]-3-phenyl-propanamide | 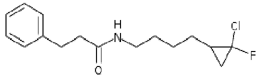 | 1.54 | 298.12 | A |
| A22 | N-[4-(2-chloro-2-fluoro-cyclopropyl)butyl]-6-methoxy-pyridine-3-sulfonamide | 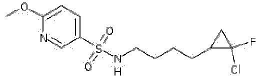 | 1.51 | 337.08 | A |
| A23 | N-[4-(2-chloro-2-fluoro-cyclopropyl)butyl]-2,2-dimethyl-propanamide | 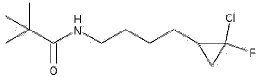 | 1.46 | 250.13 | A |
| A24 | 1-chloro-2-[4-(dimethylsulfamoylamino)but yl]-1-fluoro-cyclopropane | 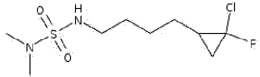 | 1.36 | 273.06 | A |
| A25 | N-[4-(2-chloro-2-fluoro-cyclopropyl)butyl]-1-methyl-cyclopropanecarboxamide | 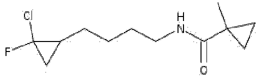 | 1.38 | 248.13 | A |
| A26 | N-[4-(2-chloro-2-fluoro-cyclopropyl)butyl]-1-(methoxymethyl)cyclopropan esulfonamide | 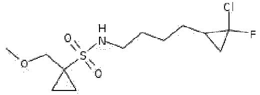 | 1.45 | 314.09 | A |
| A27 | N-[4-(2-chloro-2-fluoro-cyclopropyl)butyl]-3-methyl-but-2-enamide | 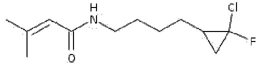 | 1.41 | 248.13 | A |
| A28 | N-[4-(2-chloro-2-fluoro-cyclopropyl)butyl]butane-1-sulfonamide | 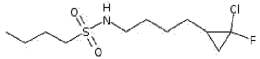 | 1.53 | 286.09 | A |
| A29 | N-[4-(2-chloro-2-fluoro-cyclopropyl)butyl]-4-fluoro-benzamide | 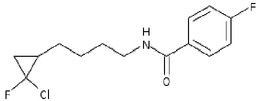 | 1.49 | 288.09 | A |
| A30 | N-[4-(2-chloro-2-fluoro-cyclopropyl)butyl]-1-methylpyrazole-3-sulfonamide | 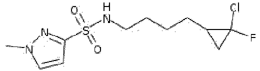 | 1.25 | 310.07 | A |
| A31 | N-[4-(2-chloro-2-fluoro-cyclopropyl)butyl]thiadiazole -4-carboxamide | 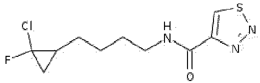 | 1.32 | 278.05 | A |
| A32 | N-[4-(2-chloro-2-fluoro-cyclopropyl)butyl]-3,5-dimethyl-isoxazole-4-sulfonamide | 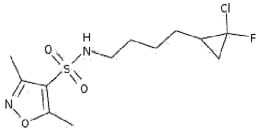 | 1.5 | 325.09 | A |
| A33 | N-[4-(2-chloro-2-fluoro-cyclopropyl)butyl]-4-methylsulfanyl-benzamide | 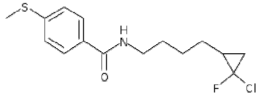 | 1.59 | 316.1 | A |
| A34 | 4-chloro-N-[4-(2-chloro-2-fluoro-cyclopropyl)butyl]benzamide | 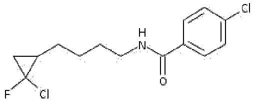 | 1.62 | 304.07 | A |
| A35 | N-[4-(2-chloro-2-fluoro-cyclopropyl)butyl]-2,2-difluoro-1,3-benzodioxole-5-carboxamide | 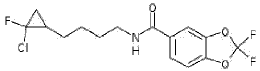 | 1.71 | 350.1 | A |
| A36 | N-[4-(2-chloro-2-fluoro-cyclopropyl)butyl]-2-methylpyrazole-3-carboxamide | 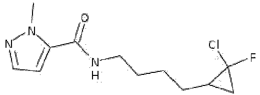 | 1.27 | 274.11 | A |
| A37 | 4-(2-chloro-2-fluoro-cyclopropyl)butan-1-amine | 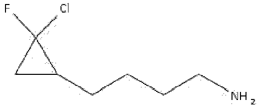 | See NMR below | | |
| A38 | N-[4-(2-chloro-2-fluoro-cyclopropyl)butyl]pyridin-2-amine | 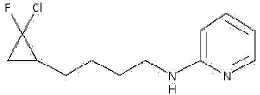 | See NMR below | | |
| A39 | N-[4-(2-chloro-2-fluoro-cyclopropyl)butyl]cyclobutan esulfonamide | 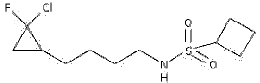 | 1.41 | 284.08 | A |
| A40 | N-[4-(2-chloro-2-fluoro-cyclopropyl)butyl]benzamide | 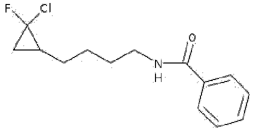 | See NMR below | | |
| A41 | N-[4-(2-chloro-2-fluoro-cyclopropyl)butyl]ethanesulf onamide | 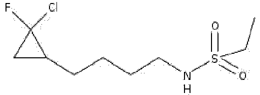 | See NMR below | | |

| | | | | | |
|---|---|---|---|---|---|
| * Rt = retention time | | | | | |

| Entry | NMR |
|---|---|
| A1 | ¹H NMR (400 MHz, DMSO-*d*₆): δ 8.07 (brs, 3H), 2.78 - 2.72 (m, 2H),1.63 - 1.41 (m, 8H), 1.10 - 1.06 (m, 1H). |
| | ¹⁹F NMR (376 MHz, DMSO-*d*₆): δ -126.42 (d, 1F), -142.48 (d, 0.97F). |
| A2 | ¹H NMR (400 MHz, DMSO-*d*₆): δ 9.73 (s, 1H), 8.24 - 8.22 (m, 1H), 7.79 - 7.69 (m, 2H), 7.02 - 6.99 (m, 1H), 1.47 (s, 9H). |
| A3 | ¹H NMR (400 MHz, DMSO-*d*₆): δ 8.48 - 8.44(m, 1H), 7.84 - 7.62 (m, 2H), 7.51 - 7.42 (m, 3H), 3.34 - 3,22 (m, 2H), 1.60 - 1.31 (m, 12H),1.04 - 1.01 (m, 1H). |
| | ¹⁹F NMR (376 MHz, DMSO-d6): δ -126.35 (d, 1F), -146.63 (d, 0.98). |
| A4 | ¹H NMR (400 MHz, DMSO-*d*₆): δ 6.99 - 6.91(m, 1H), 3.88 - 3.79 (m, 1H), 2.91 - 2.87 (m, 2H), 2.31 - 2.12 (m, 4H), 1.96 - 1.81 (m, 2H), 1.69 - 1.21 (m, 12H), 1.08 - 1.00 (m, 1H). |
| | ¹⁹F NMR (376 MHz, DMSO-*d*₆): δ -126.37 (d, 1F), -142.66 (d, 0.91F). |
| A5 | ¹H NMR (400 MHz, DMSO-*d*₆): δ 6.99 - 6.96(m, 1H), 2.97 - 2.93 (m, 2H), 2.90 - 2.85 (m, 2H), 1.46 - 1.28 (m, 12H), 1.17 (t, 3H),1.08 - 1.00 (m, 1H). |
| | ¹⁹F NMR (376 MHz, DMSO-d6): δ -126.34 (d, 1F), -142.62 (d, 0.91F). |
| A6 | ¹H NMR (400 MHz, DMSO-*d*₆): δ 8.06 (brs, 3H), 2.76 - 2.70 (m, 2H), 1.66 - 1.30 (m, 12H), 1.06 - 1.02 (m, 1H). |
| | ¹⁹F NMR (376 MHz, DMSO-*d*₆): δ -126.35 (d, 1F), -142.58 (d, 0.98F). |
| A37 | ¹H NMR (400 MHz, DMSO-*d*₆): δ 8.13 (brs, 3H), 2.76 - 2.73 (m, 2H),1.71 - 1.00 (m, 9H). ¹⁹F NMR (376 MHz, DMSO-d6): δ -128.64 (s, 1F), -149.23 (s, 0.73F). |
| A38 | ¹H NMR (400 MHz, CDCl₃): δ 8.08 - 8.06 (m,1H), 7.43 - 7.39 (m, 1H), 6.57- 6.54 (m, 1H), 6.37 (d, 1H), 4.69 (brs, 1H), 3.31 - 3.26 (m, 2H), 1.78 - 1.44 (m, 8H), 1.10 - 1.06 (m, 0.5H), 0.83 - 0.82 (m, 0.5H). |
| | ¹⁹F NMR (376 MHz, CDCl₃): δ -130.29 (s, 1F), -151.2 (s, 0.90F). |
| A40 | ¹H NMR (400 MHz, CDCl₃): δ 7.77 - 7.75 (m, 2H), 7.51 - 7.48 (m, 3H), 6.13 (brs, 1H), 1.71-1.48 (m, 5H), 3.51 - 3.48 (m, 2H), 1.84 - 1.56 (m, 2H), 0.88 - 0.78 (m, 2H). |
| | ¹⁹F NMR (376 MHz, CDCl₃): δ -130.31 (s, 1F), -151.25 (s, 0.96F). |
| A41 | ¹H NMR (400 MHz, CDCl₃): δ 4.14 - 4.09 (m, 1H), 3.15 - 3.11 (m, 2H), 3.07 - 3.01 (m, 2H), 1.63 - 1.35 (m, 9H), 1.12 - 1.09 (m, 1H), 0.86 - 0.82 (m, 2H). |
| | ¹⁹F NMR (376 MHz, CDCl₃): δ -130.40 (s, 1F), -151.19 (s, 1F). |

### BIOLOGICAL EXAMPLES:

### Myzus persicae (Green peach aphid) Systemic activity

Roots of pea seedlings infested with an aphid population of mixed ages were placed directly into aqueous test solutions prepared from 10,000 ppm DMSO stock solutions. The samples were assessed for mortality 6 days after placing seedlings into test solutions.

The following compounds resulted in at least 80% mortality at a test rate of 24 ppm:
A41.

### Myzus persicae (Green peach aphid) Intrinsic activity

Test compounds prepared from 10,000 ppm DMSO stock solutions were applied by pipette into 24-well microtiter plates and mixed with sucrose solution. The plates were closed with a stretched Parafilm. A plastic stencil with 24 holes was placed onto the plate and infested pea seedlings were placed directly on the Parafilm. The infested plate was closed with a gel blotting paper and another plastic stencil and then turned upside down. The samples were assessed for mortality 5 days after infestation.

The following compounds resulted in at least 80% mortality at a test rate of 12 ppm:
A21.

### Myzus persicae (Green peach aphid) Feeding/Contact activity

Eggplant leaf discs were placed onto agar in a 24-well microtiter plate and sprayed with aqueous test solutions prepared from 10,000 ppm DMSO stock solutions. After drying, the leaf discs were infested with an aphid population of mixed ages. The samples were assessed for mortality 6 days after infestation.

The following compounds resulted in at least 80% growth inhibition at an application rate of 200 ppm:
A12, A14, A39.

### Aphis craccivora (Black bean aphid), mixed population, systemic/feeding

Pea seedlings infested with mixed aged aphid population, were put into glass vials with test solutions. 5 days later, samples were assessed for mortality.

The following compounds resulted in at least 80% mortality at a test rate of 12 ppm:
A2, A38.

## Claims

1. A compound of Formula (I): 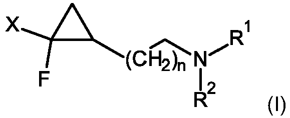 wherein:
n is 3 or 5;
X is F or Cl;
R¹ is hydrogen, hydroxy, C₁-C₆alkyl, C₁-C₄haloalkyl, C₁-C₆alkoxy, C₁-C₄haloalkoxy, C₂-C₆alkenyl, C₂-C₄haloalkenyl, C₂-C₆alkynyl, C₂-C₄haloalkynyl, C₁-C₄alkylsulfanyl, C₁-C₄alkylsulfinyl, C₁-C₄haloalkylsulfanyl, C₁-C₄haloalkylsulfinyl, phenoxy, -N=C(H)Ph, -NHC₁-C₄alkyl, -N(C₁-C₂alkyl)(C₁-C₂alkyl), C₁-C₄alkoxy substituted by a cyclopropyl group, C₁-C₄nitroalkyl, C₁-C₄alkoxyC₁-C₄alkyl, C₁-C₄haloalkoxyC₁-C₄alkyl, C₁-C₄alkylsulfonylC₁-C₄alkyl, 4-(2,2-difluorocyclopropyl)butyl, 4-(2-chloro-2-fluorocyclopropyl)butyl, 6-(2,2-difluorocyclopropyl)hexyl, 6-(2-chloro-2-fluorocyclopropyl)hexyl, -SO₂R⁴ or -C(O)R⁵; or
R¹ is phenyl optionally substituted by 1 to 3 substituents independently selected from R³, C₃-C₆cycloalkyl optionally substituted by 1 to 3 substituents independently selected from R³, a 4- to 6-membered saturated or partially saturated heterocyclic ring system comprising 1 or 2 heteroatoms individually selected from N, O, and S, wherein the heterocyclic ring is optionally substituted by 1 to 3 substituents independently selected from R³, a 5- or 6-membered heteroaromatic ring system comprising 1 to 3 heteroatoms individually selected from N, O, and S, wherein the heteroaromatic ring is optionally substituted by 1 to 3 substituents independently selected from R³, or a 9- or 10-membered heteroaromatic bicyclic ring system comprising 1 to 4 nitrogen atoms, or 0, 1 or 2 nitrogen atoms and a single atom selected from O or S, wherein the heteroaromatic bicyclic ring is optionally substituted by 1 to 3 substituents independently selected from R³; or
R¹ is C₁-C₂alkyl mono-substituted by:
(i) a 5- or 6-membered heteroaromatic ring system comprising 1 to 3 heteroatoms individually selected from N, O, and S, wherein the heteroaromatic ring is optionally substituted by 1 to 3 substituents independently selected from R³,
(ii) phenyl optionally substituted by 1 to 3 substituents independently selected from R³,
(iii) a 4- to 6-membered saturated or partially saturated heterocyclic ring system comprising 1 or 2 heteroatoms individually selected from N, O, and S, wherein the heterocyclic ring is optionally substituted by 1 to 3 substituents independently selected from R³, or
(iv) C₃-C₆cycloalkyl optionally substituted by 1 to 3 substituents independently selected from R³;
and
R² is hydrogen, amino, C₁-C₆alkyl, C₂-C₆alkenyl, C₂-C₆alkynyl, C₁-C₄cyanoalkyl, C₁-C₄alkoxyC₁-C₄alkyl, C₁-C₄haloalkoxyC₁-C₄alkyl, or -C(O)C₁-C₄alkyl;
or
R¹ and R², together with the nitrogen atom they share, form a 4- to 6-membered saturated or partially saturated heterocyclic ring system, optionally further comprising 1 or 2 heteroatoms individually selected from N, O, and S, wherein the heterocyclic ring is optionally substituted by 1 to 3 substituents independently selected from R⁶, or R¹ and R², together with the nitrogen atom they share, form a 9-membered saturated or partially saturated heterocyclic ring system comprising 1 or 2 heteroatoms individually selected from N, O, and S;
R³ is halogen, cyano, nitro, hydroxyl, formyl, C₁-C₆alkyl, C₁-C₄haloalkyl, C₂-C₆alkenyl, C₂-C₄haloalkenyl, C₂-C₆alkynyl, C₂-C₄haloalkynyl, C₃-C₆cycloalkyl, C₃-C₆halocycloalkyl, C₁-C₄alkylsulfanyl, C₁-C₄alkylsulfinyl, C₁-C₄alkylsulfonyl, C₁-C₄haloalkylsulfanyl, C₁-C₄haloalkylsulfinyl, C₁-C₄haloalkylsulfonyl, -C(O)C₁-C₄alkyl, -C(O)NH(C₁-C₄alkyl), -C(O)N(C₁-C₄alkyl)₂, C₁-C₆alkoxy, C₁-C₄alkoxyC₁-C₄alkyl, C₁-C₄haloalkoxy, phenyl, thiophen, C₁-C₄alkylNHC(O)C₁-C₄alkyl, C₁-C₄alkylC(O)NHC₁-C₄alkyl, -NC(O)(C₁-C₄alkyl)C₁-C₄alkyl, -NHC(O)C₁-C₄alkyl , C₁-C₄alkylNHS(O)₂-, - NHS(O)₂C₁-C₄alkyl, -N(C₁-C₄alkyl)S(O)₂C₁-C₄alkyl, -OCH₂C₃-C₆cycloalkyl, C₁-C₄cyanoalkyl, or C₃-C₆cycloalkyl substituted by a cyano;
R⁴ is C₁-C₆alkyl, C₁-C₄haloalkyl, C₁-C₆alkoxy, or C₁-C₆haloalkoxy; or
R⁴ is C₃-C₆cycloalkyl optionally substituted by 1 to 3 substituents independently selected from R³, phenyl optionally substituted by 1 to 3 substituents independently selected from R³; a 5- or 6-membered heteroaromatic ring system comprising 1 to 3 heteroatoms individually selected from N, O, and S, wherein the heteroaromatic ring is optionally substituted by 1 to 3 substituents independently selected from R³; or a 4- to 6-membered saturated or partially saturated heterocyclic ring system comprising 1 or 2 heteroatoms individually selected from N, O, and S, wherein the heterocyclic ring is optionally substituted by 1 to 3 substituents independently selected from R³; or
R⁴ is C₁-C₂alkyl mono-substituted by:
(i) a 5- or 6-membered heteroaromatic ring system comprising 1 to 3 heteroatoms individually selected from N, O, and S, wherein the heteroaromatic ring is optionally substituted by 1 to 3 substituents independently selected from R³,
(ii) phenyl optionally substituted by 1 to 3 substituents independently selected from R³,
(iii) a 4- to 6-membered saturated or partially saturated heterocyclic ring system comprising 1 or 2 heteroatoms individually selected from N, O, and S, wherein the heterocyclic ring is optionally substituted by 1 to 3 substituents independently selected from R³, or
(iv) C₃-C₆cycloalkyl optionally substituted by 1 to 3 substituents independently selected from R³; or
R⁴ is -NH(C₁-C₆alkyl), -N(C₁-C₂alkyl)(C₁-C₂alkyl), -NH(C₂-C₆alkenyl), -N(C₂-C₆alkenyl)(C₁-C₆alkyl), -NH(C₂-C₆alkynyl), -N(C₂-C₆alkynyl)(C₁-C₆alkyl), -NH(C₃-C₆cycloalkyl), -N(C₃-C₆cycloalkyl)(C₁-C₆alkyl), or -NH(phenyl) or -NH(CH₂phenyl), wherein phenyl is optionally substituted by 1 to 3 substituents independently selected from R⁶;
R⁵ is C₁-C₆alkyl, C₁-C₆haloalkyl, C₁-C₆alkoxy, C₁-C₄haloalkoxy, C₂-C₆alkenyl, C₁-C₄alkoxyC₁-C₄alkyl, 3-(2,2-difluorocyclopropyl)propyl, 3-(2-chloro-2-fluorocyclopropyl)propyl, 5-(2,2-difluorocyclopropyl)pentyl, 5-(2-chloro-2-fluorocyclopropyl)pentyl, benzylamino, or naphthyl; or
R⁵ is phenyl optionally substituted by 1 to 3 substituents independently selected from R⁶, a 5- or 6-membered heteroaromatic ring system comprising 1 to 3 heteroatoms individually selected from N, O, and S, wherein the heteroaromatic ring is optionally substituted by 1 to 3 substituents independently selected from R⁶, a 9- or 10-membered heteroaromatic bicyclic ring system comprising 1 to 4 nitrogen atoms, or 0, 1 or 2 nitrogen atoms and a single atom selected from O or S; a 5- to 6-membered saturated heterocyclic ring system comprising 1 or 2 heteroatoms individually selected from N, O, and S, wherein the heterocyclic ring is optionally substituted by 1 to 3 substituents independently selected from R⁶; or C₃-C₆cycloalkyl optionally substituted by 1 to 3 substituents independently selected from R⁶; or
R⁵ is C₁-C₂alkyl mono-substituted by:
(i) phenoxy; or
(ii) phenyl optionally substituted by 1 to 3 substituents independently selected from R⁶; or
R⁵ is 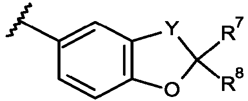 wherein Y is O or -CH₂;
R⁶ is halogen, cyano, C₁-C₄alkyl, C₁-C₄haloalkyl, C₁-C₄alkoxy, C₁-C₄haloalkoxy, C₁-C₄alkylsulfanyl, or C₁-C₄alkylNHSO₂-; and
R⁷ and R⁸ are independently selected from hydrogen, fluoro, or chloro, or R⁷ and R⁸, together with the carbon atom they share, form a C=O group;
or a salt, stereoisomer, enantiomer, tautomer or N-oxide thereof.

2. A compound according to claim 1, wherein R¹ is hydrogen, hydroxy, C₁-C₄alkyl, C₁-C₄haloalkyl, C₁-C₄alkoxy, C₂-C₄alkynyl, -N=C(H)Ph, C₄-C₆cycloalkyl, -N(C₁-C₂alkyl)(C₁-C₂alkyl), 4-(2,2-difluorocyclopropyl)butyl, 4-(2-chloro-2-fluorocyclopropyl)butyl, 6-(2,2-difluorocyclopropyl)hexyl, 6-(2-chloro-2-fluorocyclopropyl)hexyl, -SO₂R⁴, or -C(O)R⁵; or R¹ is phenyl, benzyl, pyridyl, (pyridyl)methyl, (pyridyl)ethyl, or pyrimidinyl, which are optionally substituted by 1 or 2 substituents selected from R³.

3. A compound according to claim 1 or claim 2, wherein R³ is halogen, cyano, hydroxy, C₁-C₄alkyl, C₁-C₄alkoxy, C₁-C₄alkoxy-C₁-C₄alkyl, C₁-C₂alkylsulfonyl, or C₁-C₂cyanoalkyl.

4. A compound according to any one of claims 1 to 3, wherein R⁴ is C₁-C₄alkyl, pyrrolidinyl, or piperidyl; or R⁴ is a ring selected from cyclopropyl, cyclobutyl, imidazolyl, triazolyl, thiophenyl, isoxazolyl, pyridyl, pyrimidinyl, pyrazolyl, or phenyl, wherein the ring is optionally substituted by 1 or 2 substituents independently selected from R³; or R⁴ is C₁-C₂alkyl mono-substituted by cyclopropyl, cyclobutyl, pyridyl, or phenyl, which are optionally substituted by 1 or 2 substituents independently selected from R³; or R⁴ is -NH(C₁-C₄alkyl), or -N(C₁-C₂alkyl)(C₁-C₂alkyl).

5. A compound according to 1 or claim 2, wherein R⁵ is C₁-C₄alkyl, C₁-C₆haloalkyl, C₂-C₄alkenyl, C₁-C₄alkoxy-C₁-C₄alkyl, 3-(2,2-difluorocyclopropyl)propyl, 3-(2-chloro-2-fluorocyclopropyl)propyl, 5-(2,2-difluorocyclopropyl)pentyl, 5-(2-chloro-2-fluorocyclopropyl)pentyl,benzylamino, naphthyl, quinolyl, or 2,2-difluoro-1,3-benzodioxolyl; or R⁵ is a ring selected from cyclopropyl, cyclobutyl, phenyl, pyrrolidinyl, piperidyl, 4,5-dihydroisoxazolyl, isoxazolyl, pyrrolyl, pyrazolyl, thiazolyl, thiadiazolyl, imidazolyl, isothiazolyl, pyridazinyl, or pyridyl, wherein each ring is optionally substituted by 1 or 2 substituents independently selected from R⁶; or R⁵ is C₁-C₂alkyl mono-substituted by: (i) phenoxy; or (ii) phenyl optionally substituted by a single substituent independently selected from R⁶.

6. A compound according to claim 5, wherein R⁶ is chloro, fluoro, methyl, ethyl, trifluoromethyl, methoxy, ethoxy or methylsulfanyl.

7. A compound according to claim 1 or claim 2, wherein R¹ is -SO₂R⁴, wherein:
R⁴ is C₁-C₄alkyl, C₁-C₄haloalkyl, C₁-C₄alkoxy, or C₁-C₄haloalkoxy; or R⁴ is C₃-C₆cycloalkyl optionally substituted by 1 or 2 substituents independently selected from R³; phenyl optionally substituted by 1 or 2 substituents independently selected from R³; a 5- or 6-membered heteroaromatic ring system comprising 1 or 2 heteroatoms individually selected from N, O, and S, wherein the heteroaromatic ring is optionally substituted by 1 or 2 substituents independently selected from R³; or a 5- or 6-membered saturated heterocyclic ring system comprising 1 or 2 nitrogen atoms; or R⁴ is C₁-C₂alkyl mono-substituted by: (i) a 5- or 6-membered heteroaromatic ring system comprising 1 or 2 nitrogen atoms, wherein the heteroaromatic ring is optionally substituted by a single substituent independently selected from R³, (ii) phenyl optionally substituted by 1 or 2 substituents independently selected from R³, or (iii) C₃-C₆cycloalkyl optionally substituted by a single substituents independently selected from R³; or R⁴ is -NH(C₁-C₄alkyl), or -N(C₁-C₂alkyl)(C₁-C₂alkyl); and
R³ is halogen, cyano, hydroxy, C₁-C₄alkyl, C₁-C₄alkoxy, C₁-C₄alkoxy-C₁-C₄alkyl, C₁-C₂alkylsulfonyl, or C₁-C₂cyanoalkyl.

8. A compound according to claim 1 or claim 2, wherein R¹ is -C(O)R⁵, wherein:
R⁵ is C₁-C₄alkyl, C₁-C₆haloalkyl, C₂-C₄alkenyl, C₁-C₄alkoxy-C₁-C₄alkyl, 3-(2,2-difluorocyclopropyl)propyl, 3-(2-chloro-2-fluorocyclopropyl)propyl, 5-(2,2-difluorocyclopropyl)pentyl, 5-(2-chloro-2-fluorocyclopropyl)pentyl,benzylamino, or naphthyl; or R⁵ is phenyl optionally substituted by 1 or 2 substituents independently selected from R⁶, a 5- or 6-membered heteroaromatic ring system comprising 1 or 2 heteroatoms individually selected from N, O, and S, wherein the heteroaromatic ring is optionally substituted by 1 or 2 substituents independently selected from R⁶, a 9- or 10-membered heteroaromatic bicyclic ring system comprising 1 to 4 nitrogen atoms, or 0, 1 or 2 nitrogen atoms and a single atom selected from O or S; a 5- to 6-membered saturated heterocyclic ring system comprising 1 or 2 heteroatoms individually selected from N, O, and S, wherein the heterocyclic ring is optionally substituted by 1 or 2 substituents independently selected from R⁶; or C₃-C₆cycloalkyl optionally substituted by a single substituent independently selected from R⁶; or
R⁵ is C₁-C₂alkyl mono-substituted by:
(i) phenoxy; or
(ii) phenyl optionally substituted by a single substituent independently selected from R⁶; or
R⁵ is 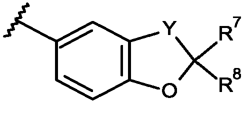 wherein Y is O or -CH₂; and
R⁶ is chloro, fluoro, methyl, ethyl, trifluoromethyl, methoxy, ethoxy or methylsulfanyl.

9. A compound according to claim 1 or claim 2, wherein:
R¹ is hydrogen, hydroxy, C₁-C₄alkyl, C₁-C₄haloalkyl, C₁-C₄alkoxy, C₂-C₄alkynyl, -N=C(H)Ph, C₃-C₆cycloal kyl, -N(C₁-C₂alkyl)(C₁-C₂alkyl), 4-(2,2-difluorocyclopropyl)butyl, 4-(2-chloro-2-fluorocyclopropyl)butyl, 6-(2,2-difluorocyclopropyl)hexyl, 6-(2-chloro-2-fluorocyclopropyl)hexyl, -SO₂R⁴ or -C(O)R⁵; or R¹ is phenyl, benzyl, pyridyl, (pyridyl)methyl, (pyridyl)ethyl, or pyrimidinyl, which are optionally substituted by 1 or 2 substituents independently selected from R³; and wherein
R³ is halogen, cyano, hydroxy, C₁-C₄alkyl, C₁-C₄alkoxy, C₁-C₄alkoxy-C₁-C₄alkyl, C₁-C₂alkylsulfonyl, or C₁-C₂cyanoalkyl;
R⁴ is C₁-C₄alkyl, pyrrolidinyl, or piperidyl; or R⁴ is a ring selected from cyclopropyl, cyclobutyl, phenyl, pyrazolyl, imidazolyl, triazolyl, thiophenyl, isoxazolyl, pyridyl, or pyrimidinyl, wherein the ring is optionally substituted by 1 or 2 substituents independently selected from R³; or R⁴ is C₁-C₂alkyl mono-substituted by cyclopropyl, cyclobutyl, pyridyl, or phenyl, which are optionally substituted by 1 or 2 substituents independently selected from R³; or R⁴ is -NH(C₁-C₄alkyl), or -N(C₁-C₂alkyl)(C₁-C₂alkyl);
R⁵ is C₁-C₄alkyl, C₁-C₆haloalkyl, C₂-C₄alkenyl, C₁-C₄alkoxy-C₁-C₄alkyl, 3-(2,2-difluorocyclopropyl)propyl, 3-(2-chloro-2-fluorocyclopropyl)propyl, 5-(2,2-difluorocyclopropyl)pentyl, 5-(2-chloro-2-fluorocyclopropyl)pentyl,benzylamino, naphthyl, quinolyl, or 2,2-difluoro-1,3-benzodioxolyl; or R⁵ is a ring selected from cyclopropyl, cyclobutyl, phenyl, pyrrolidinyl, piperidyl, 4,5-dihydroisoxazolyl, isoxazolyl, pyrrolyl, pyrazolyl, thiazolyl, thiadiazolyl, imidazolyl, isothiazolyl, pyridazinyl, or pyridyl, wherein each ring is optionally substituted by 1 or 2 substituents independently selected from R⁶; or R⁵ is C₁-C₂alkyl mono-substituted by: (i) phenoxy; or (ii) phenyl optionally substituted by a single substituent independently selected from R⁶; and
R⁶ is chloro, fluoro, methyl, ethyl, trifluoromethyl, methoxy, ethoxy or methylsulfanyl.

10. A compound according to any one of claims 5 to 9, wherein R² is hydrogen, amino, methyl or cyanomethyl, and preferably hydrogen.

11. A compound according to claim 1, wherein R' and R², together with the nitrogen atom they share, form a 4-, 5- or 6-membered saturated or partially saturated heterocyclic ring system optionally further comprising a single group selected from -O-, -S-, -N(H)- or =N-, wherein the ring system is optionally substituted by 1 or 2 substituents independently selected from fluoro, chloro, cyano, methyl, ethyl, methoxy.

12. An agrochemical composition comprising an insecticidally or acaricidally effective amount of a compound according to any one of claims 1 to 11.

13. The composition according to claim 12, further comprising at least one additional active ingredient and/or an agrochemically-acceptable diluent or carrier.

14. A method of controlling insects or acarines, which comprises applying an insecticidally or acaricidally effective amount of a compound of formula (I) as defined in any one of claims 1 to 11, or a composition comprising this compound as active ingredient, to a pest, a locus of pest (preferably a plant), to a plant susceptible to attack by a pest or to plant a propagation material thereof (such as a seed).

15. Use of a compound according to any one of claims 1 to 11 as an insecticide or an acaricide.
